# EUROPEAN PATENT SPECIFICATION

(11) **EP 1 591 069 B1**
(45) Date of publication and mention of the grant of the patent: **23.03.2016**
(21) Application number: 04705538.9
(22) Date of filing: 27.01.2004
(51) Int. Cl.: A61B 17/06

(54) **SURGICAL INSTRUMENT FOR ANASTOMOSIS**
CHIRURGISCHES INSTRUMENT ZUR ANASTOMOSE
INSTRUMENT CHIRURGICAL POUR ANASTOMOSE

(30) Priority: 27.01.2003 JP 2003017716
(43) Date of publication of application: 02.11.2005
(73) Proprietor: Olympus Corporation, Tokyo 151-0072 (JP)
(72) Inventor: IIZUKA, Shuhei, Tachikawa-shi, Tokyo 190-0011 (JP); KOGASAKA, Takahiro, Hachioji-shi, Tokyo 192-0363 (JP); TANIGUCHI, Kazunori, Hachioji-shi, Tokyo 192-0911 (JP); NAGASE, Toru, Tachikawa-shi, Tokyo 190-0012 (JP); YAZAWA, Nobuyoshi, Hachioji-shi, Tokyo 192-0004 (JP); KASAHARA, Hideyuki, Hachioji-shi, Tokyo 192-0023 (JP); MAEDA, Seiji, Kunitachi-shi, Tokyo 186-0005 (JP); BANJU, Kazuo, Hachioji-shi, Tokyo 192-0912 (JP); MURATA, Akira, Inagi-shi, Tokyo 206-0824 (JP)
(74) Representative: von Hellfeld, Axel
(86) International application number: PCT/JP2004/000702
(87) International publication number: WO 2004/066848

(56) References cited:
- WO-A1-01/00095
- WO-A1-01/00095
- BE-A6- 1 006 566
- JP-A- 7 505 801
- US-A- 3 168 097
- US-A- 3 168 097
- US-A- 5 951 575
- US-B1- 6 206 894

## Description

### Technical Field

The present invention relates to a surgical therapeutic instrument for anastomosing the tissue by grasping a needle when performing coronary artery bypass graft (CABG) using an endoscope, an operation system, and an anastomosing procedure method using the surgical therapeutic instrument.

### Background Art

Such bypass operation is well-known, as operation for performing the CABG using an endoscope, that an endoscope, a surgical therapeutic instrument, serving as a needle holder, and forceps are inserted in the chest cavity via a trocar punctured in the chest wall, the coronary artery is partly incised by surgical scissors to create the stoma of anastomosis, the internal thoracic-artery is guided to the stoma of anastomosis by a grasping-forceps, and the internal thoracic artery is anastomosed and connected to the stoma of anastomosis by using the surgical therapeutic instrument.

In the above-described operation, U.S. Patent Publication No. 5,951,575 discloses a surgical therapeutic instrument for anastomosing the tissue by grasping a suture needle with the structure including an insertion unit having, at the distal end thereof, a bending portion and further including a pair of jaws closable and rotatable around the axis of the insertion unit at the distal end of the insertion unit.

However, in the surgical therapeutic instrument disclosed in U.S. Patent Publication No. 5,951,575, the insertion unit has the pair of closable jaws at the distal end of the insertion unit, upon closing the jaws, the pair of jaws is pulled in a cylindrical portion via a cable by operating an operation unit to be closed, and the needle is grasped. Therefore, the positions of the jaws are moved forward/backward upon opening/closing the jaws, so in trying to grasp the needle with opening the jaws and approaching the needle, the positions of the jaws are changed upon closing the jaws, thereby making it impossible to grasp the needle. Consequently, the operability is inferior.

Further, in the surgical therapeutic instrument disclosed in U.S. Patent Publication No. 5,951,575, the holding force and the rotating force of the.suture needle need to synchronously be transmitted to the distal end in the anastomosis using the endoscope, and the following is necessary. That is,
(1) The holding force is kept to increase the load of the rotating force.
(2) The rotation needs to be performed while an operator applies the holding force. In other words, the suture needle is not stably held while simultaneously performing the rotation.

US 3,168,097 discloses a needle holder comprising an operation portion having a scissors-type actuating means comprising one pivoting member coupled to a proximal end of a shaft, the distal end of the shaft having a wedge-shaped member for urging a rocker to rotate about a pivot point to allow a rod to move to release a clamped position of a surgical needle. Additionally, a handle with an arm can be axially actuated to cause axial distal or proximal movement of a cable, which cable is disposed around a pulley and which causes a cylindrical member to rotate about an extending direction in which the second clamping member extends from the needle holder.

WO 2001/000095 discloses a surgical instrument having a tool disposed at a distal end thereof coupled via a ball joint, the orientation of which can be controlled by an assembly disposed at a proximal end of the surgical instrument which can be actuated to control one of several cables disposed in the surgical instrument for orienting and rotating the tool.

US 6,206,894 discloses a needle holder comprising two pinching members, wherein a first of the pinching members is configured as a first cylindrical member having a flange at a distal end thereof and a cylindrical recess therewithin, and a second pinching member having a shaft portion and a head portion, wherein the shaft portion is provided slidably within the recess of the first pinching member. The needle is pinched between the flange of the first pinching member and the proximal surface of the head of the second pinching member.

The present invention is devised in consideration of the above circumferences, and it is an object of the present invention to provide a surgical therapeutic instrument for easily and stably anastomosing the tissue by using the endoscope.

### Disclosure of Invention

According to the present invention, a surgical therapeutic instrument according to claim 1 is provided. The surgical therapeutic instrument may include an insertion unit, a treating unit that is arranged at one end of the insertion unit to be extended in the extending direction at a predetermined angle to the axial direction of the insertion unit, and an operation unit that is arranged at the other end of the insertion unit. The operation unit includes a rotating operation unit to rotate the treating unit, and an opening/closing operation unit to open/close the treating unit. The treating unit includes two pinching members having planar portions, is rotatable in accordance with the rotating operation, and can move at least one of the two pinching members in the direction substantially orthogonal to the plane of the planar portion in accordance with the opening/closing operation.

### Brief Description of the Drawings

Fig. 1 is a flowchart showing the operation procedure of CABG using a needle driver;
Fig. 2 is a first explanatory diagram for forming a port hole to the organ in the body by using a trocar in step S2 in Fig. 1;
Fig. 3 is a second explanatory diagram for forming the port hole to the organ in the body by using the trocar in step S2 in Fig. 1;
Fig. 4 is a flowchart showing the internal-thoracic-artery exfoliating procedure in step S4 in Fig. 1;
Fig. 5 is a first explanatory diagram for the internal-thoracic-artery exfoliating procedure in Fig. 4;
Fig. 6 is a second explanatory diagram for the internal-thoracic-artery exfoliating procedure in Fig. 4;
Fig. 7 is a third explanatory diagram for the internal-thoracic-artery exfoliating procedure in Fig. 4;
Fig. 8 is a fourth explanatory diagram for the internal-thoracic-artery exfoliating procedure in Fig. 4;
Fig. 9 is a fifth explanatory diagram for the internal-thoracic-artery exfoliating procedure in Fig. 4;
Fig. 10 is a sixth explanatory diagram for the internal-thoracic-artery exfoliating procedure in Fig. 4;
Fig. 11 is a seventh explanatory diagram for the internal-thoracic-artery exfoliating procedure in Fig. 4;
Fig. 12 is an eighth explanatory diagram for the internal-thoracic-artery exfoliating procedure in Fig. 4;
Fig. 13 is a ninth explanatory diagram for the internal-thoracic-artery exfoliating procedure in Fig. 4;
Fig. 14 is a tenth explanatory diagram for the internal-thoracic-artery exfoliating procedure in Fig. 4;
Fig. 15 is a flowchart showing the anastomosing procedure in step S5 in Fig. 1;
Fig. 16 is a first explanatory diagram for the anastomosing procedure in Fig. 15;
Fig. 17 is a second explanatory diagram for the anastomosing procedure in Fig. 15;
Fig. 18 is a third explanatory diagram for the anastomosing procedure in Fig. 15;
Fig. 19 is a fourth explanatory diagram for the anastomosing procedure in Fig. 15;
Fig. 20 is a fifth explanatory diagram for anastomosing the procedure in Fig. 15;
Fig. 21 is a sixth explanatory diagram for anastomosing the procedure in Fig. 15;
Fig. 22 is a seventh explanatory diagram for the anastomosing procedure in Fig. 15;
Fig. 23 is an eighth explanatory diagram for the anastomosing procedure in Fig. 15;
Fig. 24 is a ninth explanatory diagram for the anastomosing procedure in Fig. 15;
Fig. 25 is a tenth explanatory diagram for the anastomosing procedure in Fig. 15;
Fig. 26 is an eleventh explanatory diagram for the anastomosing procedure in Fig. 15;
Fig. 27 is a twelfth explanatory diagram for the anastomosing procedure in Fig. 15;
Fig. 28 is a thirteenth explanatory diagram for the anastomosing procedure in Fig. 15;
Fig. 29 is a fourteenth explanatory diagram for the anastomosing procedure in Fig. 15;
Fig. 30 is a fifteenth explanatory diagram for the anastomosing procedure in Fig. 15;
Fig. 31 is a sixteenth explanatory diagram for the anastomosing procedure in Fig. 15;
Fig. 32 is a seventeenth explanatory diagram for the anastomosing procedure in Fig. 15;
Fig. 33a is an eighteenth explanatory diagram for the anastomosing procedure in Fig. 15;
Fig. 33b is a nineteenth explanatory diagram for the anastomosing procedure in Fig. 15;
Fig. 33c is a twentieth explanatory diagram for the anastomosing procedure in Fig. 15;
Fig. 33d is a twenty-first explanatory diagram for the anastomosing procedure in Fig. 15;
Fig. 33e is a twenty-second explanatory diagram for the anastomosing procedure in Fig. 15;
Fig. 34a is a twenty-third explanatory diagram for the anastomosing procedure in Fig. 15;
Fig. 34b is a twenty-fourth explanatory diagram for the anastomosing procedure in Fig. 15;
Fig. 34c is a twenty-fifth explanatory diagram for the anastomosing procedure in Fig. 15;
Fig. 35 is a twenty-sixth explanatory diagram for the anastomosing procedure in Fig. 15;
Fig. 36 is a twenty-seventh explanatory diagram for the anastomosing procedure in Fig. 15;
Fig. 37 is a twenty-eighth explanatory diagram for anastomosing procedure in Fig. 15;
Fig. 38 is a twenty-ninth explanatory diagram for the anastomosing procedure in Fig. 15;
Fig. 39 is a thirtieth explanatory diagram for the anastomosing procedure in Fig. 15;
Fig. 40 is a thirty-first explanatory diagram for the anastomosing procedure in Fig. 15;
Fig. 41 is a thirty-second explanatory diagram for the anastomosing procedure in Fig. 15;
Fig. 42 is a thirty-third explanatory diagram for the anastomosing procedure in Fig. 15;
Fig. 43a is a thirty-fourth explanatory diagram for the anastomosing procedure in Fig. 15;
Fig. 43b is a thirty-fifth explanatory diagram for the anastomosing procedure in Fig. 15;
Fig. 43c is a thirty-sixth explanatory diagram for the anastomosing procedure in Fig. 15;
Fig. 43d is a thirty-seventh explanatory diagram_for the anastomosing procedure in Fig. 15;
Fig. 43e is a thirty-eighth explanatory diagram for the anastomosing procedure in Fig. 15;
Fig. 44 is a thirty-ninth explanatory diagram for the anastomosing procedure in Fig. 15;
Fig. 45 is a fortieth explanatory diagram for the anastomosing procedure in Fig. 15;
Fig. 46 is a forty-first explanatory diagram for the anastomosing procedure in Fig. 15;
Fig. 47 is a forty-second explanatory diagram for the anastomosing procedure in Fig. 15;
Fig. 48 is a forty-third explanatory diagram for the anastomosing procedure in Fig. 15;
Fig. 49 is a forty-fourth explanatory diagram for the anastomosing procedure in Fig. 15;
Fig. 50 is a forty-fifth explanatory diagram for the anastomosing procedure in Fig. 15;
Fig. 51 is a forty-sixth explanatory diagram for the anastomosing procedure in Fig. 15;
Fig. 52 is a forty-seventh explanatory diagram for the anastomosing procedure in Fig. 15;
Fig. 53 is a forty-eighth explanatory diagram for the anastomosing procedure in Fig. 15;
Fig. 54 is a forty-ninth explanatory diagram for the anastomosing procedure in Fig. 15;
Fig. 55 is a diagram showing the arrangement position of the trocar shown in Fig. 1 arranged at the chest according to a modification;
Fig. 56 is a diagram showing a graft for CABG shown in Fig. 1;
Fig. 57 is a diagram showing a graft for CABG shown in Fig. 56 according to a first modification;
Fig. 58 is a diagram showing a graft for CABG shown in Fig. 56 according to a second modification;
Fig. 59 is a diagram showing a graft for CABG shown in Fig. 56 according to a third modification;
Fig. 60 is a diagram showing a graft for CABG shown in Fig. 56 according to a fourth modification;
Fig. 61 is a perspective view showing a needle driver according to the embodiment;
Fig. 62 is a perspective view showing a needle driver in view of another angle different from an angle shown in Fig. 61;
Fig. 63 is a plan view showing the needle driver according to the embodiment;
Fig. 64 is a front view showing the needle driver according to the embodiment;
Fig. 65 is a bottom view showing the needle driver according to the embodiment;
Fig. 66 is a perspective view showing a needle driver according to a first modification;
Fig. 67 is a plan view showing the needle driver according to the first modification;
Fig. 68 is a front view showing the needle driver according to the first modification;
Fig. 69 is a cross-sectional view showing the distal end including a treating unit of the needle driver according to the embodiment;
Fig. 70 is a cross-sectional view showing the distal end upon opening a pinching portion of the treating unit of the needle driver according to the embodiment;
Fig. 71 is a cross-sectional view along an A-A line shown in Fig. 69;
Fig. 72 is a perspective view showing the distal end including the treating unit of the needle driver, partly omitting a distal cover, in the bottom direction according to the embodiment;
Fig. 73 is a cross-sectional view showing the proximal end including the operation unit of the needle driver according to the embodiment;
Fig. 74 is a perspective view showing the proximal end including the operation unit of the needle driver, excluding a top cover, in the upper diagonal direction according to the embodiment;
Fig. 75 is a cross-sectional view along a B-B line shown in Fig. 73;
Fig. 76 is one explanatory diagram for rotation;
Fig. 77 is another explanatory diagram for rotation;
Fig. 78 is an explanatory diagram for a mechanism that adjusts the amount of friction;
Fig. 79 is an explanatory diagram for operation for opening a treating unit to pinch a needle;
Fig. 80 is an explanatory diagram for operation for pinching and rotating the needle;
Fig. 81 is an explanatory diagram for needle rotation;
Fig. 82 is a diagram showing an example of setting the color of the surface of the treating unit to be different from the color of the needle;
Fig. 83 is a diagram showing an example of the color of the surface of one of two pinching members to be different from the color of the other;
Fig. 84 is a perspective view showing the appearance of the operation unit having a finger placing unit;
Fig. 85 is a perspective view in the view different from that shown in Fig. 84;
Fig. 86 is one explanatory diagram for the structure of the finger placing unit and its using method;
Fig. 87 is another explanatory diagram for the structure of the finger placing unit and its using method;
Fig. 88 is another explanatory diagram for the structure of the finger placing unit and its using method;
Fig. 89 is a perspective view showing the operation unit with the structure for changing the position of the finger placing unit;
Fig. 90 is a cross-sectional view along a C-C line shown in Fig. 67;
Fig. 91 is a cross-sectional view along a D-D line shown in Fig. 67 when the treating unit is opened;
Fig. 92 is a cross-sectional view along the D-D line shown in Fig. 67 when the treating unit is closed;
Fig. 93 is a perspective view showing a treating unit according to a first modification;
Fig. 94 is a perspective view for explaining a rotation-force transmitting mechanism according to the first modification;
Fig. 95 is a view in the direction of an arrow E shown in Fig. 93;
Fig. 96 is a longitudinal cross-sectional view showing a needle driver according to a second modification;
Fig. 97 is a longitudinal cross-sectional view showing a treating unit according to a second modification;
Fig. 98 is a cross-sectional view along an F-F line shown in Fig. 96;
Fig. 99 is a schematic diagram showing a state in which the needle driver approaches the operation portion via the trocar;
Fig. 100 is a longitudinal cross-sectional view showing a needle driver according to a third modification;
Fig. 101 is a longitudinal cross-sectional view showing a treating unit according to a third modification;
Fig. 102 is a longitudinal cross-sectional view showing a needle driver according to a fourth modification;
Fig. 103 is a longitudinal cross-sectional view showing a treating unit of a needle driver according to a fifth modification;
Fig. 104 is a longitudinal cross-sectional view showing a needle driver according to a sixth modification;
Fig. 105 is an explanatory diagram for the structure of the needle driver according to the sixth modification; and
Fig. 106 is an explanatory diagram of an example using a foot switch.

### Best Mode for Carrying Out the Invention

A detailed description is given of the present invention in accordance with the drawings.

First, a description is given of the operation procedure of CABG, serving as an anastomosing procedure method using an endoscope with a needle driver, serving as a surgical therapeutic instrument according to an embodiment.

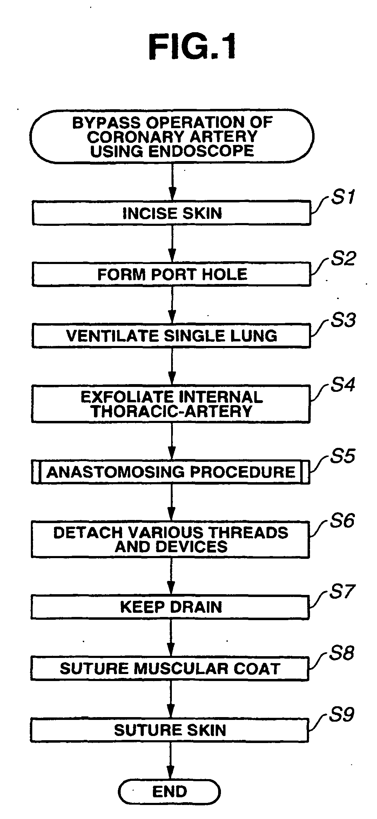

Referring to Fig. 1, in the CABG, the skin at a predetermined position of the chest (for example, positions in between the third, fourth and sixth ribs on the left side) is incised by using a surgical knife in step S1.

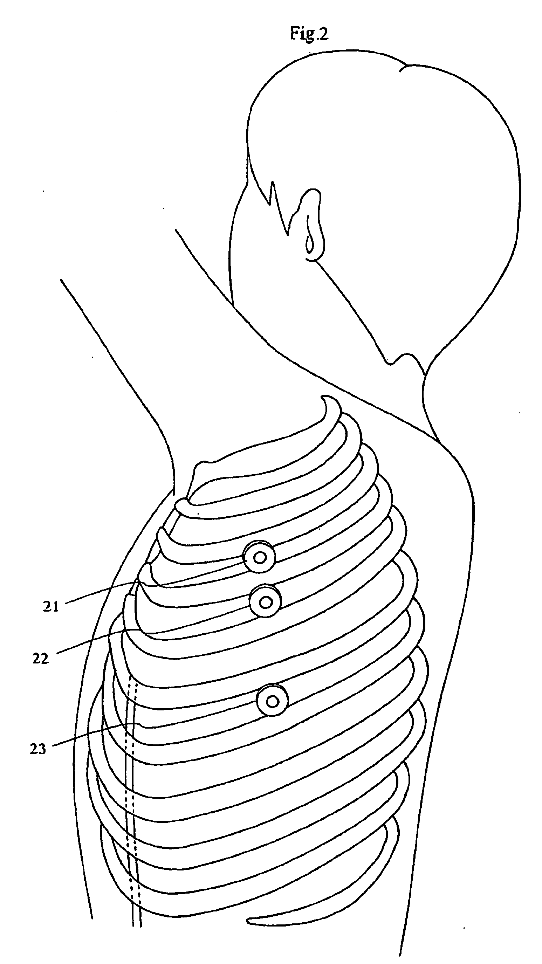

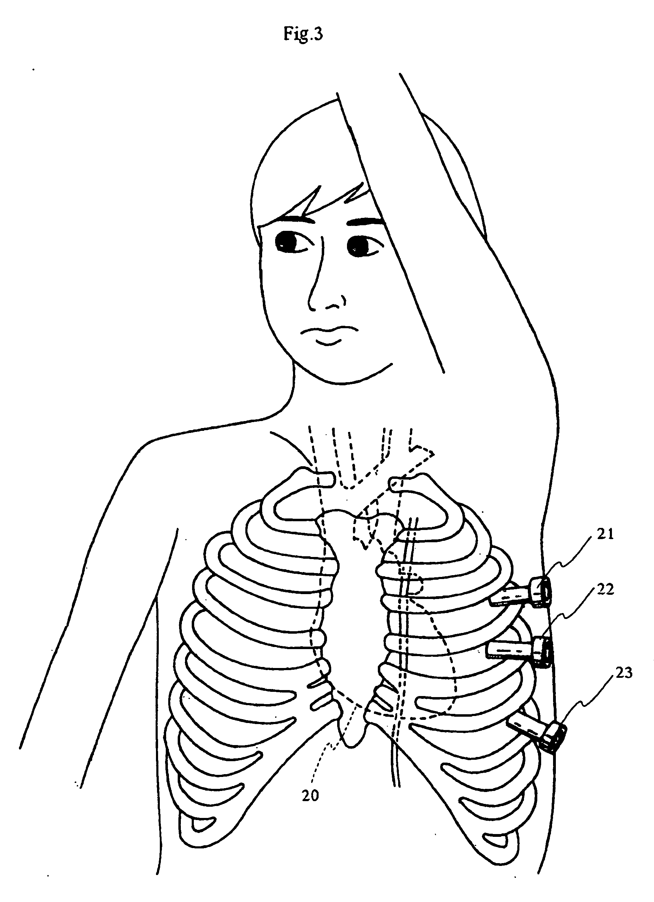

After the incision, in step S2, the finger or an inner needle having a conical-shaped distal end thereof is inserted in a sheath of a trocar to be projected from the distal end, the incision portion of the skin is widened to form a hole in the body, and the inner needle is pulled-out from the sheath of the trocar when the hole is formed to a desired position. Thus, referring to Figs. 2 and 3, a port hole is formed to the organ in the body by trocars 21 to 23. Thus, various therapeutic instruments can approach the left chest-cavity via the trocars 21 to 23.

In step S3, as used in the procedure using a normal (well-known) thracoscope, a single-lung ventilation is performed so as to ensure the field of view. That is, a tracheal tube for single-lung ventilation is inserted in the trachea, the ventilation in the single (right) lung is executed, and another (left) lung is degassed.

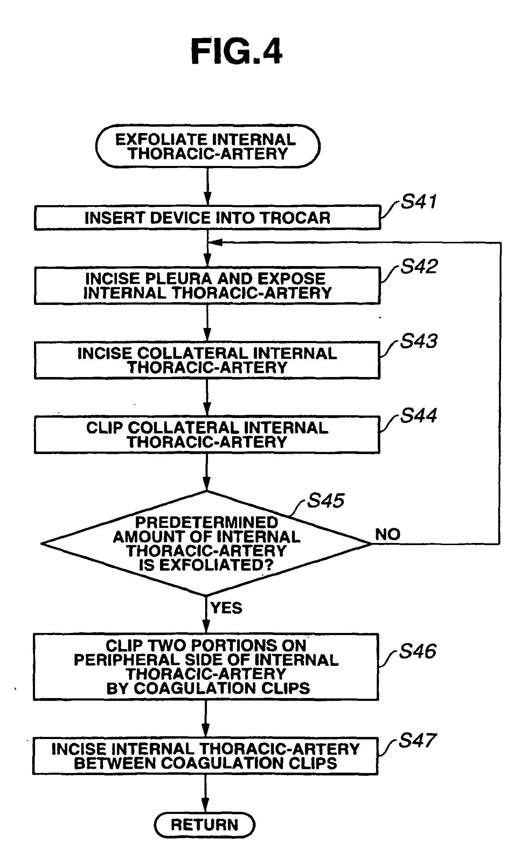

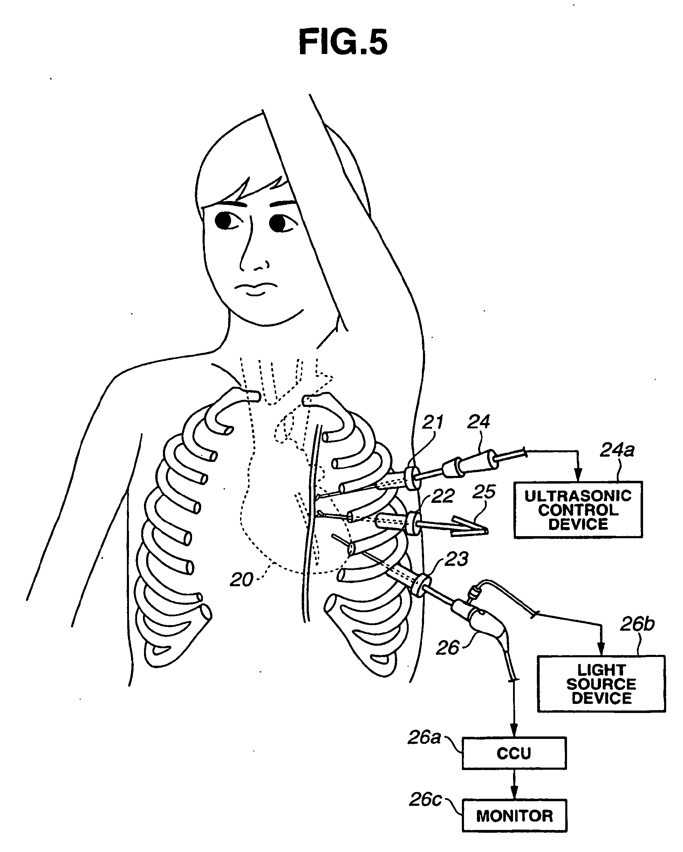

In step S4, the internal-thoracic-artery exfoliating procedure is performed. The internal-thoracic-artery exfoliating procedure will be described with reference to Figs. 5 to 14 by using a flowchart shown in Fig. 4.

Referring to Fig. 4, in the internal-thoracic-artery exfoliating procedure, in step S41 in Fig. 5, an ultrasonic therapeutic instrument 24, grasping forceps 25, and an endoscope 26 are inserted in the trocars 21 to 23 arranged to the port holes. The ultrasonic therapeutic instrument 24 is connected to an ultrasonic control device 24a for controlling the supply of ultrasonic driving energy to the ultrasonic therapeutic instrument 24. The endoscope 26 is connected to a light source unit 26b for supplying illumination light and a CCU (camera control unit) 26a for processing signals of an endoscope image. The ultrasonic therapeutic instrument 24 may be an ultrasonic therapeutic instrument having an electric-knife function or an electric knife.

The endoscope image obtained from the endoscope 26 is an image of an endoscope image picked-up by a TV camera arranged to an eyepiece, and is displayed on a monitor 26c by the CCU 26c.

The endoscope image is picked-up by the TV camera in the endoscope 26. However, the present invention is not limited to this and the procedure according to the embodiment can be performed while observing the endoscope image by the eyepiece portion of the endoscope. Further, the endoscope 26 has the TV camera at the eyepiece portion. However, the endoscope 26 has image pick-up means such as a CCD at the distal end of the insertion unit to process an image pickup signal from the CCD by the CCU 26a.

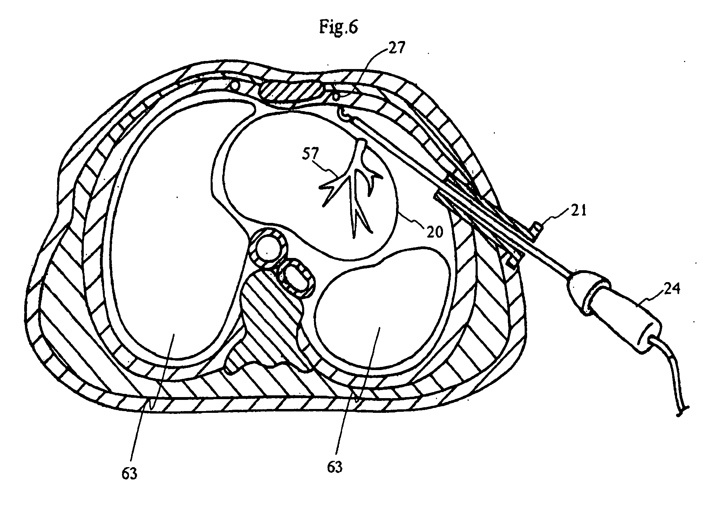

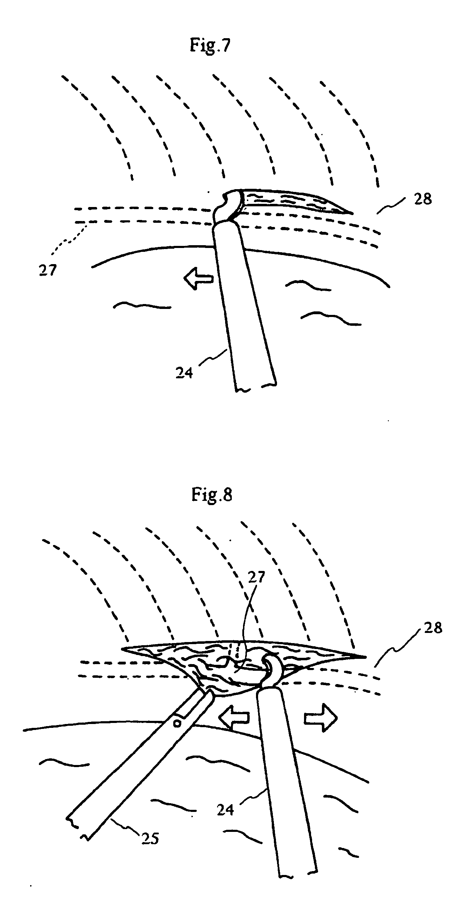

In step S42, referring to Fig. 6, the ultrasonic therapeutic instrument 24 is approached by an internal thoracic-artery 27 under the observation using the endoscope 26. Referring to Fig. 7, a pleura 28 that covers the internal thoracic-artery 27 is incised.

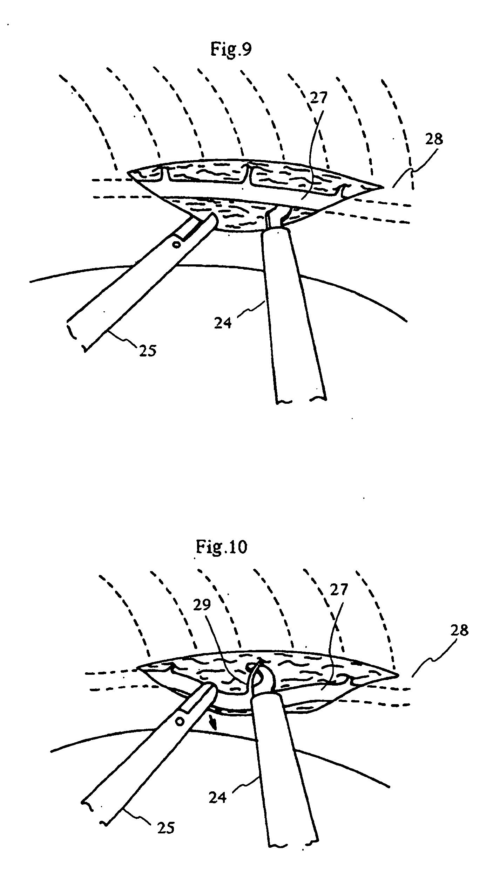

In step S43, the internal thoracic-artery 27 and the collateral incision portion of the pleura 28 are exposed from the peripheral organ by using the grasping forceps 25 and the ultrasonic therapeutic instrument 24 as shown in Figs. 8 and 9. A collateral (vessel) 29 extended from the lateral wall of the internal thoracic-artery 27 is severed by the ultrasonic therapeutic instrument 24 as shown in Fig. 10. In step S44, the severed collateral (vessel) 29 is clipped by the ultrasonic therapeutic instrument 24, thereby partly abrading the internal thoracic-artery 27.

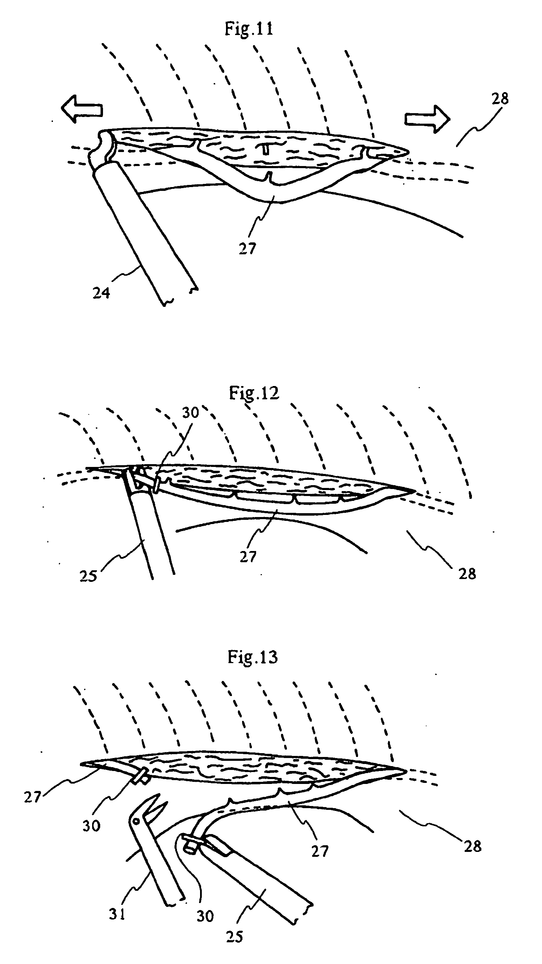

In step S45, an area is enlarged as shown in Fig. 11 until exfoliating a predetermined amount of the internal thoracic-artery 27 (e.g., 15 to 20 cm), and the pleura 28 is continuously incised. Then, the processing in steps S42 to S44 is repeated.

After exfoliating a predetermined amount of the internal thoracic-artery 27, in step S46, two portions on the peripheral side at the severing position of the internal thoracic-artery 27 are clipped by coagulation clips 30 with the grasping forceps 25 as shown in Fig. 12, thereby clipping the blood.

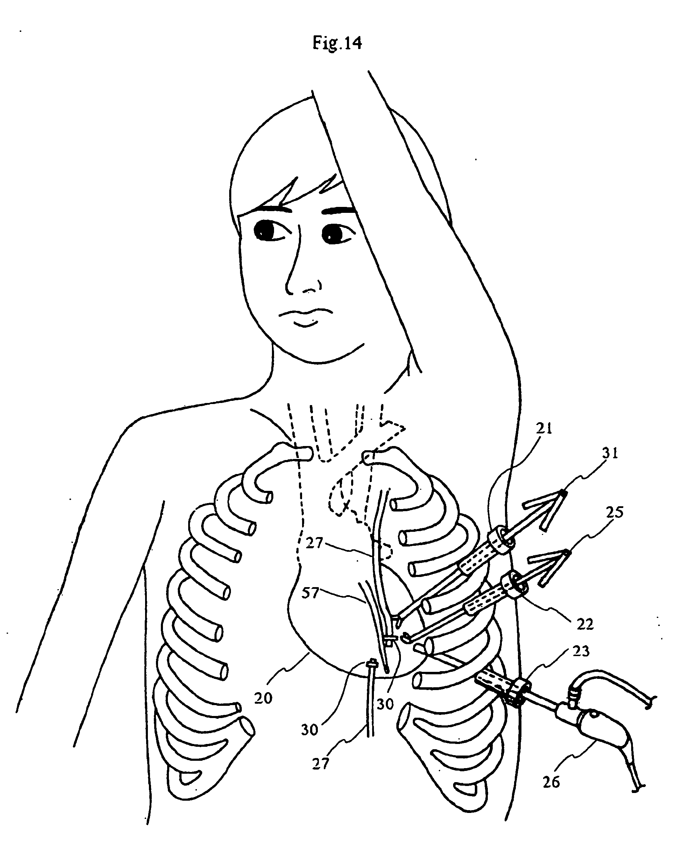

In step S47, in place of the ultrasonic therapeutic instrument 24, the internal thoracic-artery 27 is severed at the severing position of the internal thoracic-artery 27 between the coagulation clips 30 by using surgical scissors 31 as shown in Fig. 13. The internal-thoracic-artery exfoliating procedure ends as shown in Fig. 14.

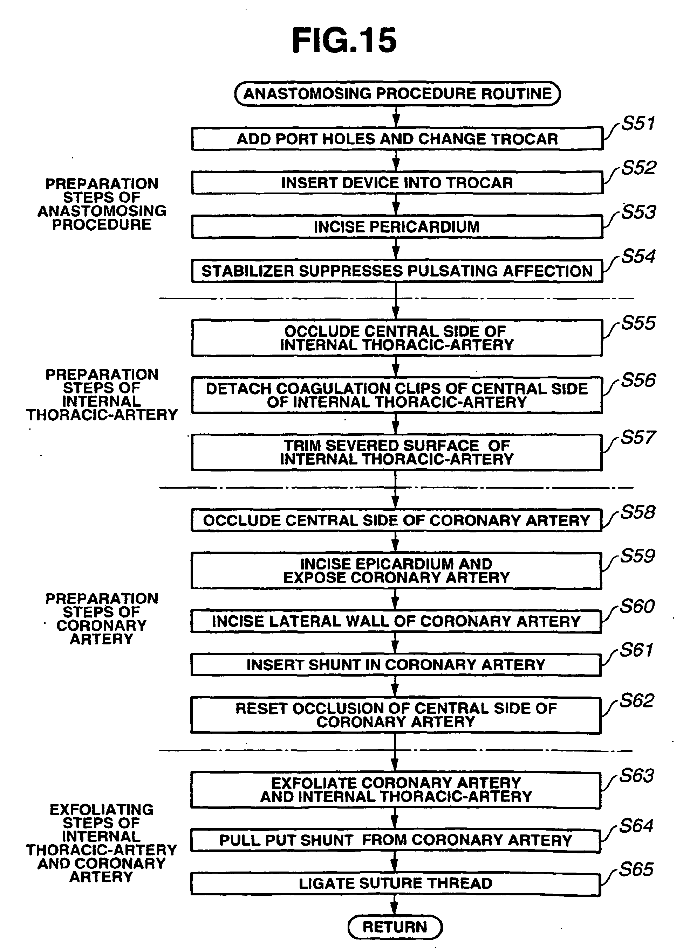

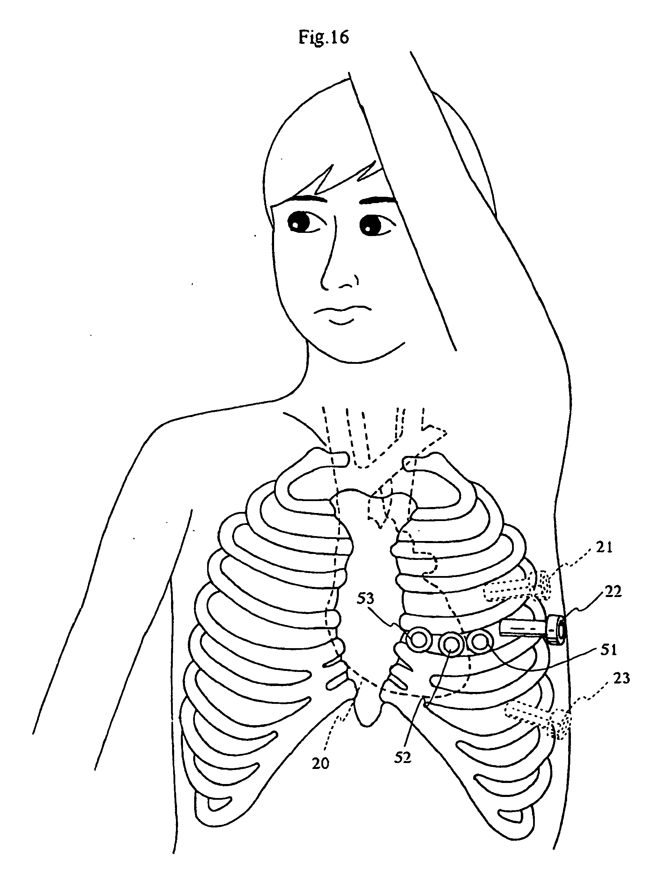

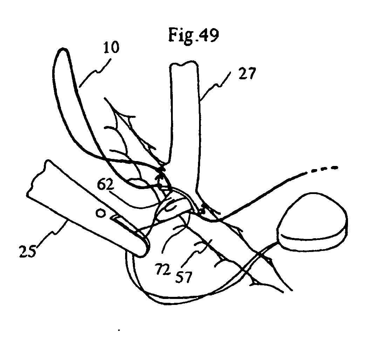

Referring back to Fig. 1, after ending the internal-thoracic-artery exfoliating procedure in step S4, in step S5, the anastomosing procedure of the internal thoracic-artery and the coronary artery is performed. Here, the anastomosing procedure will be described with reference to Figs. 16 to 54 in accordance with the flowchart shown in Fig. 15.

Referring to Fig. 15, in the anastomosing procedure of the internal thoracic-artery and the coronary artery, in step S51, three port holes are added at the position for approach to the periphery of the heart of various therapeutic instruments from the top as shown in Fig. 16, thereby changing the inserting position of the trocar. Referring to Fig. 16, three port holes are added to the fourth intercostal upper portion, the trocars 21 and 23 are pulled-out, the trocar 22 remains, and other trocars 51 to 53 are inserted into the added port holes. Further, the trocars 21 and 23 may remain as shown by a dotted line in Fig. 16.

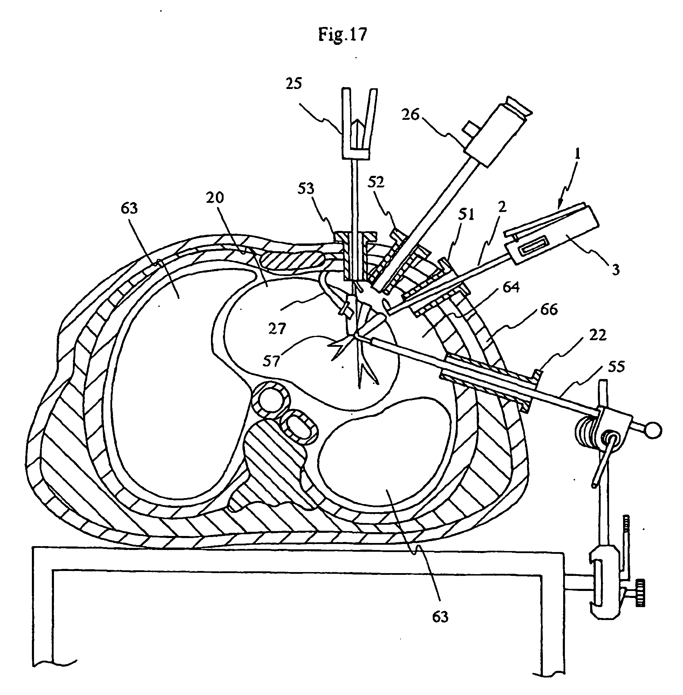

Fig. 17 is a cross-sectional view showing the chest including the heart. Reference numeral 20 denotes the heart, reference numeral 63 denotes the lung, reference numeral 64 denotes the thoracic cavity, reference numeral 57 denotes the coronary artery (left anterior descending branch), and reference numeral 27 denotes the internal thoracic-artery (transplanting vessel). Four trocars 22, 51, 52, and 53 are inserted in the chest wall 66. The endoscope 26 is inserted via the trocar 52 just above the coronary artery 57. That is, the operation is performed with the observation just above the operation portion.

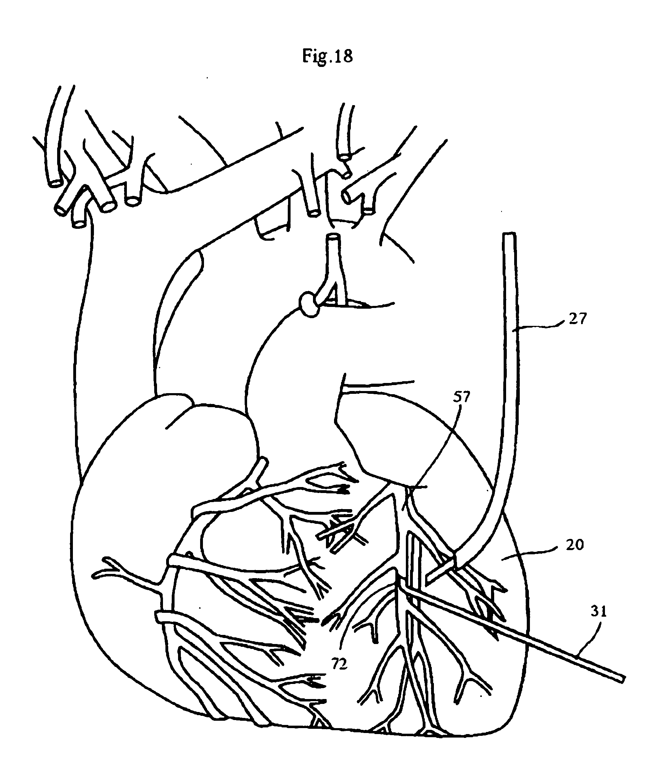

Fig. 18 shows the state of ending the pre-stage for anastomosis of vessel. The internal thoracic-artery 27 is exfoliated from the chest wall 66 shown in Fig. 17 by using the port opened on the chest side (not shown). A stoma of anastomosis 72 is formed by the incision with the surgical scissors 31 downstream the stenosis in the coronary artery 57.

In step S52, a stabilizer 55, the needle driver 1 serving as the surgical therapeutic instrument according to the embodiment or other forceps, the endoscope 26, the grasping forceps 25 are inserted in the trocar 22, 51, 52, and 53 as shown in Fig. 17. The insertion unit 2 of the needle driver 1 is inserted in the thoracic cavity 64, and the operation unit 3 of the needle driver 1 is out of the body cavity. The therapeutic instrument such as the grasping forceps 25 is inserted in the trocar 53. The diameter of vessel of the coronary artery 57 or internal thoracic-artery 27 is 2 to 3 mm.

The stabilizer 55 is a therapeutic instrument for suppressing the pulsating affection of the heart 20, as disclosed in, e.g., U.S. Patent Publication No. 5,807,243, and a description thereof is thus omitted.

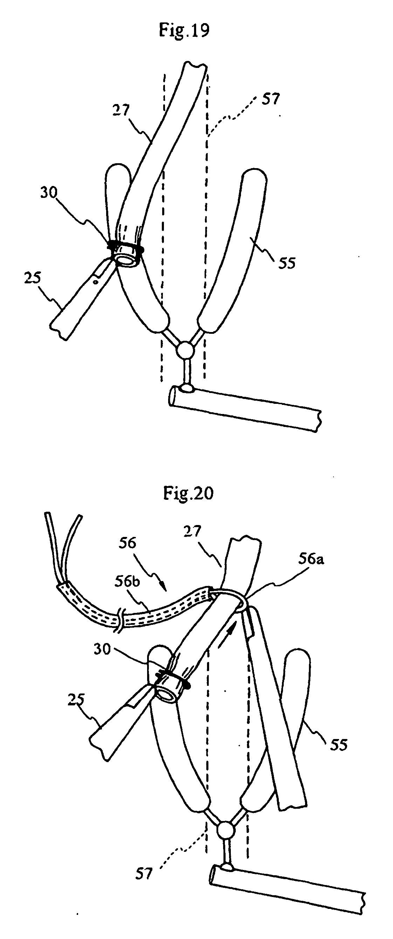

Fig. 19 is a diagram showing the target coronary artery 57 in the field of view for endoscope (operator view). Referring to Fig. 19, in step S53, a pericardium 60 is incised to expose the epicardial surface. In step S54, the stabilizer 55 suppresses the pulsating affection of the heart 20 near the target coronary artery 57.

Steps S51 to S54 correspond to preparation steps of the anastomosing procedure in the anastomosing procedure routine.

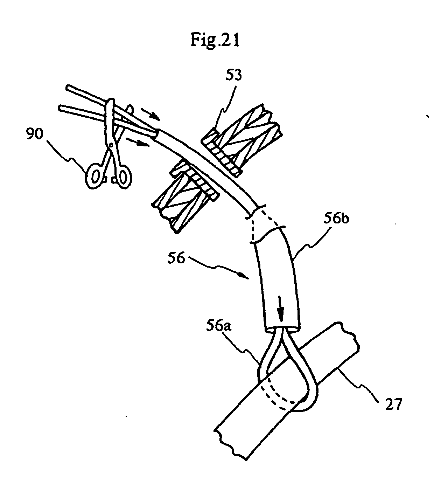

The stabilizer 55 suppresses the pulsating affection of the heart 20. Simultaneously, in step S55, the internal thoracic-artery 27 is inserted to a tourniquet 56 for occluding the internal thoracic-artery 27 via the trocar 51, a distal ring 56a of the tourniquet 56 passes through the portion on the central side from the coagulation clip 30 of the central internal thoracic-artery 27 which is exfoliated and incised by using the two grasping forceps 25 as shown in Fig. 20. The proximal end of the tourniquet 56 is extracorporeally pulled-out as shown in Fig. 21, thereby tightening the distal ring 56a by the tube 56b arranged at the distal end of the tourniquet 56 and occluding the internal thoracic-artery 27. Then, in step S56, the portion having the coagulation clip 30 is removed.

The internal thoracic-artery 27 is tightened by feeding the tube 56b to the distal ring 56a and a grasping tool 90 fixes the distal ring 56a and the tube 56b, thereby keeping the occluding state using the tourniquet 54.

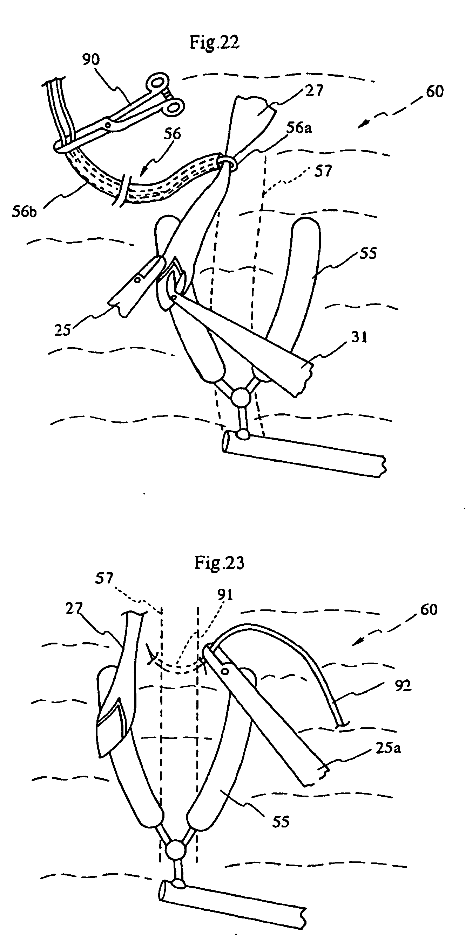

In step S57, the cross section of the internal thoracic-artery 27 is trimmed with a predetermined shape by using the surgical scissors 31 via the trocar 51 as shown in Fig. 22.

Steps S55 to S57 correspond to an internal thoracic-artery preparation process.

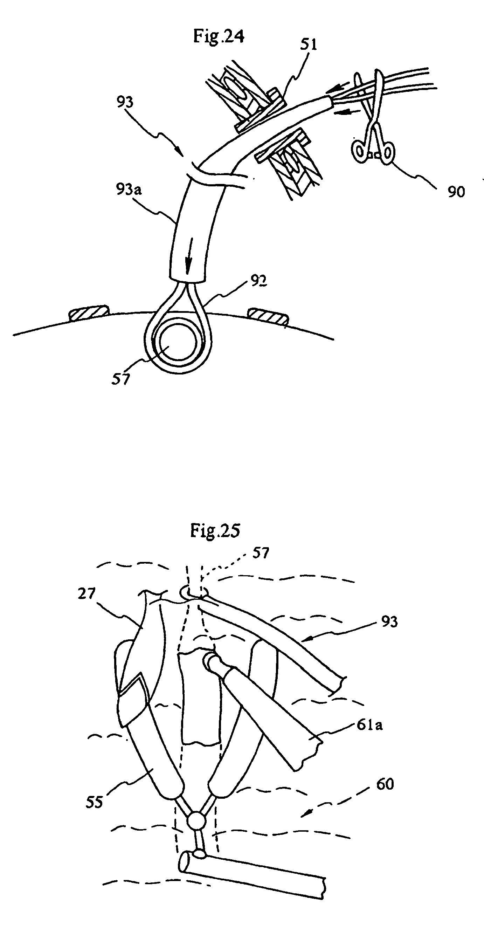

In step S58, a needle 55 and a thread 56 for occluding the vessel are inserted by using a needle holder 25a via the trocar 51 and a thread 92 is wound to the portion on the central side of the coronary artery 57 by using a needle 91 and the thread 92 as shown in Fig. 23. As shown in Fig. 24, the end of the needle 91 is extracorporeally pulled-out via the trocar 51, the distal end and the proximal end of the thread 92 are inserted in a tube 93a to form a tourniquet 93, and the portion on the central side of the coronary artery 57 is occluded by the tourniquet 93.

The thread 92 may be any of 5-0, 4-0, and 3-0 threads based on the USP size (USP23 specification).

In step 59, a beaver knife 61a (or micro-knife) having a circular distal end incises the epicardial surface 60 for covering the coronary artery 57 to expose the coronary artery 57, as shown in Fig. 25.

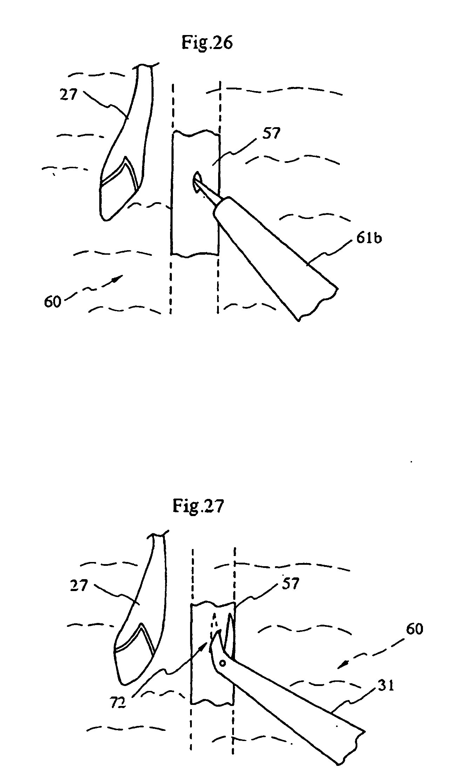

In step S60, via trocar 51 the micro-knife 61b having the sharp end incises the lateral wall as shown in Fig. 26, and the stoma of anastomosis 72 is formed by opening a predetermined amount by the surgical scissors 31 via the trocar 51 as shown in Fig. 27.

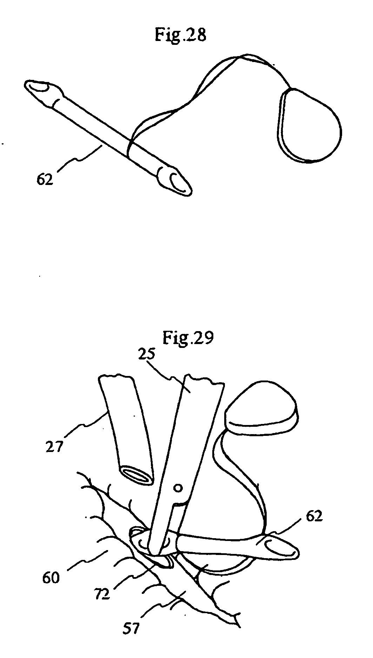

Subsequently, in step S61, a shunt 62 as shown in Fig. 28 is inserted via the trocar 51, and the shunt 62 is inserted in the coronary artery 57 from the stoma of anastomosis 72 of the coronary artery 57 as shown in Fig. 29. In step S62, the tourniquet 93 is loosened and the occlusion of the coronary artery 57 is reset. Thus, the blood flow of the coronary artery 57 is ensured.

Steps S58 to S62 correspond to a preparation process of the coronary artery in the anastomosing procedure routine.

After that, in step S63, the vessel anastomosis (suture) procedure of the internal thoracic-artery 27 and the coronary artery 57 is performed by the needle driver 1, serving as surgical therapeutic instrument according to the embodiment.

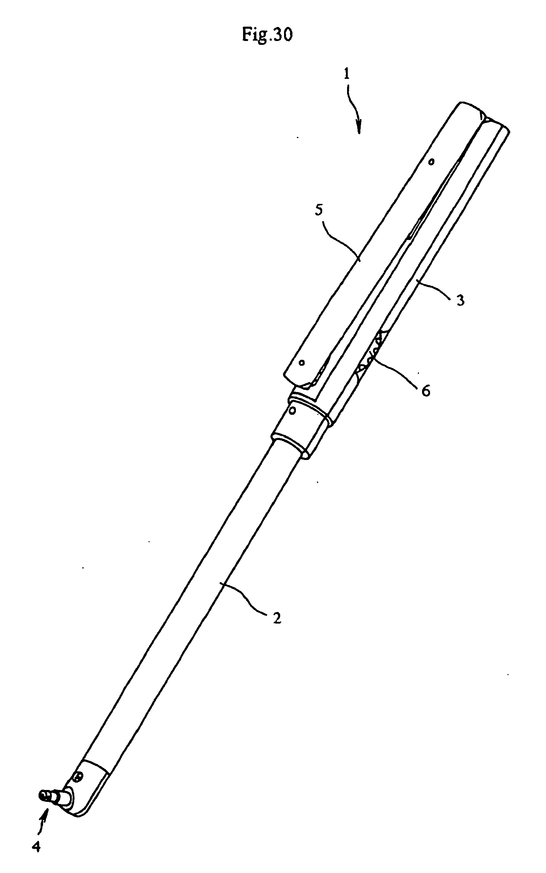

Hereinbelow, the vessel anastomosis (suture) procedure of the internal thoracic-artery 27 and the coronary artery 57 will be described with reference to Figs. 30 to 48.

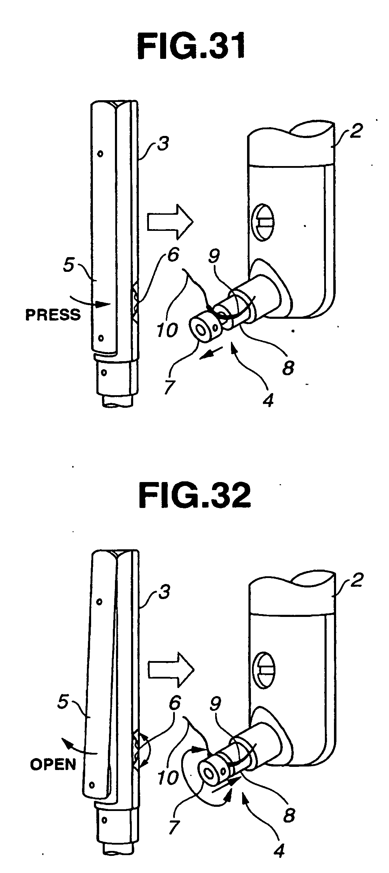

The needle driver 1 comprises the insertion unit 2, the operation unit 3, and a treating unit 4 arranged at the distal end of the insertion unit 2 as shown in Fig. 30, which will be described in detail. The operation unit 3 comprises an open/close lever 5 and a rotating dial 6. The treating unit 4 is arranged on the inserting axis of the insertion unit 2 at a predetermined angle. As shown in Fig. 31, the axis of the insertion unit 2 has a first pinching unit 7 and a second pinching unit 8. The second pinching unit 8 is slidable in the axial direction to the first pinching unit 7 fixed to the axis, and the first pinching unit 7 and the second pinching unit 8 are rotatable around the axis.

Referring to Fig. 31, the finger presses the open/close lever 5, thereby sliding the second pinching unit 8 in the treating unit 4. Further, the gap is formed between the first pinching unit 7 and the second pinching unit 8, thereby positioning the proximal end of the needle 9 having a thread 10 in the gap. Referring to Fig. 32, the open/close lever 5 is detached and the force is applied to close the interval between the first pinching unit 7 and the second pinching unit 8. Thus, the energization force keeps the holding state of the needle 9. That is, the operator applies pressing force only upon opening the first pinching unit 7 and the second pinching unit 8. However, the closing state of the first pinching unit 7 and the second pinching unit 8 is kept by the energization force. Thus, the operator can perform another operation, e.g., operation of the rotating dial 6 without operating the open/close lever 5.

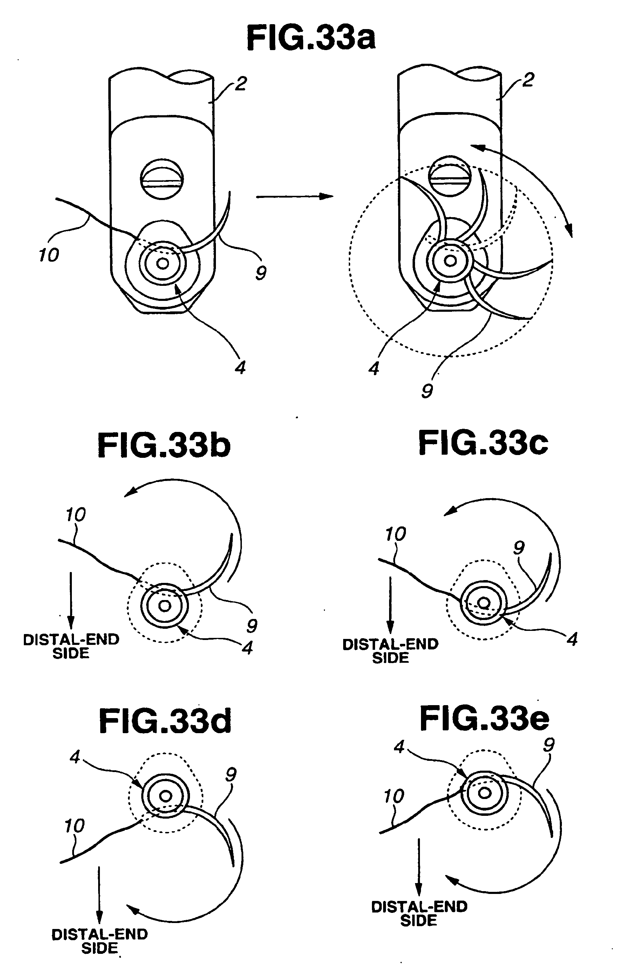

Referring to Fig. 32, the finger rotates the rotating dial 6, thereby transmitting the rotating force to the treating unit 4. As shown in Fig. 33a, the needle 9 can be rotated around the axis of the treating unit 4 while being held by the treating unit 4.

The rotating direction for puncture at the distal end of the needle 9 varies depending on the pinching state at the treating unit 4 of the needle 9. That is, when the needle 9 is pinched on the proximal-end side of the insertion unit 2 of the axis of the treating unit 4 as shown in Fig. 33b, or when the needle 9 is pinched on the distal-end side of the insertion unit 2 of the axis of the treating unit 4 as shown in Fig. 33c, while the bending state of the needle 9 is directed to the proximal-end side of the insertion unit 2, the rotation for puncture corresponds to the counterclockwise direction when the axis of the treating unit 4 is viewed from the top. Further, when the needle 9 is pinched on the distal-end side of the insertion unit 2 of the axis of the treating unit 4 as shown in Fig. 33d or when the needle 9 is pinched on the proximal-end side of the insertion unit 2 of the axis of the treating unit 4 as shown in Fig. 33e while the bending state of the needle 9 is directed to the distal-end side of the insertion unit 2, the rotation for puncture corresponds to the clockwise direction when the axis of the treating unit 4 is viewed from the top.

For the purpose of omitting the description, the pinching state of the needle 9 is set to the state shown in Fig. 33c, and the rotation for puncture is in the counterclockwise direction. As mentioned above, the rotating direction for puncture is defined depending on the pinching state of the needle 9.

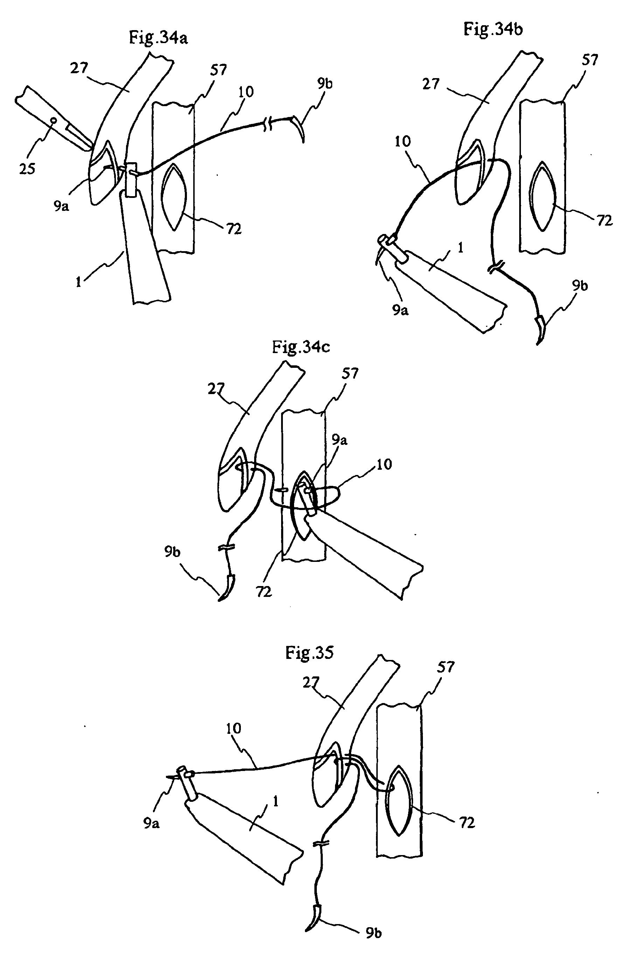

Referring to Fig. 34a, a thread 10 for vessel suture and needles 9a and 9b for vessel suture arranged to the thread 10 for vessel suture are inserted via the trocar 51. The thread 10 may contain any of 8-0 and 7-0 mono-filament threads based on the USP size (USP23 specification).

Figs. 34a to 35, 37 to 39, and 41 to 43e show the flows of the vessel anastomosis (suture) procedure using the endoscope. The first needle 9a and the second needle 9b are excessively fine to suture the vessel with the diameter of vessel within 2 to 3 mm. The needles 9a and 9b are connected to both ends of the thread 10.

First, the first needle 9a is grasped by the treating unit 4 to position a sharp end portion of the second needle 9b on the right side of the treating unit 4.

Referring to Fig. 34a, the epicardial surface of the internal thoracic-artery 27 exfoliated from the chest wall 66 is grasped by the grasping forceps 25. In this state, the rotating dial 6 is rotated to direct the first needle 9a to the outer wall near the severed portion (end portion) of the internal thoracic-artery 27, and the treating unit 4 is rotated in the rotating direction for puncture. The rotation of the treating unit 4 punctures the sharp end portion of the first needle 9a to the internal thoracic-artery 27. That is, the sharp end portion of the first needle 9a is punctured from the outer wall of the internal thoracic-artery 27 to the inside.

Next, the open/close lever 5 is operated to open the first pinching unit 7 and the second pinching unit 8 of the treating unit 4. The first needle 9a pierced through the internal thoracic-artery 27 is detached once. The distal end of the first needle 9a is pinched again in the inner cavity of the internal thoracic-artery 27 by the first pinching unit 7 and the second pinching unit 8. Referring to Fig. 34b, the needle 9a is pulled-out from the inside of the internal thoracic-artery 27.

Referring to Fig. 34c, the rotating dial 6 is rotated to direct the first needle 9a to the outer wall from the inner wall of the stoma of anastomosis 72 of the coronary artery 57. The rotating dial 6 is rotated to rotate the treating unit 4 in the rotating direction for puncture. The rotation of the treating unit 4 punctures the sharp end portion of the first needle 9a to the inner wall of the coronary artery 57. That is, the sharp end portion of the first needle 9a is punctured from the inner wall to the outer wall of the stoma of anastomosis 72 in the coronary artery 57.

Referring to Fig. 35 again, the treating unit 4 is guided to the internal thoracic-artery 27 by operating the operation unit 3, the rotating dial 6 is rotated to direct the first needle 9a to the outer wall near the severed portion of the internal thoracic-artery 27, the treating unit 4 is rotated in the rotating direction for puncture to puncture the sharp end portion of the first needle 9a to the internal thoracic-artery 27, the first needle 9a is grasped again by the treating unit 4, and the needle 9a is pulled-out from the inside of the internal thoracic-artery 27.

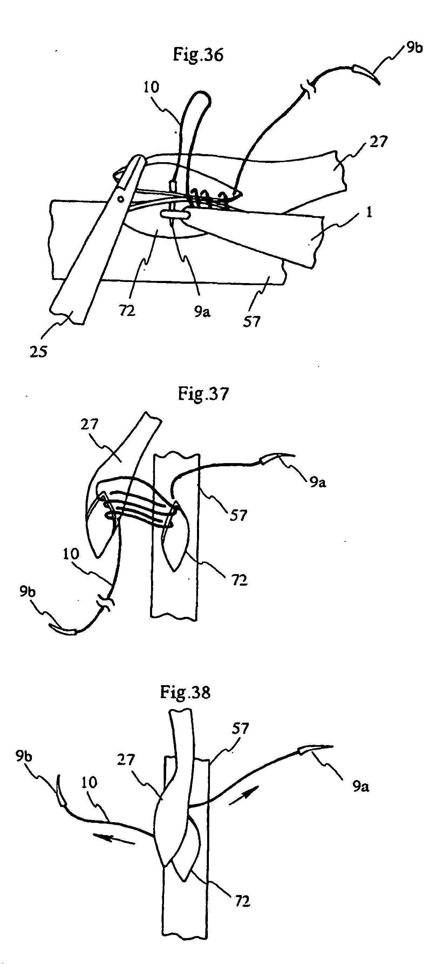

By repeating the above-described operation a plurality of times, as shown in Fig. 37, the thread 10 connects the stoma of anastomosis 72 of the coronary artery 57 and the severed portion of the internal thoracic-artery 27 while the thread 10 draws the locus of spiral loop. Next, one end of the thread 10 is grasped by the grasping forceps 25, the other end is grasped by the treating unit 4 in the needle driver 1, and the grasping forceps 25 and the needle driver 1 are moved to be separated from each other. Then, referring to Fig. 38, the thread 10 is pulled in the direction of an arrow to reduce the loop diameter of the thread 10, and a part of the stoma of anastomosis 72 in the coronary artery 57 and a part of the severed portion in the internal thoracic-artery 27 are pulled-in to be jointed to each other.

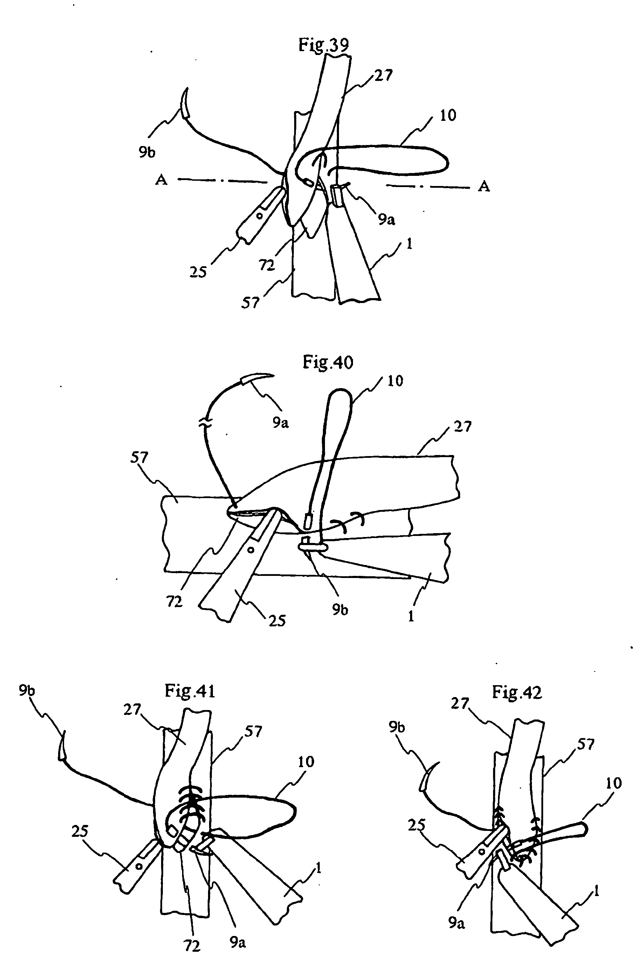

In this state, a part of the stoma of anastomosis 72 in the coronary artery 57 and a part of the severed portion of the internal thoracic-artery 27 are anastomosed by the thread 10 for vessel suture. In order to anastomose a non-anastomosed portion of the stoma of anastomosis 72 in the coronary artery 57 and the severed portion of the internal thoracic-artery 27, the stoma of anastomosis 72 of the coronary artery 57 and the severed portion of the internal thoracic-artery 27 are anastomosed in accordance with the sequence shown in Figs. 39, 41, and 42, and Figs. 43a to 43e serving as A-A cross sections.

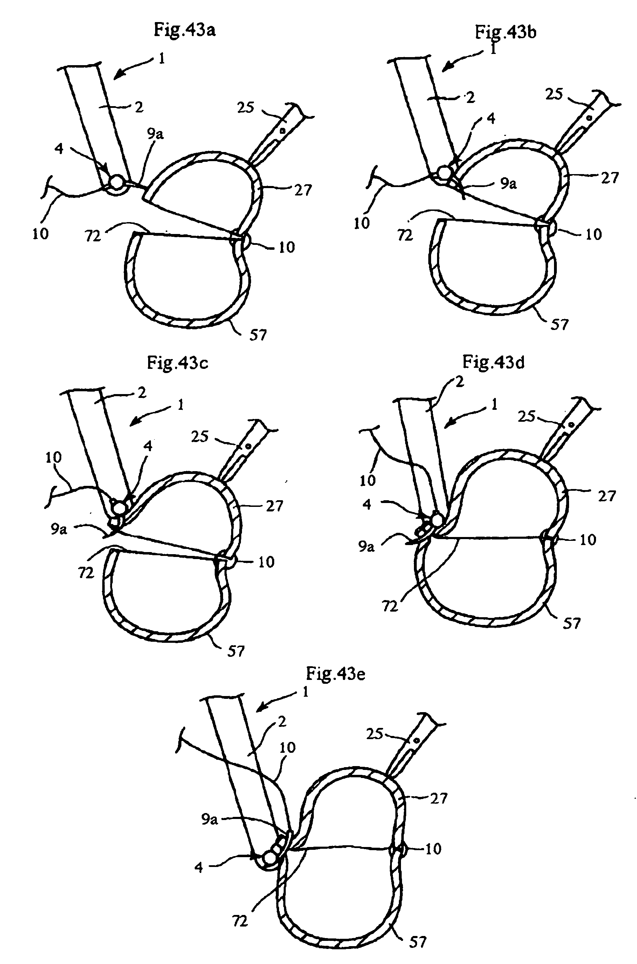

That is, referring to Fig. 43a, the grasping forceps 25 elevates the internal thoracic-artery 27 and, in this state, the rotating dial 6 is rotated to direct the first needle 9a to the outer wall near the severed portion of the internal thoracic-artery 27, and the treating unit 4 is rotated. Referring to Fig. 43b, the rotation of the treating unit 4 punctures the sharp end portion of the first needle 9a to the internal thoracic-artery 27.

Referring to Fig. 43c, in this state, the insertion unit 2 advances and returns while grasping the operation unit 3, the sharp end portion of the first needle 9a is guided to the inner wall of the coronary artery 57 via the treating unit 4, and the treating unit 4 is rotated. Then, referring to Fig. 43d, the sharp end portion of the first needle 9a is punctured from the inner wall of the coronary artery 57 to the outside.

Next, the first pinching unit 7 and the second pinching unit 8 of the treating unit 4 are opened by operating the open/close lever 5, and the first needle 9a pierced through the coronary artery 57 is released once. Referring to Fig. 43e, the periphery of the distal end of the first needle 9a is pinched again by the first pinching unit 7 and the second pinching unit 8 of the treating unit 4 outside the coronary artery 57, and the first needle 9a and the thread 10 are pulled-out from the coronary artery 57 while rotating the treating unit 4, thereby completing the suture of a first needle in the state in which a part of the stoma of anastomosis 72 of the coronary artery 57 and a part of the severed portion of the internal thoracic-artery 27 are pulled-in to be jointed to each other.

Subsequently, the suture of a second needle starts in the state in which a part of the stoma of anastomosis 72 of the coronary artery 57 and a part of the severed portion of the internal thoracic-artery 27 are pulled-in to be jointed to each other. Referring to Fig. 39, the treating unit 4 is rotated again, thereby puncturing the first needle 9a to the internal thoracic-artery 27 and the coronary artery 57 similarly to the suture of the first needle. The first needle 9a is pulled-out from the internal thoracic-artery 27 and the coronary artery 57, thereby piercing the thread 10 to the internal thoracic-artery 27 and the coronary artery 57.

Referring to Figs. 41 and 42, the thread 10 anastomoses the stoma of anastomosis 72 of the coronary artery 57 and the severed portion of the internal thoracic-artery 27 by repeating the above-described operation while the thread 10 for vessel suture draws the spiral loop locus.

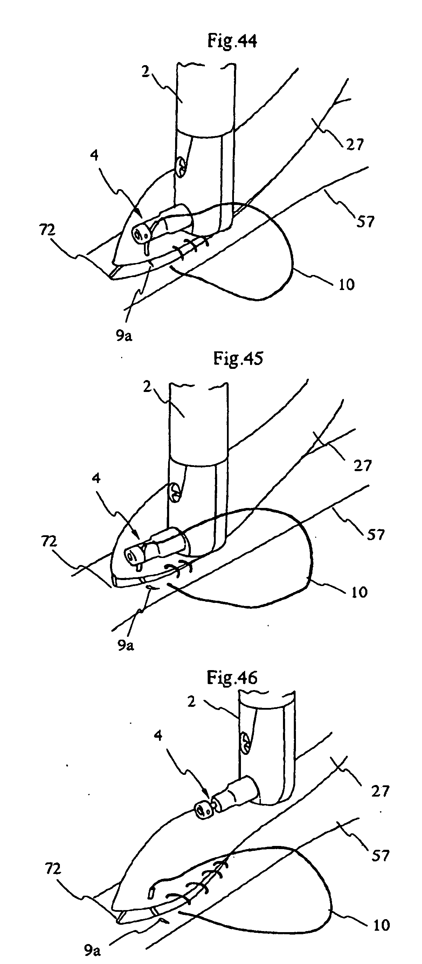

Here, a description is given of the detailed motion in the anastomosis of the treating unit 4 in the needle driver 1 in the state in which a part of the stoma of anastomosis 72 of the coronary artery 57 and a part of the severed portion of the internal thoracic-artery 27 are pulled-in to be jointed to each other with reference to Figs. 44 to 48.

Referring to Fig. 44, the rotating dial 6 is rotated in the state of pinching the first needle 9a by the treating unit 4 so that the first needle 9a is directed to the outer wall near the severed portion of the internal thoracic-artery 27, and the treating unit 4 is rotated. Then, the rotation of the treating unit 4 punctures the sharp end portion of the first needle 9a to the internal thoracic-artery 27. Referring to Fig. 45, further rotation of the rotating dial 6 punctures the sharp end portion of the first needle 9a to the outside from the inside of the coronary artery 57.

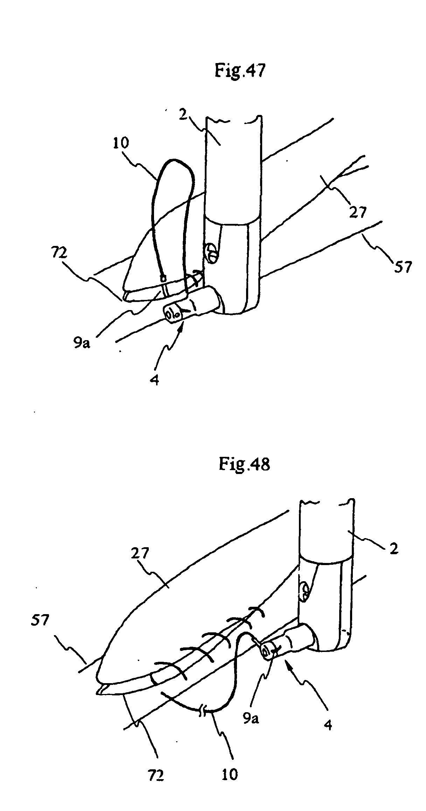

Referring to Fig. 46, the open/close lever 5 is operated (pressed), thereby opening the first pinching unit 7 and the second pinching unit 8 in the treating unit 4. Further, the first needle 9a pieced through the internal thoracic-artery 27 is released once. Referring to Fig. 47, the periphery of the distal end of the first needle 9a is pinched again by the first pinching unit 7 and the second pinching unit 8 in the treating unit 4 outside the coronary artery 57.

Referring to Fig. 48, the needle driver 1 separates the first needle 9a from the coronary artery 57, thereby piercing the thread 10 through the internal thoracic-artery 27 and the coronary artery 57.

One or two stitches before the end of the vessel anastomosis (suture) procedure of the internal thoracic-artery 27 and the coronary artery 57, in step S64 in Fig. 15, the grasping forceps 25 remaining at the coronary artery 57 pulls-out the shunt 62 and one or two stitches are additionally sutured. After that, in step S65, the thread 10 is subjected to ligation procedure.

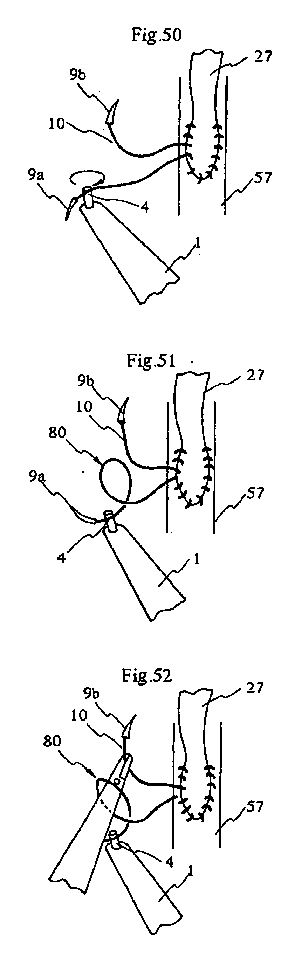

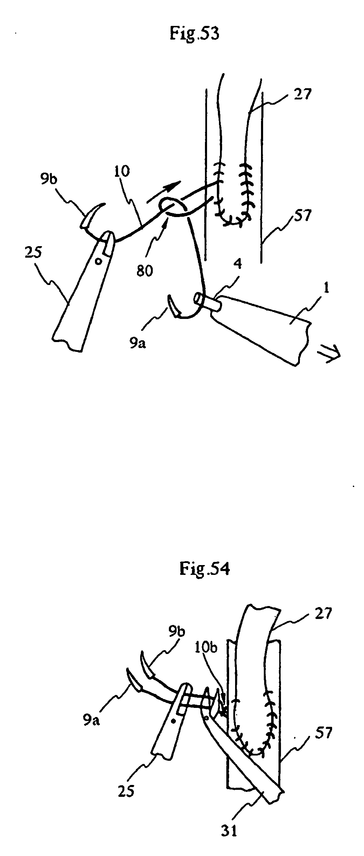

Here, the ligation procedure of the thread 10 will be described with reference to Figs. 50 to 54. Referring to Fig. 50, a thread end in the first needle 9a of the thread 10 is grasped by the first pinching unit 7 and the second pinching unit 8 and the rotating dial 6 is rotated, thereby forming a loop 80 on the end side of the thread 10. Referring to Fig. 52, the distal end of the grasping forceps 25 is pierced through the loop 80 and the grasping forceps 25 grasp the thread end of the second needle 9b. Referring to Fig. 53, the distal end of the grasping forceps 25 is pulled-out from the loop 80.

Referring to Fig. 54, a knot 10b is formed to the thread 10 by repeating the above-described operation a plurality of times. The thread connected to the first and second needles 9a and 9b are cut near the knot 10b by the surgical scissors 31. Finally, the cut thread 10 connected to the first and second needles 9a and 9b is extracorporeally collected by the grasping forceps 25.

Steps S63 to S65 correspond to the anastomosing procedure routine of the internal thoracic-artery to the coronary artery in the anastomosing procedure.

The ligation procedure of the thread 10 ends as mentioned above, thereby ending the anastomosing procedure in step S5 in Fig. 1. Referring back to Fig. 1, in step S6, various threads and devices are separated from the trocars of the ports. In step S7, a drain for keeping the negative pressure in the chest cavity is kept in the body via the port holes. After that, in step S8, the muscular coat is sutured. In step S9, the skin is sutured and the CABG using the needle driver, serving as the surgical therapeutic instrument according to the embodiment, ends.

In the CABG using the needle driver serving as the surgical therapeutic instrument according to the embodiment, the needle holding of the needle driver is kept by the energization of energizing means. Since the needle is rotated while the needle is certainly and easily held, the organ is easily and stably sutured by using the endoscope. The loop is formed easily and certainly in the ligation of suture thread by using the needle driver.

According to the embodiment, the periphery of heart is approached from above by using the trocars 22, 51, 52, and 53 for anastomosis of the internal thoracic-artery and the coronary artery as shown in Fig. 16. Alternatively, the periphery of heart may be approached from the left by the trocars 21, 22, and 23 without arranging the trocars 51, 52, and 53 for anastomosis of the internal thoracic-artery and the coronary artery and, thus, the minimally-invasive procedure is possible because another port hole is not formed. The routine for anastomosis of the internal thoracic-artery and the coronary artery in this case will be described with reference to Figs. 36 and 40.

Referring to Fig. 36, the grasping forceps 25 inserted by the trocar 21 elevates the internal thoracic-artery 27, in this state, the first needle 9a is continuously punctured from the inner wall to the outer wall of the internal thoracic-artery and further from the outer wall to the inner wall of the stoma of anastomosis 72 of the coronary artery 57 by pinching and rotating the first needle 9a with the treating unit 4 in the needle driver 1 inserted by the trocar 23, the operation for pulling-out the first needle 9a from the inner wall of the stoma of anastomosis 72 of the coronary artery 57 is repeated, and the stoma of anastomosis 72 of the coronary artery 57 is sutured to one side of the severed portion of the internal thoracic-artery 27.

Referring to Fig. 40, the treating unit 4 in the needle driver 1 pinches and rotates the second needle 9b while the stoma of anastomosis 72 of the coronary artery 57 is sutured to one side of the severed portion of the internal thoracic-artery 27. Thus, another side of the severed portion of the internal thoracic-artery 27 is sutured. That is, the second needle 9b is continuously punctured from the outer wall to the inner wall of the internal thoracic-artery and further from the inner wall to the outer wall of the stoma of anastomosis 72 of the coronary artery 57, the operation for pulling-out the second needle 9b from the inner wall of the stoma of anastomosis 72 of the coronary artery 57 is repeated, and the stoma of anastomosis 72 of the coronary artery 57 is sutured to the other side of the severed portion of the internal thoracic-artery 27. Referring to Figs. 36 and 40, the anastomosing procedure of the internal thoracic-artery and the coronary artery is performed by suturing the stoma of anastomosis 72 of the coronary artery 57 and both the sides of the severed portion of the internal thoracic-artery 27.

The needle driver according to the embodiment can be applied in the occlusion procedure of the coronary artery using the needle 91 for vessel occlusion shown in Fig. 23.

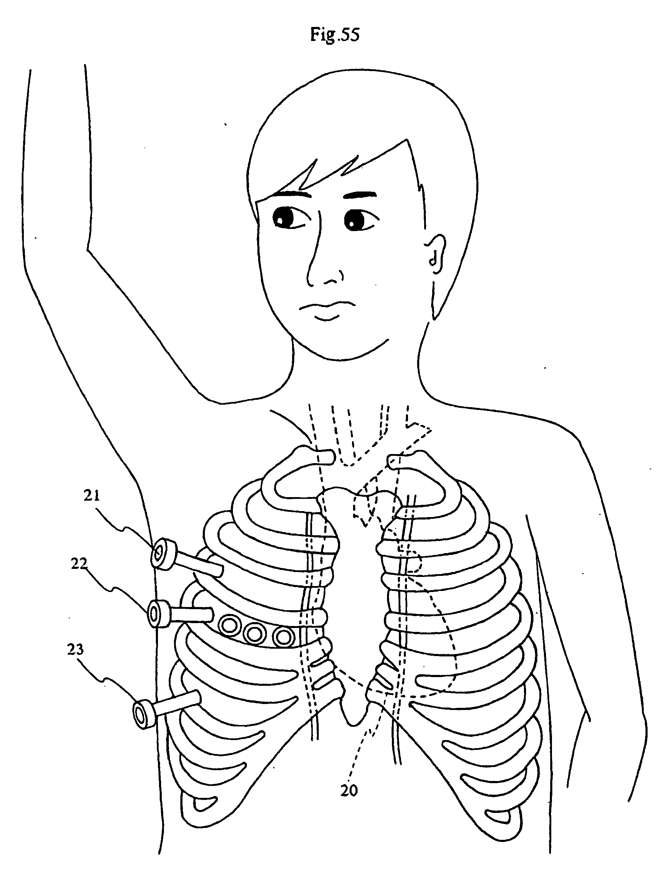

The trocars are arranged at the third, fourth, and sixth intercostal positions. However, the present invention is not limited to this. Referring to Fig. 55, in the operation for abrading the right internal thoracic-artery and anastomosing the right internal thoracic-artery, the trocars 21 to 23 are arranged at predetermined positions on the right of the chest for procedure. Obviously, the procedure according to the embodiment can be performed by arranging the trocars at proper positions corresponding to the affected parts.

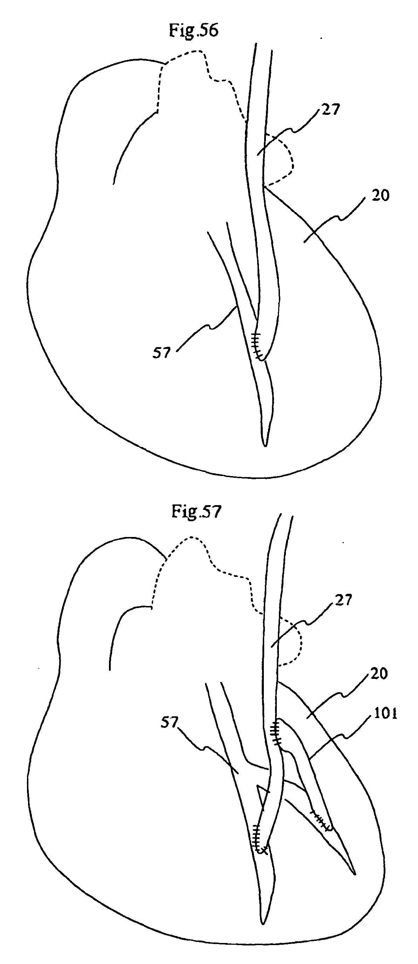

Referring to Fig. 56, a description is given of an example of bypassing, to the coronary artery 57, the severed portion on the peripheral side of a pedunculated graft such as the left internal thoracic-artery 27, serving as a bypass graft in the procedure according to the embodiment, and the present invention it not limited to this.

That is, referring to Fig. 57, the severed portion on the peripheral side of the internal thoracic-artery 27 can be bypassed to the coronary artery 57. Further, the left internal thoracic-artery 27 can be bypassed to the coronary artery 57 by a free graft 101 of the radial artery sampled from the upper arm.

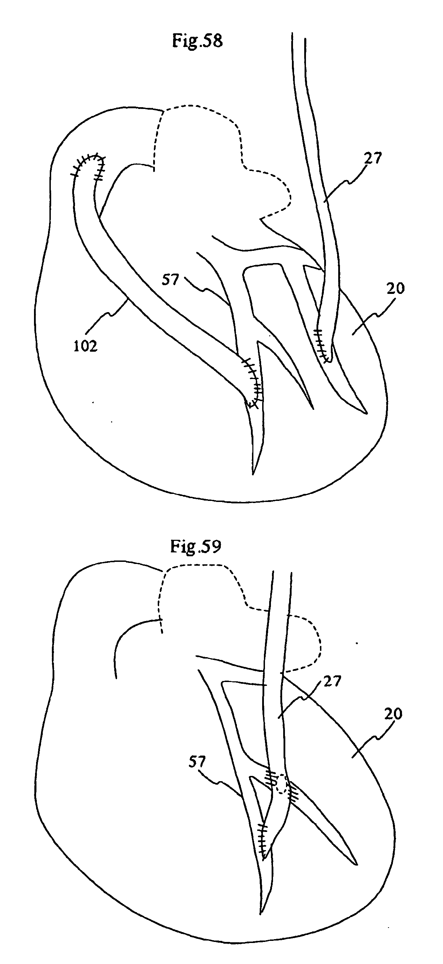

Referring to Fig. 58, the severed portion on the peripheral side of the left internal thoracic-artery 27 can be bypassed and a free graft 102 of the large saphenous vein sampled from the foot can be bypassed between the coronary artery 57 and the large artery.

Referring to Figs. 57 and 58, as the free graft 101, 102 the internal thoracic-artery whose both ends are severed, in addition to the hypogastric-wall artery may be used.

Referring to Fig. 59, the severed portion on the peripheral side of the left internal thoracic-artery 27 can be bypassed between the coronary artery 57 and the lateral wall in the halfway of the left internal thoracic-artery 27.

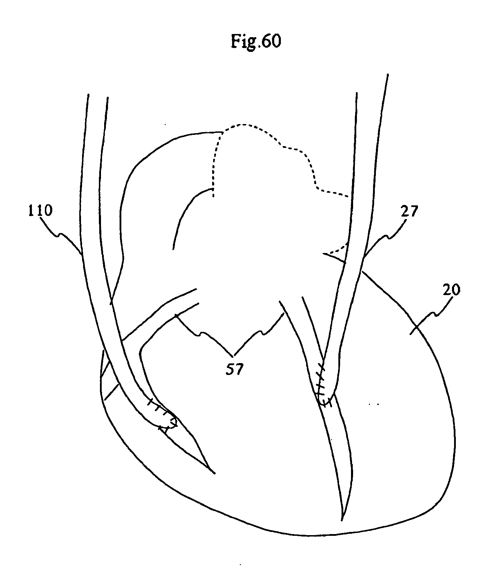

Referring to Fig. 60, the severed portion on the peripheral side of the left internal thoracic-artery 27 can be bypassed to the coronary artery 57, and the severed portion on the peripheral side of a right internal thoracic-artery 110 can be bypassed to the coronary artery 57. In this case, the procedure is performed by arranging the trocars 21 to 23 at predetermined positions on the left of the chest in the exfoliation of the internal thoracic-artery 27 as shown in Fig. 3. After that, the procedure is performed by arranging the trocars 21 to 23 at predetermined positions on the right of the chest in the exfoliation of the right internal thoracic-artery 110 as shown in Fig. 55. The anastomosing procedures shown in Figs. 56 to 60 may be combined.

According to the embodiment, the description is given of the bypass operation using the endoscope under the cardiac beat using the stabilizer. However, the present invention is not limited to this and may be applied to the CABG using the endoscope under the cardiac arrest using a cardiopulmonary pumps. Alternatively, it may be applied to the open operation using the general incision. And further it may be applied to the anastomosis of another vessel and luminal organ as well as to the CABG and further more to the suture of the parenchymatous organ, the body wall, and the skin.

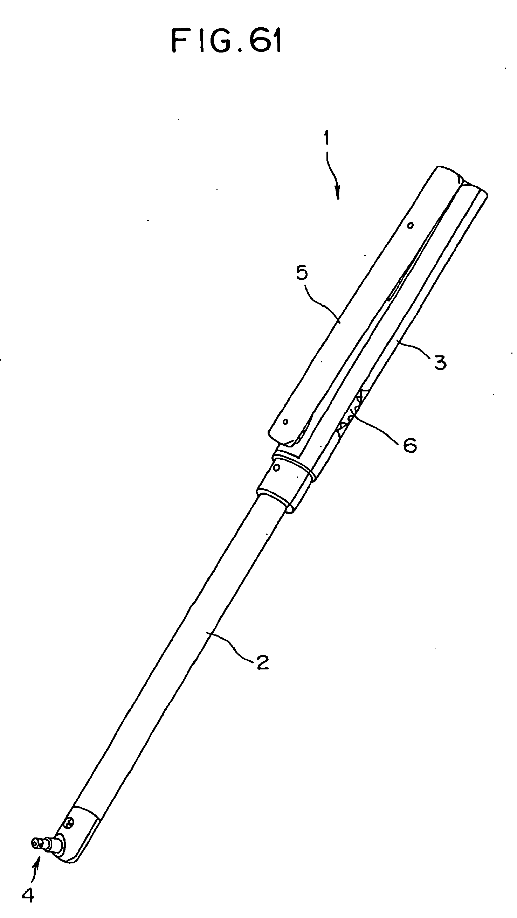

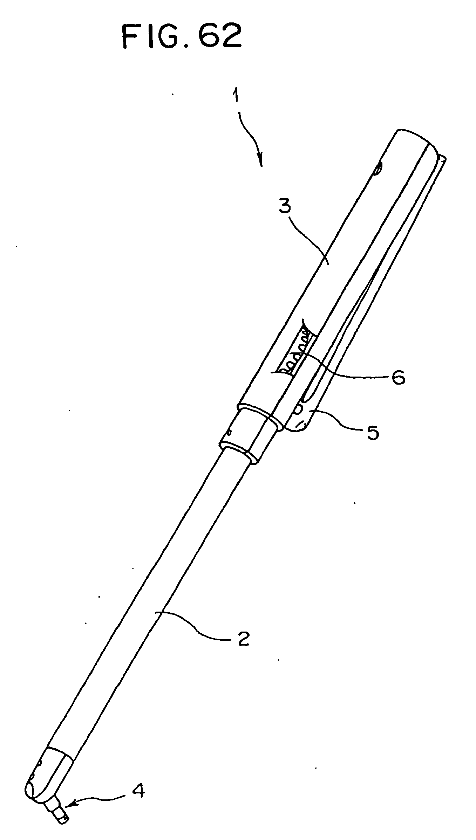

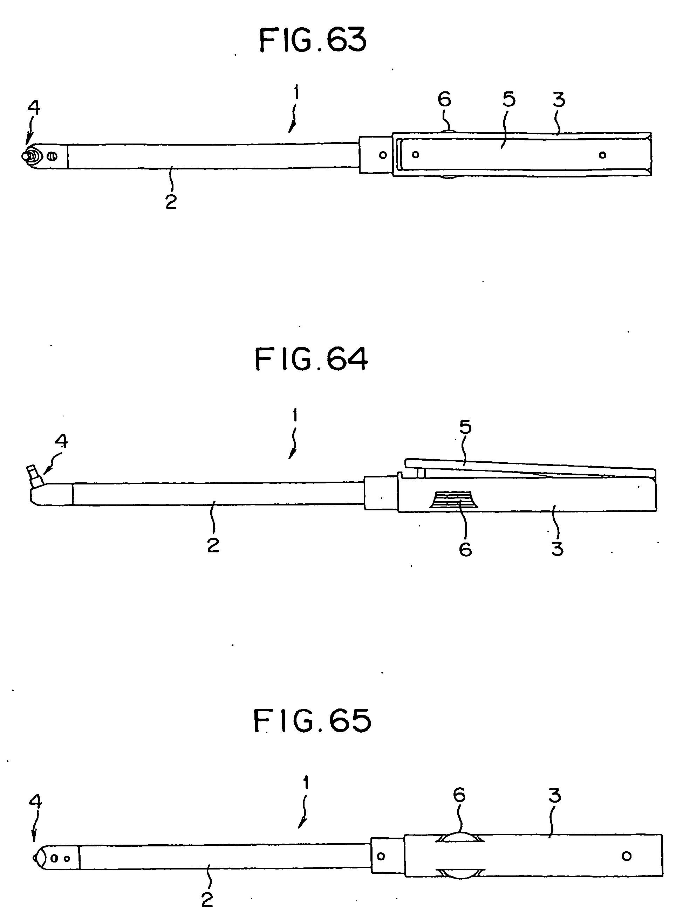

Hereinbelow, a description is given of the structure of the above-described needle driver serving as a surgical therapeutic instrument of a needle holder with reference to the drawings. Fig. 61 is a perspective view showing the needle driver according to the embodiment. Fig. 62 is a perspective view showing the needle driver as viewed from another angle of that shown in Fig. 61. Fig. 63 is a plan view showing the needle driver. Fig. 64 is a front view showing the needle driver. Fig. 65 is a bottom view showing the needle driver.

The needle driver 1 comprises the insertion unit 2, the operation unit 3 arranged to one end of the insertion unit 2, and the treating unit 4 arranged to the other end of the insertion unit 2. According to the embodiment, the insertion unit 2 is cylindrical with a predetermined length. The operation unit 3 is cylindrical with the axis matching the axis of the insertion unit 2, and is grasped by the operator's one hand for operation, which will be described later. The treating unit 4 is extended in the extending direction at a predetermined angle to the axial direction of the insertion unit 2. The operation unit 3 has the open/close lever 5 and the rotating dial 6. One end of the open/close lever 5 is axially supported to the proximal end of the operation unit 3, and the other end of the open/close lever 5 is energized in the direction separating from the outer-peripheral surface of the operation unit 3 by the energizing force of a plate spring, which will be described later.

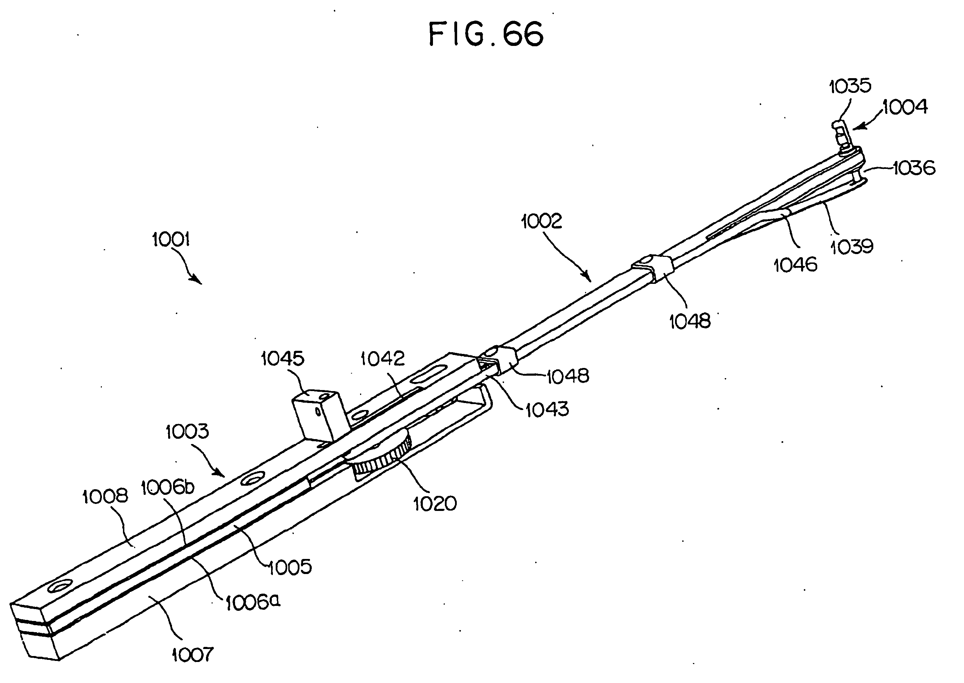

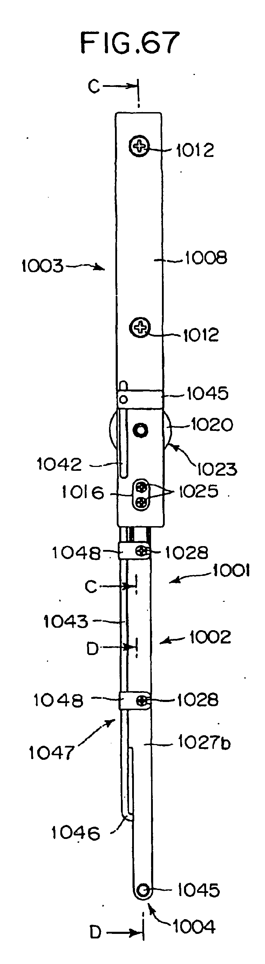

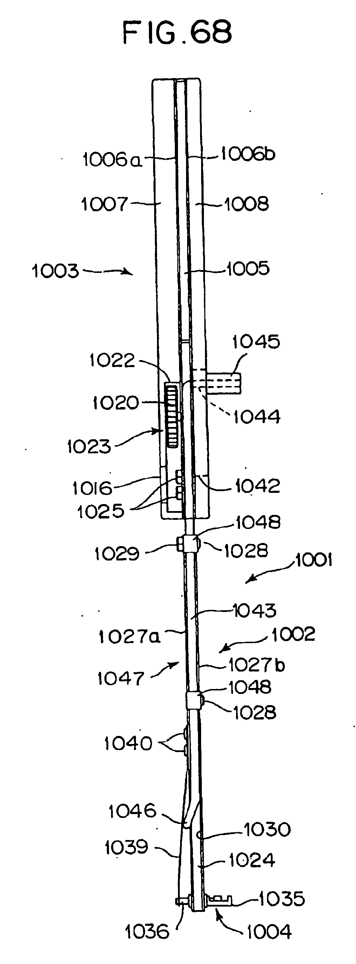

The overall structure shown in Figs. 61 to 65 according to the first modification may be the structure shown in Figs. 66 to 68. Fig. 66 is a perspective view showing a needle driver according to the first modification. Fig. 67 is a plan view showing the needle driver according to the first modification. Fig. 68 is a front view showing the needle driver according to the first modification. A needle driver 1001 according to the first modification comprises an insertion unit 1002, an operation unit 1003, and a treating unit 1004. The operation unit 1003 which is grasped for operation by operator's one hand is arranged at the proximal end of the insertion unit 1002, and the treating unit 1004 is arranged to the other end of the operation unit 1003. The operation unit 1003 comprises an open/close knob 1045 and a rotating dial 1020. With the structure according to the first modification, the operation similar to the needle driver 1 shown in Figs. 61 to 65 is performed by the operator. The numerals of the components in Figs. 66 to 68 according to the first modification will be described in detail.

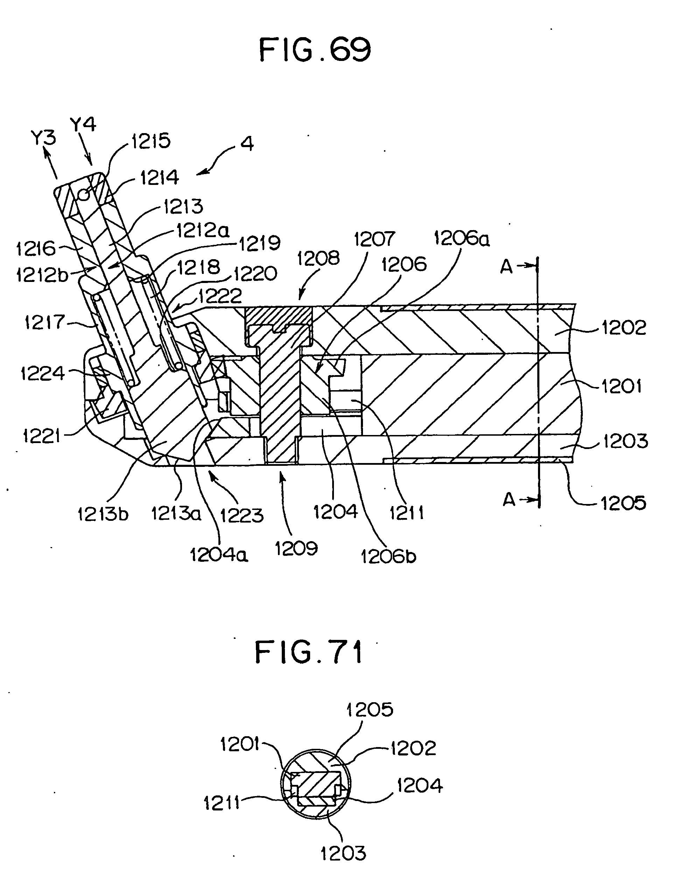

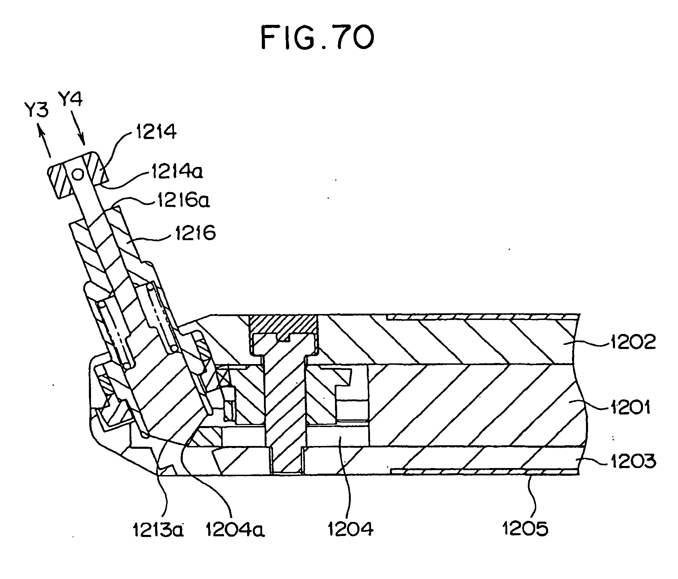

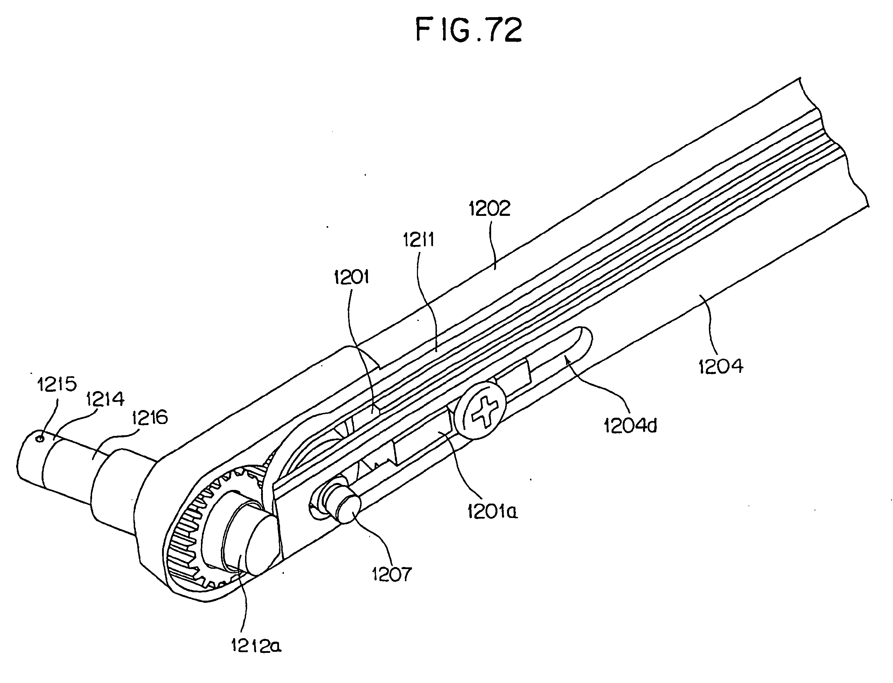

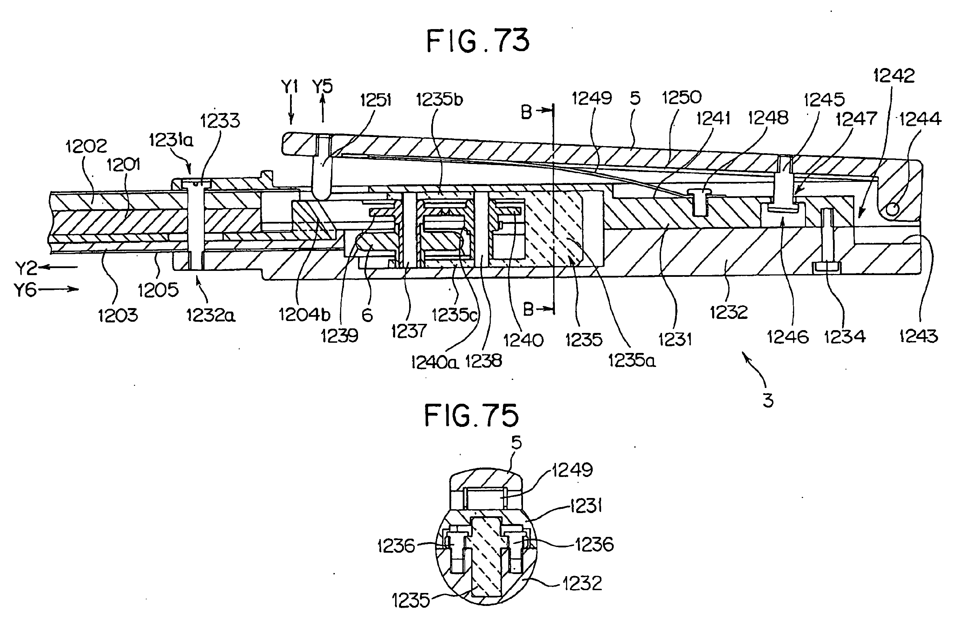

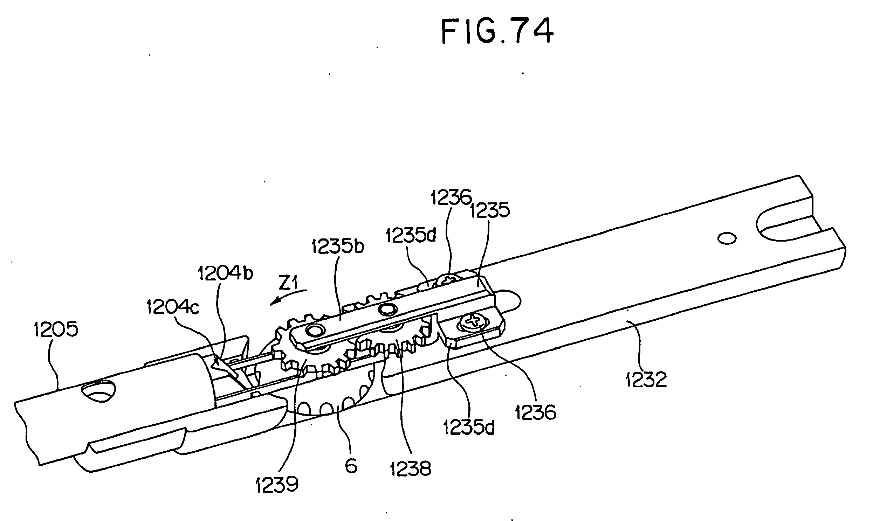

Next, a description is given of the inner structure of the needle driver 1 with reference to the drawings. First, the inner structure of the distal end of the needle driver 1 will be described. Fig. 69 is a cross-sectional view showing the distal end including a treating unit of the needle driver 1. Fig. 70 is a cross-sectional view showing the distal end upon opening a pinching portion of the treating unit of the needle driver 1. Fig. 71 is a cross-sectional view along an A-A line shown in Fig. 69. Fig. 72 is a perspective view showing the distal end including the treating unit 4 of the needle driver 1, partly excluding a distal cover, in the bottom direction. Fig. 73 is a cross-sectional view showing the proximal end including the operation unit of the needle driver 1. Fig. 74 is a perspective view showing the proximal end including the operation unit 3 of the needle driver 1, excluding a top cover, in the upper diagonal direction. Fig. 75 is a cross-sectional view along a B-B line shown in Fig. 73.

A core member 1201 is arranged in the insertion unit 2. Referring to Fig. 71, the core member 1201 is covered, from the top and bottom, with a first distal cover 1202 and a second distal cover 1203. The core member 1201 contains plastic. The first distal cover 1202 and the second distal cover 1203 contain engineering plastic. A wedge member 1204 slidable in the axial direction of the insertion unit 2 is arranged in the first distal cover 1202 and the second distal cover 1203 in the down portion of the core member 1201. The periphery of the first distal cover 1202 and the second distal cover 1203 is covered with a stainless pipe 1205, except for the distal end of the insertion unit 2. The distal end of the pipe 1205 is fixed to the first distal cover 1202, the second distal cover 1203, by an adhesive, such as an epoxy-resin-system adhesive. At the distal end of the insertion unit 2, the first distal cover 1202 and the second distal cover 1203 have hole portions 1208 and 1209 for a screw member 1207, serving as a rotating-axis member of a first rotating member 1206. The screw member 1207 fixes the first distal cover 1202 and the second distal cover 1203. At the screw head of the screw member 1207, e.g., an epoxy-resin-system adhesive is filled in the hole portion 1208. Further, an adhesive, e.g., an epoxy-resin-system adhesive is adhered at the distal end of the screw member 1207 appearing to the second distal cover 1202 side and the screw member 1207 is thus fixed to the second distal cover 1202. The first rotating member 1206 is substantially cylindrical and is rotatable around the axis of the screw member 1207 as center. The first rotating member 1206 has a bevel gear unit 1206a functioning as a bevel gear member, having a plurality of teeth at a predetermined angle to the axis of the screw member 1207 along the outer-peripheral surface. Further, the first rotating member 1206 has a pulley unit 1206b. A belt 1211 is hung to the pulley unit 1206b of the first rotating member 1206, and the rotation operating force of the rotating dial 6 is transmitted to the first rotating member 1206, which will be described later. The belt 1211 is a timing belt containing a urethane material, and the pulley unit 1206b has a groove corresponding to a groove of the timing belt.

The wedge member 1204 is a plate member arranged along the axis of the insertion unit 2 therein. Further, the wedge member 1204 has, at the distal end thereof, an inclined portion having an inclined surface 1204a at a predetermined angle to the axis of the insertion unit 2.

The treating unit 4 arranged at the distal end of the insertion unit 2 comprises two pinching members 1212a and 1212b (hereinafter, commonly referred to as 1212) forming the two pinching units 7 and 8. The first pinching member 1212a, serving as one of the two pinching members 1212 forming the pinching unit of the treating unit 4, comprises a shaft member 1213 and a circular member 1214. The shaft member 1213 and the circular member 1214 contain metal. An adhesive, e.g., an epoxy-resin-system adhesive is adhered to the distal end of the shaft member 1213, and the distal end of the shaft member 1213 is inserted in a hole of the circular member 1214 and is fixed to the circular member 1214 by a pin 1215. Both sides of the pin 1215 are fixed to the circular member 1214 by laser welding. At the end on the opposite side of the distal end of the shaft member 1213, a cylindrical portion 1213b, with the diameter larger than that of the shaft member 1213, having a conical wedge receiving portion 1213a is formed.

The second pinching member 1212b, serving as the other of the two pinching members 1212, contains cylindrical metal, and further comprises a first tube unit 1216 to which the shaft member 1213 is slidable and a second tube unit 1217 with the inner diameter larger than that of the tube 1216. The second tube unit 1217 has a coil spring 1218. One end of the coil spring 1218 comes into contact with a step portion 1219 between the first tube unit 1217 and the second tube unit 1218, and the other end of the coil spring 1218 comes into contact with a step portion 1220 between the distal end of the shaft member 1213 and the cylindrical portion 1213b. The coil spring 1218 is arranged in the second pinching member 1212b so that the shaft member 1213 is inserted in the coil spring 1218. Further, the coil spring 1218 is arranged in the second pinching member 1212b in the compressed state in the contracting direction of the coil spring 1218.

Further, the second pinching member 1212b has, on the outer circumference thereof, a bevel gear member 1221 having a plurality of teeth, which are engaged with the bevel gear unit 1206a of the first rotating member 1206. The second pinching member 1212b and the bevel gear member 1221 are fixed by an adhesive, e.g., an epoxy-resin-system adhesive. The second pinching member 1212b and the bevel gear member 1221 are slid and rotatable to the first distal cover 1202.

One of the first rotating member 1206 and the bevel gear member 1221 contains plastic or metal, and the other contains a material other than that of the one member. Alternatively, both the first rotating member 1206 and the bevel gear member 1221 may contain engineering plastic with high slidability.

A space for partly arranging the treating unit 4 is formed in the first distal cover 1202 and the second distal cover 1203. Further, a hole 1222 is formed on the first distal cover 1202 so that the treating unit 4 is projected at a predetermined angle in the axial direction of the insertion unit 2. A hole 1223 for feeding the air is formed on the second distal cover 1203 so that a conical distal end of the wedge receiving portion 1213a is punctured to the second distal cover 1203 and sterilization gas enters the insertion unit 2.

A flange unit 1224 projected in the outer-circumferential direction is formed on the outer circumference of the second tube unit 1217. The two pinching members are arranged in the space formed in the first distal cover 1202 and the second distal cover 1203 so that the flange unit 1224 comes into contact with the inner surface of the hole 1222 and the wedge receiving portion 1213a comes into contact with the inner surface of the hole 1223. Further, the teeth of the bevel gear member 1221 are arranged in the space formed in the first distal cover 1202 and the second distal cover 1203 to be engaged with the teeth of the bevel gear unit 1206a.

Referring to Fig. 72, the plate-shaped wedge member 1204 has an oblong hole 1204d which is long in the axial direction of the insertion unit 2. A projected portion 1201a is formed to the down portion of the core member 1201. A screw 1225 which has a screw head with the diameter larger than the short inner diameter of the oblong hole 1204d is screwed in the projected portion 1201a.

Therefore, referring to Fig. 72, a shaft portion, serving as a part of the screw member 1207 and the projected portion 1201a are arranged to enter the oblong hole 1204d.

Referring to Fig. 70, the wedge member 1204 is moved in the direction of the distal end of the insertion unit 2 in accordance with the opening/closing operation of the open/close lever 5, which will be described later, and the inclined surface 1204a of the inclined portion of the wedge member 1204 then presses the wedge receiving portion 1213a of the first pinching member 1212a. Therefore, the first pinching member 1212a is moved in the separating direction of the circular member 1214 from the first tube unit 1216, serving as the axial direction of the first pinching member 1212a.

Contact surfaces 1214a and 1216a with which the circular member 1214 of the first pinching member 1212a and the first tube unit 1216 of the second pinching member 1212b come into contact are subjected to slip stopper processing so as to prevent the sliding operation of the pinched needle 9. Thus, the needle 9 is certainly fixed upon being pinched. The slip stop processing may include slip stop processing using discharge processing or processing for making knurling to the contact surfaces. Alternatively, the slip stop processing may include processing for sticking the powders of diamond to the contact surfaces.

The treating unit 4 is extended in the direction at a predetermined angle to the axial direction of the insertion unit 2, serving as the direction substantially orthogonal to the plane of the contact surfaces of the circular member 1214 and the first tube unit 1216 for pinching the needle 9.

Next, the inner structure of the proximal end of the needle driver 1 will be described. Fig. 73 is a cross-sectional view showing the proximal end including the operation unit 3 of the needle driver 1. Fig. 74 is a perspective view showing the proximal end including the operation unit 3 of the needle driver 1, excluding a top cover, in the upper diagonal direction. Fig. 75 is a cross-sectional view along a B-B line shown in Fig. 73.

The operation unit 3 is entirely cylindrical, and has a first operation-unit cover 1231 and a second operation-unit cover 1232 having semi-circular cross-sections which cover the proximal end of the operation unit 3 at the distal end of the operation unit 3. The first operation-unit cover 1231 and the second operation-unit cover 1232 are fixed by two screws 1233 and 1234 via hole portions 1231a and 1232a arranged at the distal end of the operation unit 3. An epoxy-resin-system adhesive is filled and fixed to the screw heads of the screw 1233 and the screw 1234. The first operation-unit cover 1231 and the second operation-unit cover 1232 contain plastic.

Particularly, the screw 1233 at the distal end of the operation unit 3 passes through the center of the insertion unit 2, and fixes the first operation-unit cover 1231 and the second operation-unit cover 1232 so that the outer-circumferential surface of the insertion unit 2 comes into contact with the inner-peripheral surface of the distal ends of the first operation-unit cover 1231 and the second operation-unit cover 1232 of the operation unit 3 to fix the insertion unit 2.

The operation unit 3 has therein a space for arranging a base member 1235 having channel-shaped cross section along the axial direction of the operation unit 3. Referring to Fig. 74, the base member 1235 is stopped by the screw 1236 to the second operation-unit cover 1232. Specifically, the base member 1235 comprises a base portion 1235a in the middle thereof, and two arm portions 1235b and 1235c extended from both ends of the base portion 1235a. Further, a screw 1236 fixes the second operation-unit cover 1232 and an extended portion 1235d extended in the direction orthogonal to the axis of the operation unit 3, arranged in the center of the base portion 1235a.

Two pin members 1237 and 1238 arranged with a predetermined distance in the axial direction of the operation unit 3 are arranged to the two arm portions 1235b and 1235c on both sides of the base member 1235 having the channel-shaped cross-section. The two pin members 1237 and 1238 are arranged in the direction in which the axes of the two pin members 1237 and 1238 are orthogonal to the axis of the operation unit 3.

The first pin 1237 is fixed to the rotating dial 6 to function as a rotating shaft member of the rotating dial 6, serving as a rotating wheel. A first gear member 1239 is further fixed to the first pin 1237. The rotating dial 6 contains metal, such as aluminum, or plastic. The first gear member 1239 and the rotating dial 6 have a cylindrical portion in the centers thereof. The outer circumference of the cylindrical portion of the first gear member 1239 is screwed into the inner circumference of the cylindrical portion of the rotating dial 6, and the first gear member 1239 and the rotating dial 6 are fixed to each other by an adhesive such as an epoxy resin adhesive.

A second gear member 1240, serving as a second rotating member, is rotatably fixed to a second pin 1238. The second gear member 1240 has a pulley unit 1240a. The second pin 1238 functions as a rotating shaft member of the second gear member 1240. The distance between the first pin 1237 and the second pin 1238 and the diameters of the first gear member 1239 and the second gear member 1240 are set to engage the teeth of the first gear member 1239 with those of the second gear member 1240. The first gear member 1239 contains one of plastic and metal and the second gear member 1240 contains the other so that the first gear member 1239 and the second gear member 1240 are smoothly rotated in the engaged teeth of the first gear member 1239 and the second gear member 1240. Alternatively, both the first gear member 1239 and the second gear member 1240 may contain engineering plastic. The belt 1211 is hung to the pulley unit 1240a of the second gear member 1240. The surface of the pulley unit 1240a has a groove corresponding to the groove of the belt 1211, serving as a timing belt.

A groove portion 1241 is formed along the axial direction of the operation unit 3 on the outer-circumference side of the proximal end of the first operation-unit cover 1231. A notch 1242 is formed on the proximal-end side of the groove portion 1241. A groove portion 1243 is formed along the axial direction of the operation unit 3 on the first operation-unit cover 1231 side. Further, a pin 1244 is arranged to the notch 1242 on the proximal end of the first operation-unit cover 1231. The pin 1244 is fixed to both sides of the notch 1242 of the first operation-unit cover 1231.

The operation unit 3 has the open/close lever 5 containing plastic or metal, such as aluminum. One end of the open/close lever 5 is axially supported so that the open/close lever 5 is rotatable by the pin 1244.

The open/close lever 5 has a screw 1245, serving as a stopper member, on the first operation-unit cover 1231 side of the open/close lever 5 and on the proximal-end side of the operation unit 3. A hollow 1246 with the diameter corresponding to the head of the screw 1245 is formed on the second operation-unit cover 1232 side of the first operation-unit cover 1231. An epoxy-resin-system adhesive is adhered to the distal end of the screw 1245 to tightly be fixed to the open/close lever 5. The hollow 1246 has a hole 1247 with the diameter smaller than that of the head of the screw 1245. Therefore, the hole 1247 comes into contact with the head of the screw 1245 and, thus, the open/close lever 5 is not apart from the first operation-unit cover 1231 by a predetermined distance or more, that is, the excessively open state of the open/close lever 5 is prevented.

Further, one end of a metallic plate spring 1249 is fixed to the groove portion 1241 by a screw 1248. An epoxy-resin-system adhesive is adhered to the distal end of the screw 1248 to be fixed to the first operation-unit cover 1231. The plate spring 1249 has a shape for enabling the other end of the plate spring 1249 press the open/close lever 5 to be apart from the first operation-unit cover 1231 when the plate spring 1249 is fixed to the groove portion 1241. A groove portion 1250 is formed on the surface of the open/close lever 5 on the first operation-unit cover 1231 side, and the plate spring 1249 certainly presses the open/close lever 5 by the plate spring 1249 getting in the groove portion 1250.

A pusher 1251 is arranged at the distal end of the open/close lever 5 on the first operation-unit cover 1231 side. The distal end of the pusher 1251 has a spherical distal-end surface. An epoxy-resin-system adhesive is adhered to the pusher 1251 on the proximal-end side to be screwed in and fixed into the open/close lever 5.

The wedge member 1204 has, at the proximal end thereof, an inclined surface 1204b at a predetermined angle to the axial direction of the insertion unit 2. Referring to Fig. 74, the inclined surface 1204b has a V groove portion 1204c. The pusher 1251 and the wedge member 1204 are arranged so that the distal end of the pusher 1251 enters the V groove portion 1204c and the inclined surface 1204b is certainly pressed in the constant direction. A play space is arranged between the pusher 1251 and the V groove portion 1204c. The open/close lever 5 is energized in the opening direction while the play space is arranged between the pusher 1251 and the V groove portion 1204c.

Next, a description is given of the opening/closing operation and the rotating operation of the needle driver 1 with the above-described structure. First, the opening/closing operation will be described. The operator grasps the operation unit 3 by his one hand, then, presses the open/close lever 5 by, for example, the second finger in the direction of an arrow Y1 shown in Fig. 73, and the pusher 1251 presses the V groove portion 1204c of the inclined surface 1204b arranged at the proximal end of the wedge member 1204 in the operation unit 3. Since the pressed V groove portion 1204c is a part of the inclined surface 1204b, the wedge member 1204 is moved in the direction of an arrow Y2 indicating the direction of the distal end of the insertion unit 2. The inclined surface 1204a at the distal end of the wedge member 1204 presses the wedge receiving portion 1213b of the first pinching member 1212a in accordance with the movement of the wedge member 1204 in the direction Y2. The inclined surface 1204a presses the surface of the conical wedge receiving portion 1213b and is slid. Thus, the first pinching member 1212a having the pressed wedge receiving portion 1213b is moved in the separating direction (direction shown by an arrow Y3 in Figs. 69 and 70) of the contact surface 1214a of the circular member 1214 and the contact surface 1216a of the first tube unit 1216, serving as the axial direction of the first pinching member 1212a. In this case, the direction shown by the arrow Y3 corresponds to the direction orthogonal to the planes of the contact surface 1214a and the contact surface 1216a.

That is, the first pinching member 1212a, serving as one of the two pinching members 1212 having the planar contact surfaces, is moved in the direction orthogonal to the planes of the contact surface 1214a and the contact surface 1216a in accordance with the opening operation, serving as the pressing operation of the open/close lever 5. Namely, the treating unit 4 is opened.

As mentioned above, the first pinching member 1212a and the second pinching member 1212b are always energized by the coil spring 1218 in the attaching direction of the contact surface 1214a of the circular member 1214 and the contact surface 1216a of the first tube unit 1216 (direction shown by an arrow Y4 in Figs. 69 and 70), serving as the axial direction of the first pinching member 1212a. Therefore, the operator stops the pressing operation of the open/close lever 5, that is, performs the closing operation, and the open/close lever 5 is thus rotated around the axis of the pin 1244 as the center in the direction shown by an arrow Y5 shown in Fig. 73, namely, in the separating direction of the open/close lever 5 from the operation unit 3. As a consequence, the pressing force from the pusher 1251 is not applied to the wedge member 1204, and presses the inclined surface 1204a at the distal end of the wedge member 1204 in the direction shown by an arrow Y6 shown in Fig. 73, namely, in the direction of the proximal end of the operation unit 3, based on the energization force in the direction shown by an arrow Y4 of the coil spring 1218. Thus, the two pinching members 1212 are pressed to each other in the attaching direction. That is, the first pinching member 1212a, serving as one of the two pinching members 1212 having the planar contact surfaces, is moved in the direction substantially orthogonal to the planes of the contact surface 1216a and the contact surface 1214a, in accordance with the closing operation for releasing the open/close lever 5, and the treating unit 4 is closed. The two pinching members 1212 tightly pinch the needle 9.

Next, a description is given of the rotating operation of the rotating dial 6. The operator moves his finger, e.g., second finger, to the rotating dial 6 while the needle 9 is pinched by the two pinching members 1212 by the above-described opening/closing operation with the operation unit 3 grasped by his one hand, thereby rotating the rotating dial 6. With the above-described structure, the rotating angle for rotating the rotating dial 6 is 360° or more.

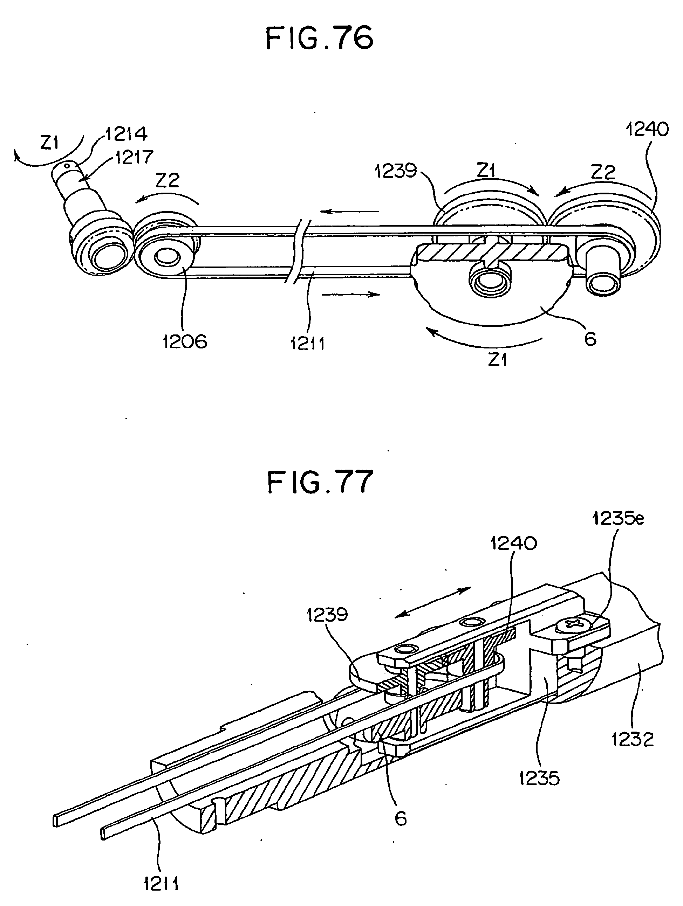

A more detailed description is given with reference to Figs. 76 and 77. Figs. 76 and 77 are explanatory diagrams for rotating operation. The rotating dial 6 is rotated in the direction of an arrow Z1 shown in Fig. 74, thereby rotating, in the direction Z1, a first gear member 1239 having a spur gear unit arranged coaxially to the rotating dial 6, as shown in Fig. 76. A second gear member 1240 is engaged with teeth of the first gear member 1239, and is rotated in a direction Z2 opposite to the direction Z1. The second gear member 1240 has a spur gear unit and a pulley unit 1240a, and the spur gear unit and the pulley unit 1240a are coaxially arranged. The rotation of the second gear member 1240 causes the rotation of the first rotating member 1206 in the distal end of the insertion unit 2 via the belt 1211. The rotating direction of the first rotating member 1206 is the same as the rotating direction Z2 of the second gear member 1240. The rotating of the first rotating member 1206 causes the rotation of the second pinching member 1212b including the second tube unit 1217. Since the first pinching member 1212a is energized to be adhered to the second pinching member 1212b, the first pinching member 1212a and the second pinching member 1212b are thus rotated together therewith. That is, the rotating force of the rotating dial 6 is transmitted to the pulley unit 1240a from the gear member, serving as a spur gear unit, is transmitted to the pulley unit 1206b from the pulley unit 1240a via the belt 1211, and is finally transmitted to the two pinching members 1212.

With the above-described structure, the rotating direction of the pinching members 1212 is the same as the direction Z1 of the rotating dial 6. Therefore, the operator rotates the needle 9 in the same direction as the rotating direction of the rotating dial 6.

Further, the gear ratio of the first gear member 1239 is equal to that of the second gear member 1240, the gear ratio of the bevel gear unit 1206a is equal to that of the bevel gear member 1221, and the diameter of the pulley unit 1206b is equal to that of the pulley unit 1240a. Thus, the rotating angle of the rotating dial 6 is equal to those of the pinching members 1212. When the gear ratio of the first gear member 1239 is not equal to that of the second gear member 1240, the gear ratio of the bevel gear unit 1206a is not equal to that of the bevel gear member 1221, or the diameter of the pulley unit 1206b is not equal to that of the pulley unit 1240a, the rotating angle of the rotating dial 6 and those of the pinching members 1212 are changed.

Referring to Fig. 76, tension is applied between the pulley unit 1206b and the pulley unit 1240a in the belt 1211 in the rotation of the rotating dial 6. Therefore, in the rotation of the rotating dial 6, friction is generated based on the tension and force of rotation by the operator is thus prevented from being too small. An extended portion 1235d of a base member 1235 has an oblong hole 1235e for passage through a screw 1236. Thus, the amount of friction is as shown in Fig. 77. Since the tension varies depending on the change in position of the screw 1236 in the oblong hole 1235e, the base member 1235 is fixed at the position of the second operation-unit cover 1232 in accordance with the desired tension.

A mechanism for adjusting the amount of friction may be arranged.

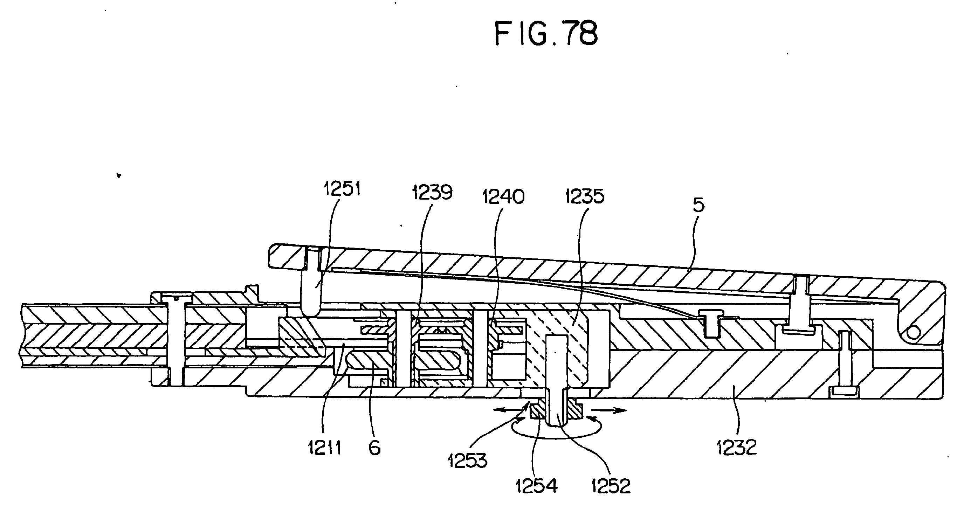

Fig. 78 is an explanatory diagram for the mechanism for adjusting the amount of friction. As mentioned above, the tension of the belt 1211, serving as the cause of the amount of friction, is determined depending on the fixing position of the base member 1235 to the second operation-unit cover 1232. Referring to Fig. 74, the extended portion 1235d of the base member 1235 is not fixed to the second operation-unit cover 1232 by the screw 1236. Referring to Fig. 78, a male screw unit 1252 projected in the outer-circumferential direction of the operation unit 3 is arranged at a base portion 1235a. Further, an oblong hole 1253 for projecting the male screw unit 1252 to the outer circumference of the operation unit 3 is formed to the second operation-unit cover. The direction of the longer inner-diameter of the oblong hole 1253 is in parallel with the axial direction of the operation unit 3. A nut 1254 screwed to the male screw unit 1252 projected from the oblong hole 1253 is screwed to the male screw unit 1252 for being tightened, thereby fixing the base member 1235 to the second operation-unit cover 1232. The base member 1235 may be fixed at any position on the longer inner-diameter of the oblong hole 1253. The position is determined depending on the desired tension, namely, desired amount of friction. Therefore, the rotation with the operator's desired friction is obtained by the rotating dial 6. In other words, the amount of friction is adjusted by a mechanism for adjusting the tension for adjusting the tension of the belt 1211.

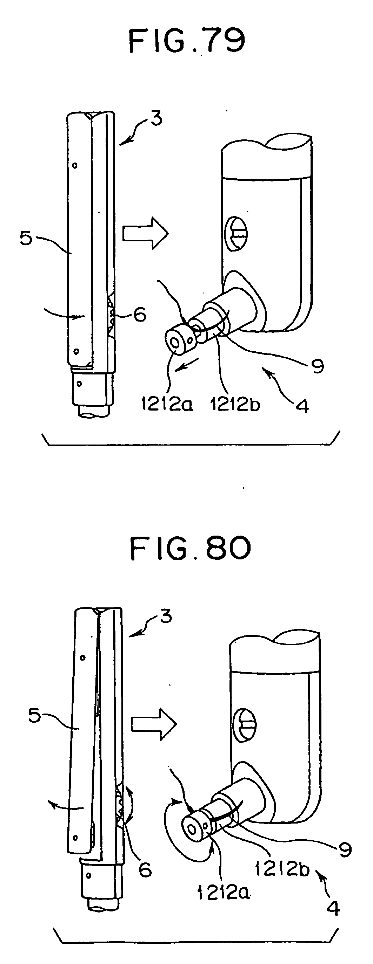

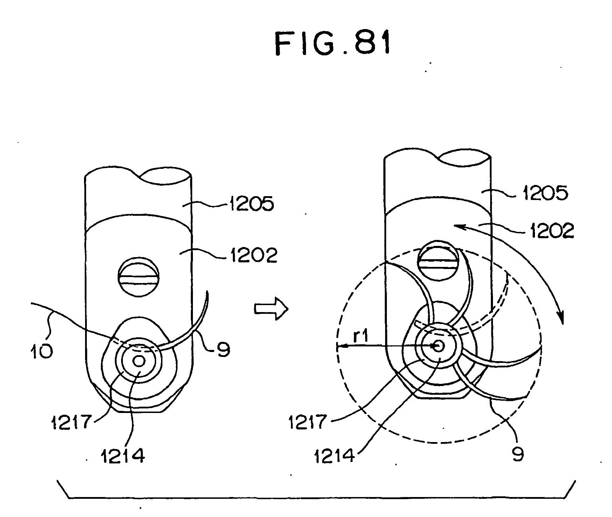

The needle driver 1 with the above-described structure has excessively high operability upon pinching the needle 9 and suturing parts. A description is given of the operation for pinching and rotating the needle 9 with reference to Figs. 79 to 81. Fig. 79 is an explanatory diagram for operation for opening the treating unit to pinch the needle. Fig. 80 is an explanatory diagram for operation for pinching and rotating the needle. Fig. 81 is an explanatory diagram for rotating the needle.

Referring to Fig. 79, the operator presses the open/close lever 5 of the operation unit 3 and, then, the first pinching member 1212a of the pinching members 1212 of the treating unit 4 is popped-up to be separated from the second pinching member 1212b. The operator moves the two pinching members 1212 between the first pinching member 1212a and the second pinching member 1212b so as to position the needle 9 at the desired pinching position in the desired pinching direction. The operator detaches his finger from the open/close lever 5 in the moving state, thereby pinching the needle 9 with the energization force of the coil spring 1218. The first and second pinching members 1212 are a single pinching unit which is always energized by the energization force of the coil spring 1218. Referring to Fig. 80, the needle 9 pinched by the two pinching members 1212 is rotated around the shaft of the shaft member 1213 in the same direction as the rotating direction of the rotating dial 6 at the same angle as the rotating angle of the rotating dial 6 by rotating the rotating dial 6. In this case, the operator can pinch the needle 9 from any direction between the shaft member 1213, serving as the umbrella portion, comprising the shaft member 1213 and the circular member 1214.

Fig. 81 is an explanatory diagram for rotating the needle 9 around the shaft of the shaft member 1213 when the needle 9 is pinched by the two pinching members 1212. In particular, Fig. 81 is a partial perspective view of the shaft member 1213 as viewed in the shaft direction of the shaft member 1213. As shown by a dotted line in Fig. 81, a radius rl of circle shown by the locus drawn by the distal end of the needle 9 is determined depending on the position of the distal end of the needle 9 when the needle 9 is pinched by the two pinching members 1212.

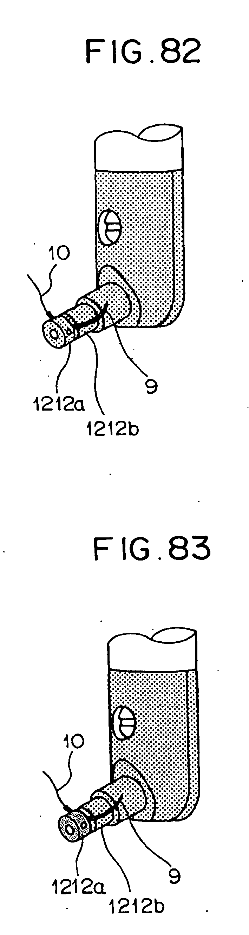

In order to improve the visibility of the needle 9 and the two pinching members 1212 upon pinching the needle 9, the surface color of the operation unit 3 may be different from the color of the needle 9. Further, the surface color of one of the pinching members 1212 may be different from the surface color of the other pinching member. Fig. 82 is a diagram showing an example of the surface color of the operation unit 3 which is different from the color of the needle 9, e.g., is blue. If the two pinching members 1212 contain metal, light is reflected to the surface and thus the halation is caused. In order to prevent the halation, the surface color of the treating unit 4 is a color which does not cause the halation. In the case shown in Fig. 82, the surface color of the distal end of the insertion unit 2 is similarly a color which does not cause the halation. Further, the color is different from that of the needle 9 so as to improve the visibility of the needle 9. Fig. 83 is a diagram showing an example of the surface color of one of the two pinching members 1212 to be different from the color of the other pinching member. In this case, the distal end of the insertion unit 2 has the color which is different from that of the needle 9, and further has the same color as that of the second pinching member 1212b of the two pinching members 1212.

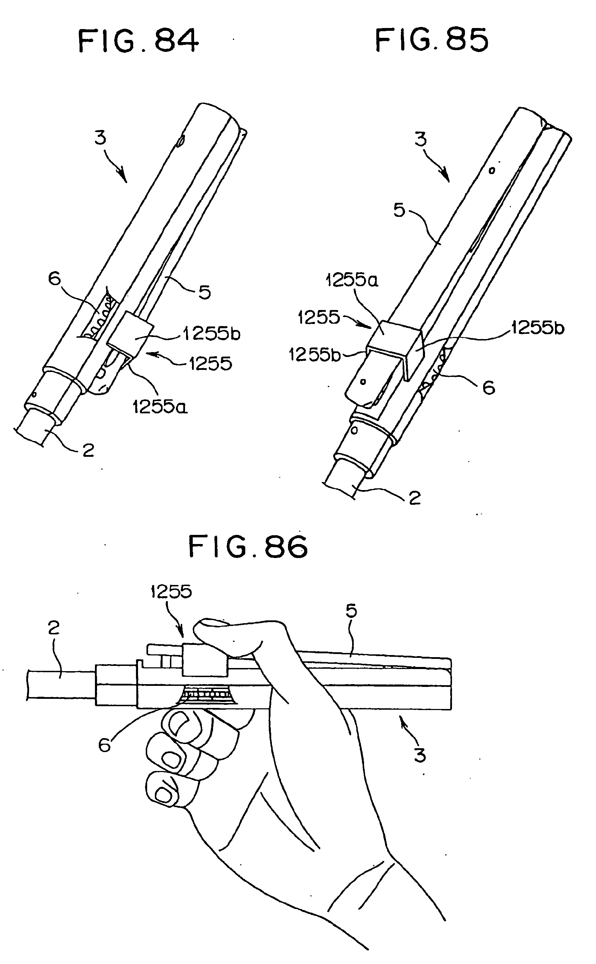

After pinching the needle 9 by the two pinching members 1212, a finger placing unit may be arranged to partly cover the open/close lever 5 upon rotating the rotating dial 6. Figs. 84 to 89 are explanatory diagrams for the structure of the finger placing unit and its using method. Fig. 84 is a perspective view showing the appearance of the operation unit 3 having the finger placing unit. Fig. 85 is a perspective view in the view different from that shown in Fig. 84. Referring to Figs. 84 and 85, a finger placing unit 1255 comprises a plate member which is channel-shaped, and two arm portions 1255b extended from both the sides of the base portion 1255a in the center of the channel shape are fixed to the outer-circumferential surface of the first operation-unit cover 1231. When the two arm portions 1255b are fixed to the outer-circumferential surface of the first operation-unit cover 1231, the height of the arm portions 1255b, or the distance of the base portion 1255a from the outer-circumferential surface of the operation unit 3 corresponds to the height in the closing state of the two pinching members 1212 when the open/close lever 5 between the two arm portions 1255b is in the base portion 1255a.

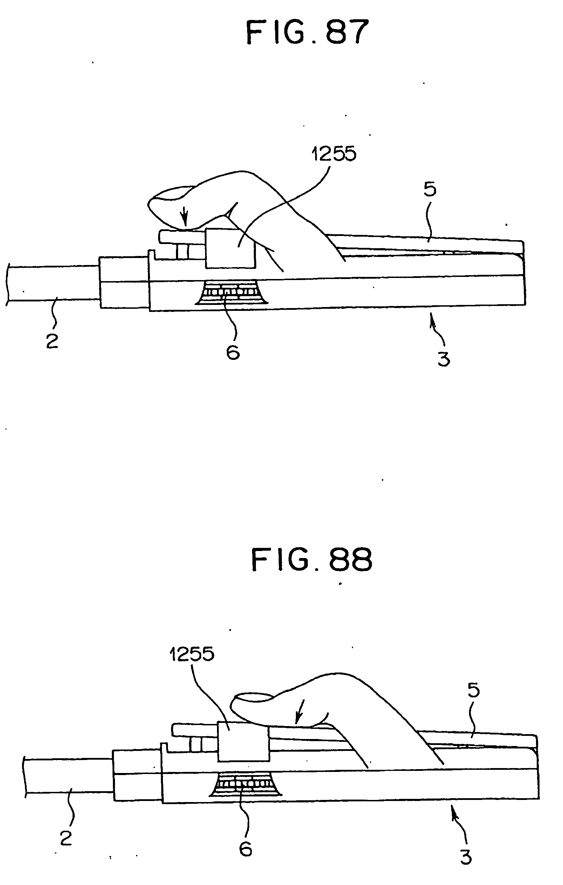

Upon rotating the rotating dial 6, the operator places the finger, e.g., first finger onto the finger placing unit 1255 and simultaneously can use another finger, e.g., second finger as shown in Fig. 86. Upon pressing the open/close lever 5, the portion which is not covered by the finger placing unit 1255 is pressed. Fig. 87 is a diagram showing a state of pressing the portion on the insertion unit 2 side, instead of the finger placing unit 1255, in the open/close lever 5. Fig. 88 is a diagram showing a state of pressing the portion on the proximal-end side of the operation unit 3, instead of the finger placing unit 1255, in the open/close lever 5. Any portion of the open/close lever 5 on the distal-end and proximal-end sides of the operation unit 3 in the finger placing unit 1255 can be pressed.

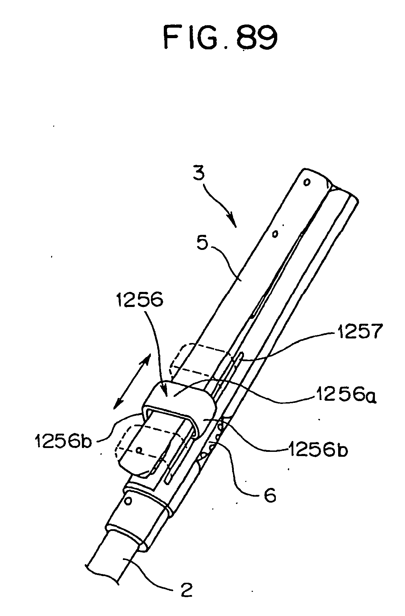

Further, the position of the finger placing unit may be changed. Fig. 89 is a perspective view showing the operation unit with the structure for changing the position. Referring to Fig. 89, the finger placing unit 1256 has a channel-shaped cross section. Two arm portions 1256b extended from both the ends of a base portion 1256a of the finger placing unit 1256 have arc-shaped cross-sections. The distal ends of the two arm portions 1256b can be engaged with two grooves 1257 arranged to the surface of the operation unit 3. The two grooves 1257 are formed on the surface of the first operation-unit cover 1231 of the operation unit 3, with predetermined lengths thereof along the axial direction of the operation unit 3. The finger placing unit 1256 can be moved along the axial direction of the operation unit 3 with predetermined lengths of the grooves 1257, and the positions of the finger placing unit 1256 are set to the operator's easily using positions.

As mentioned above, according to the embodiment, the rotating-force transmitting mechanism comprising the belt and the pulleys for rotating the first and second pinching members 1212a and 1212b of the treating unit 4 is independent of the opening/closing-power transmitting mechanism comprising the wedge members for opening/closing the contact surface 1214a and the contact surface 1216a. Therefore, even though the first and second pinching members 1212a and 1212b are greatly rotated on the right and left directions, constant pinching force is obtained without changing the pinching force for pinching the needle 9. In other words, the treating unit 4 independently keeps the closing state without any external force. That is, the rotating dial 6 is rotated, the treating unit 4 is thus rotated, any members for keeping the closing state of the treating unit 4 do not exist, and the large friction in the rotation is not generated. Therefore, the large rotating force is not necessary, and the operator can perform the rotation by a small amount of force. As a result, the operator can smoothly rotate the needle in the vessel anastomosis requiring fine operation after pinching the bent needle, and the convenience is greatly improved, as compared with the conventional treating unit. Further, the convenience is improved because the rotating angle is not limited and the operation is simple.

The contact surface 1214a and the contact surface 1216a of the first pinching member 1212a and the second pinching member 1212b and the pinching directions are in parallel with the bending direction of the needle 9. Advantageously, the needle 9 is certainly pinched at any arbitrary position in the stable state of the needle 9.

Next, a description is given of the structure for pinching and rotating the needle according to the first modification. Referring to Fig. 66, a needle driver 1001, serving as a surgical treating unit, according to the first modification comprises an operation unit 1003 which is grasped for operation by operator's one hand. One end of an insertion unit 1002 is connected to the operation unit 1003, and the other end of the insertion unit 1002 has a treating unit 1004.

Referring to Fig. 66, the operation unit 1003 has, on the proximal-end side thereof, a first fixing plate 1005 containing a belt-shaped plate member, and the first fixing plate 1005 has, on both the top and bottom surfaces thereof, pinching plates 1006a and 1006b containing, for example, stainless thin plates, which are jointed. Further, the first fixing plate 1005 has, on both the top and bottom surfaces thereof, a first grip member 1007 and a second grip member 1008 containing stripe plate members which are jointed via the pinching plates 1006a and 1006b.

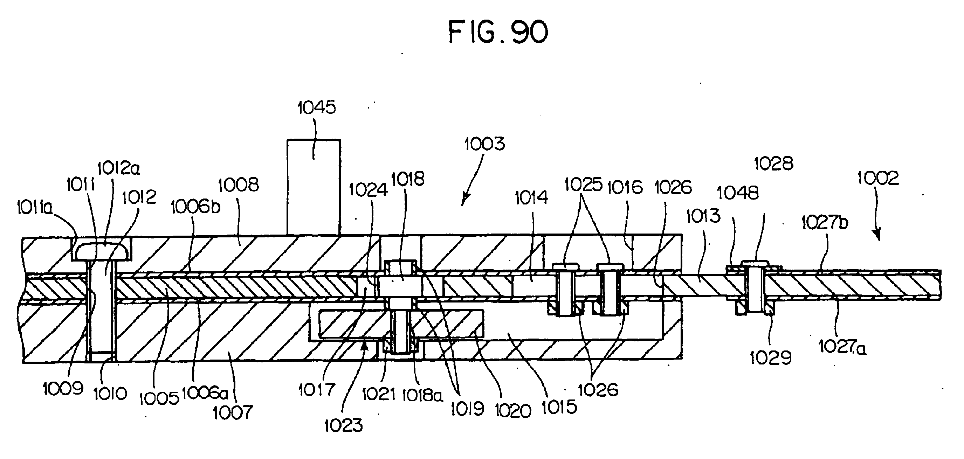

Fig. 90 is a cross-sectional view along a C-C line shown in Fig. 67. As shown in the longitudinal cross-sectional view showing the operation unit 1003 in Fig. 90, the first fixing plate 1005 including the pinching plates 1006a and 1006b comprises a plurality of through holes 1009 which are separated in the longitudinal direction. Screw holes 1010 facing the through holes 1009 are pierced through the first grip member 1007 and attaching holes 1011 facing the through holes 1009 are pierced through the second grip member 1008. A grip-member fixing screw 1012 is screwed into the screw hole 1010 from the attaching hole 1011 via the through holes 1009, and the first grip member 1007 and the second grip member 1008 are pinched and fixed to the first fixing plate 1005. The attaching hole 1011 has a caved portion 1011a for accommodating two screw heads 1012a of the grip-member fixing screw 1012.

The proximal end of a second fixing plate 1013 is arranged movably in the forward/backward direction to the distal end of the operation unit 1003 via the pinching plates 1006a and 1006b lying between the first grip member 1007 and the second grip member 1008. The second fixing plate 1013 is a strip plate member narrower than the first fixing plate 1005 and an oblong hole 1014 long in the forward/backward direction is pierced through the proximal end of the second fixing plate 1013. Further, a caved portion 1015 is arranged to the first grip member 1007 facing the oblong hole 1014 of the second fixing plate 1013 and the distal end of the first fixing plate 1005, and an opening 1016 facing the oblong hole 1014 is arranged to the second grip member 1008.

An interval 1017 is formed between the distal-end surface of the first fixing plate 1005 and the proximal-end surface of the second fixing plate 1013, and the interval 1017 has an operation pulley 1018. Shaft portions on both ends of the operation pulley 1018 are rotatable to a sliding bearing 1019, and the sliding bearing 1019 is fixed to the pinching plates 1006a and 1006b. The operation pulley 1018 has, at one shaft portion thereof, a screw shaft portion 1018a projected in the caved portion 1015 of the first grip member 1007. A nut 1021 tightens and fixes a rotating dial 1020 at the screw shaft portion 1018a.

The outer-circumferential surface of the rotating dial 1020 is partly externally projected from an opening 1022 on both sides of the first grip member 1007, and is further subjected to knurling. The finger rotates the rotating dial 1020, thereby applying the rotating force to the operation pulley 1018 via the screw shaft portion 1018a. A belt 1024 is hung to the operation pulley 1018, and the operation pulley 1018 and the belt 1024 form a rotating-force transmitting mechanism 1023 which transmits the rotating force to the treating unit 1004, which will be described later.

Further, two adjusting screws 1025 are pierced through the oblong hole 1014 from the opening 1016 of the second grip member 1008, and are arranged to the pinching plates 1006a and 1006b of the second fixing plate 1013. The adjusting screws 1025 are screwed to an adjusting nut 1026. Therefore, the second fixing plate 1013 advances and returns within the range of the oblong hole 1014 by loosening the adjusting screw 1025, and the tension of the belt 1024 is adjusted in the assembling.

Referring to Fig. 68, pinching plates 1027a and 1027b containing, for example, stainless thin plates are arranged on both the top and bottom surfaces of the second fixing plate 1013 forming the insertion unit 1002. The pinching plates 1027a and 1027b are fixed to the second fixing plate 1013 by a plurality of plate-stop screws 1028 and a nut 1029. The pinching plates 1027a and 1027b are slightly wider than the second fixing plate 1013, and a belt guiding mechanism 1030 is arranged to both sides of the second fixing plate 1013 to freely advance and return the belt 1024 and prevent the movement of the belt 1024 in the width direction. The thickness of the second fixing plate 1013 is substantially equal to the width of the belt 1024.

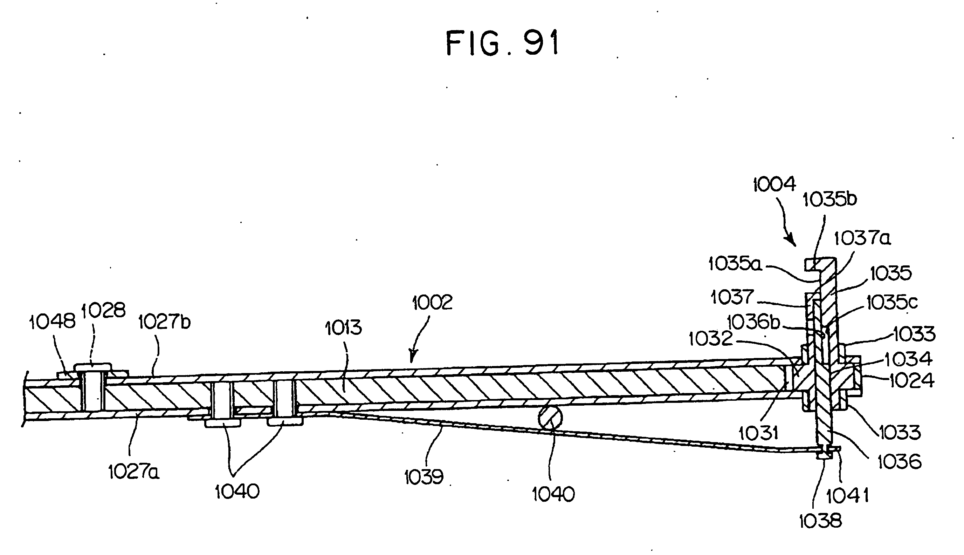

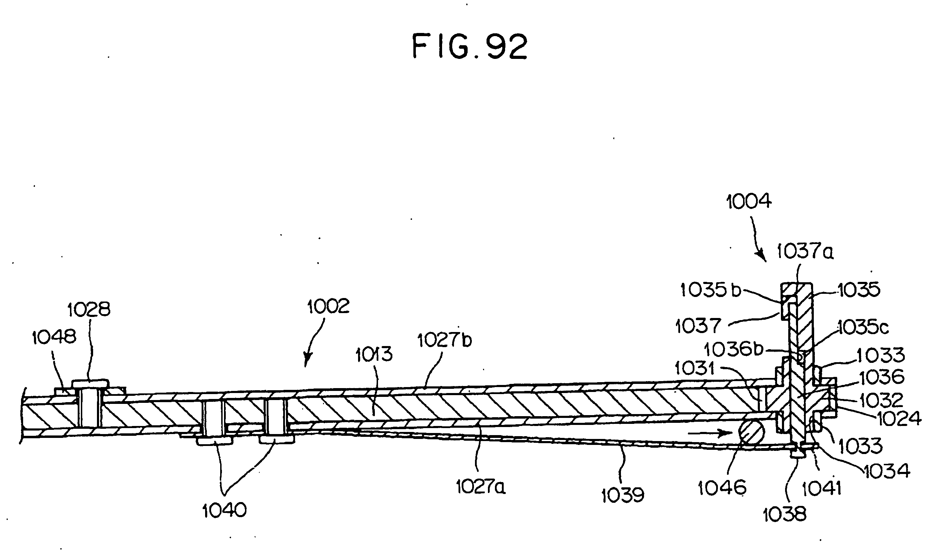

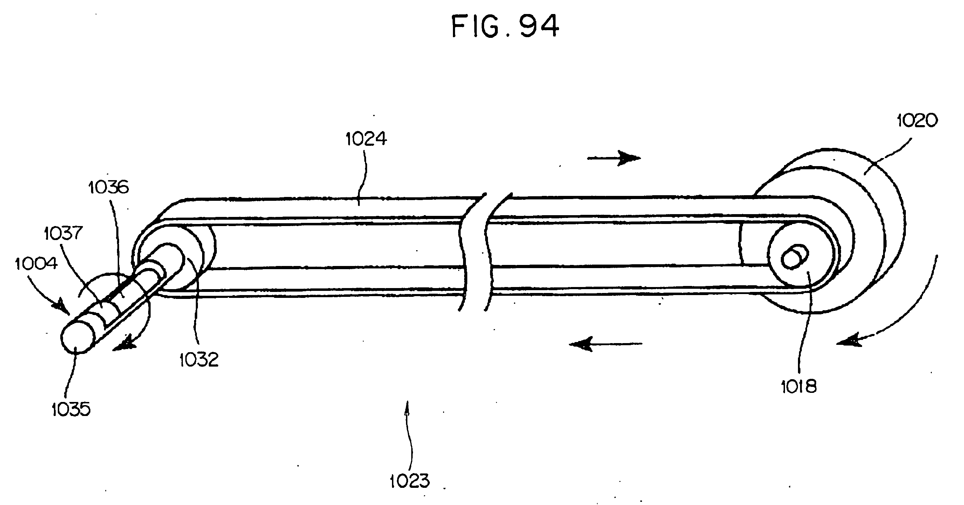

Next, a description is given of the structure of the treating unit 1004 with reference to Figs. 91 to 95. That is, the distal end of the second fixing plate 1013 is shorter than the pinching plates 1027a and 1027b, and a space 1031 is arranged at the distal end of the second fixing plate 1013. A rotating pulley 1032 is arranged to the space 1031. A central axis O of the rotating pulley 1032 is perpendicular to the insertion unit 1002, and shaft portions on both ends of the rotating pulley 1032 are rotatable to a sliding bearing 1033. The sliding bearing 1033 is fixed to the pinching plates 1027a and 1027b. Referring to Fig. 94, the belt 1024 is hung to the rotating pulley 1032, and the rotating-force transmitting mechanism 1023 is structured by transmitting the rotation of the operation pulley 1018 to the rotating pulley 1032 by the belt 1024. The belt 1024 is a toothed timing belt, thereby certainly transmitting motive power without any slip.

A through-hole 1034 is arranged to the central axis O of the rotating pulley 1032 in the axial direction thereof. A hook member (hereinafter, referred to as a first jaw) 1035, serving as a first pinching member, pierced through the pinching plate 1027b and projected to the side, is integrally arranged at one shaft portion of the rotating pulley 1032 and at the deviated position from the through-hole 1034. The first jaw 1035 is cylindrically stick-shaped, and the central axis of the first jaw 1035 in the long-axis direction is perpendicular to the axial line of the insertion unit 1002. The first jaw 1035 is rotatable to the central axis. Further, the first jaw 1035 has, substantially in the middle thereof in the longitudinal direction, a planar surface 1035a substantially matching the central axis O of the first jaw 1035. In addition, the first jaw 1035 has, at the distal end thereof, a pinching surface 1035b orthogonal to the central axis in the long-axis direction.

A substantially cylindrical member (hereinafter, referred to as a second jaw) 1036, serving as a part of the second pinching member, movable in parallel with the central axis of the first jaw 1035, is fit into the through-hole 1034 of the rotating pulley 1032. The second jaw 1036 is cylindrically stick-shaped, and a pinching piece 1037 movable to the pinching surface 1035b of the first jaw 1035 is fixed at the distal end of the second jaw 1036 (hereinafter, the member 1036 and the pinching piece 1037 are referred to as second jaws). A pinching surface 1037a facing the pinching surface 1035b of the first jaw 1035 is arranged to the pinching piece 1037.

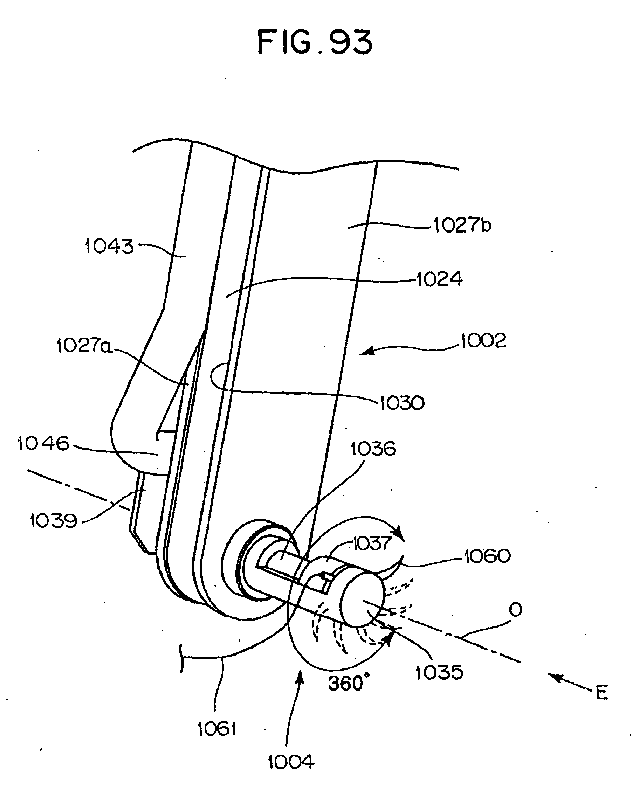

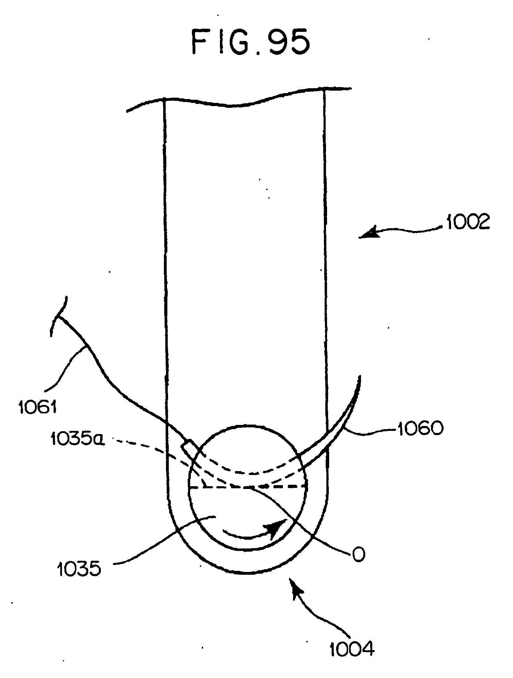

Referring to Figs. 93 and 95, the pinching surfaces 1035b and 1037a of the first jaw 1035 and the second jaw 1036 and the pinching directions are in parallel with the bending direction of a bent suture needle (hereinafter, simply referred to as a needle) 1060, to certainly pinch the needle 1060 at any arbitrary position in the stable state. Further, the pinching surfaces 1035b and 1037a have coarse surfaces formed by electrodepositing small particles of diamond in order to stably pinch the needle without displacement upon pinching the needle 1060. The pinching surfaces 1035b and 1037a may be electrodeposited with small particles of ruby or sapphire as well as diamond. Further, the pinching surfaces 1035b and 1037a may have coarse surfaces by discharge processing or etching. Furthermore, the pinching surfaces 1035b and 1037a may be formed by adhering another cemented material through knurling processing.

A notch portion 1035c with steps and a notch portion 1036b with steps are arranged symmetrically with respect to a point to a joint surface between the first jaw 1035 and the second jaw 1036 having a guide function for sliding and advancing and returning the second jaw 1036 to the first jaw 1035. The proximal end of the second jaw 1036 is pieced through the pinching plate 1027a and is projected to the side, and has a thin-diameter portion 1038 at the end thereof. A plate spring 1039, serving as energizing means, containing, for example, stainless metal is connected to the thin-diameter portion 1038, and enables the pinching surface 1037a of the second jaw 1036 to come into contact with the pinching surface 1035b of the first jaw 1035 to energize the pinching surfaces 1035b and the 1037a.

The proximal end of the plate spring 1039 is fixed to the halfway of the second fixing plate 1013 forming the insertion unit 1002 by a plurality of fixing screws 1040 via the pinching plate 1027a. An engaging unit 1041 engaged with the thin-diameter portion 1038 of the second jaw 1036 is arranged to the other end of the plate spring 1039.

Referring to Figs. 66 to 68, a guiding groove 1042 long in the forward/backward direction is arranged at the position deviated to one side on the top of the second grip member 1008 forming the operation unit 1003. An L-shaped bending portion 1044 formed at the proximal end of a transmission rod 1043 is inserted in the guiding groove 1042. Therefore, the proximal end of the transmission rod 1043 is pierced through the guiding groove 1042, and is projected to the upper portion of the second grip member 1008. A closing knob 1045 advanced/returned by the operator's finger is fixed to the projected portion.

The transmission rod 1043 is, for example, stainless stick-shaped member, is extended near to the treating unit 1004 along the second fixing plate 1013 forming the insertion unit 1002, and is supported to be movable in the axial direction. An L-shaped bending portion 1046 is arranged at the distal end of the transmission rod 1043. The bending portion 1046 is inserted between the pinching plate 1027a and the plate spring 1039, and forms an opening/closing-force transmitting mechanism 1047 which operates the second jaw 1036 by elastically modifying the plate spring 1039. Rod holding fittings 1048 are tightened to the plate-stop screws 1028 which fixes the pinching plates 1027a and 1027b to the second fixing plate 1013, and the rod holding fittings 1048 hold the transmission rod 1043 movably in the axial direction.

Therefore, when the closing knob 1045 returns the transmission rod 1043, the bending portion 1046 is moved to the proximal end of the plate spring 1039, and the distal-end side of the plate spring 1039 is pressed and widened in the separating direction from the pinching plate 1027a against the energization force of the plate spring 1039. When the closing knob 1045 advances the transmission rod 1043, the bending portion 1046 is moved on the distal-end side of the plate spring 1039, the binding force to the plate spring 1039 is reset, and the energization force of the plate spring 1039 enables the distal-end side of the plate spring 1039 to be pulled-in in the close direction of the pinching plate 1027a.

The second jaw 1036 connected to the engaging unit 1041 of the plate spring 1039 is moved in parallel with the axial direction of the first jaw 1035, the pinching surface 1035b and the pinching surface 1037a are opened/closed, and the needle 1060 is pinched/reset. Referring to Figs. 93 and 95, the needle 1060 is arcuately bent, a sharp end is arranged at one end of the needle 1060, and a threader unit is arranged at the other end of the needle 1060. A suture thread 1061 is connected to the threader unit. The needle 1060 is pinched by the pinching surfaces 1035b and 1037a of the first jaw 1035 and the second jaw 1036 in the parallel with the arcuate direction of the needle 1060. Further, a projected arcuate portion of the needle 1060 is in contact with the planar surface 1035a of the first jaw 1035 and then is pinched. That is, since the needle 1060 is pinched at the position substantially matching the central axis O of the first jaw 1035, when the first jaw 1035 and the second jaw 1036 are rotated around the central axis O as center, the needle 1060 is rotated around the central axis O of the first jaw 1035 as general center.

With the above-described structure of the surgical treating unit 1001, the operator grasps the operation unit 1003 by his one hand, the first finger is placed to the closing knob 1045, and the second finger is placed to the rotating dial 1020. The rotating dial 1020 is rotated, thereby rotating the operation pulley 1018. The rotation of the operation pulley 1018 is transmitted to the rotating pulley 1032 via the belt 1024. Therefore, the first jaw 1035 and the second jaw 1036 are rotated around the central axis O integrally with the rotating pulley 1032.

When the closing knob 1045 is returned along the guiding groove 1042, the bending portion 1046 of the transmission rod 1043 is moved to the proximal-end side of the plate spring 1039. The distal-end side of the plate spring 1039 is pressed and widened in the separating direction from the pinching plate 1027a against the energization force of the plate spring 1039. Therefore, the second jaw 1036 is moved to be separated from the first jaw 1035, and the pinching surface 1037a of the second jaw 1036 is apart from the pinching surface 1035b of the first jaw 1035 and is opened.

The closing knob 1045 advances the transmission rod 1043, thereby moving the bending portion 1046 to the distal-end side of the plate spring 1039. The binding force to the plate spring 1039 is reset, and the distal end of the plate spring 1039 is pulled-in in the close direction of the pinching plate 1027a by the energization force of the plate spring 1039. Therefore, the second jaw 1036 is moved to the first jaw 1035, and the pinching surface 1037a of the second jaw 1036 is in contact with the pinching surface 1035b of the first jaw 1035 and is closed. Further, even though the finger is released from the closing knob 1045, the pinching surface 1035b and the pinching surface 1037a are in contact with each other and are closed by the energization force of the plate spring 1039. Thus, the needle 1060 is not carelessly pulled-out.

The rotating-force transmitting mechanism 1023 for rotating the first and second jaws 1035 and 1036 is independent of the opening/closing-force transmitting mechanism 1047 for opening/closing the pinching surfaces 1035b and the 1037a. Thus, even when the first and second jaws 1035 and 1036 are greatly rotated in the horizontal direction, the pinching force for pinching the needle 1060 is not changed and constant pinching force is obtained. The similar operation is possible, with the first finger placed to the rotating dial 1020, the second finger placed to the closing knob 1045.

With the above-described structure according to the first modification, the treating unit 1004 is perpendicular to the insertion unit 1002. Therefore, the treating unit 1004 is easily approached to the target portion diagonally or laterally, thereby improving the operability. The rotating-force transmitting mechanism 1023 for rotating the first and second jaws 1035 and 1036 of the treating unit 1004 is independent of the opening/closing-force transmitting mechanism 1047 for opening/closing the pinching surfaces 1035b and 1037a. Therefore, even when the first and second jaws 1035 and 1036 are greatly rotated in the right and left directions, the pinching force for pinching the needle 1060 is not changed and constant pinching force is obtained. Since the rotating angle is not limited, the convenience is preferable, the operation is simple, and it is convenient.

The pinching surfaces 1035b and 1037a of the first jaw 1035 and the second jaw 1036 and the pinching directions are in parallel with the bending direction of the needle 1060. Advantageously, the needle 1060 is certainly pinched at any arbitrary position in the stable state.

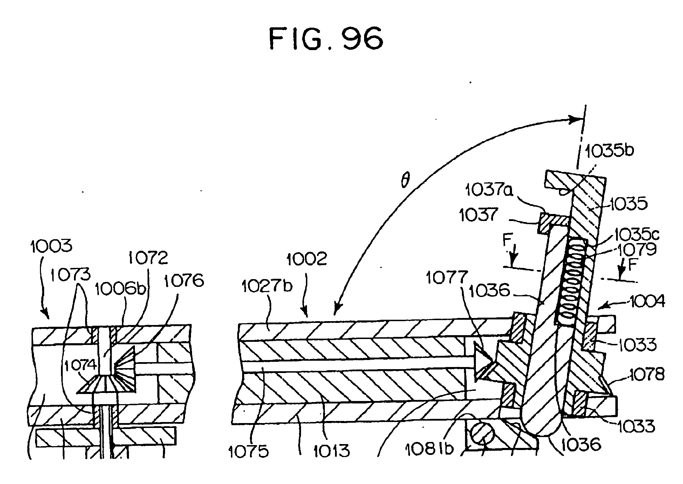

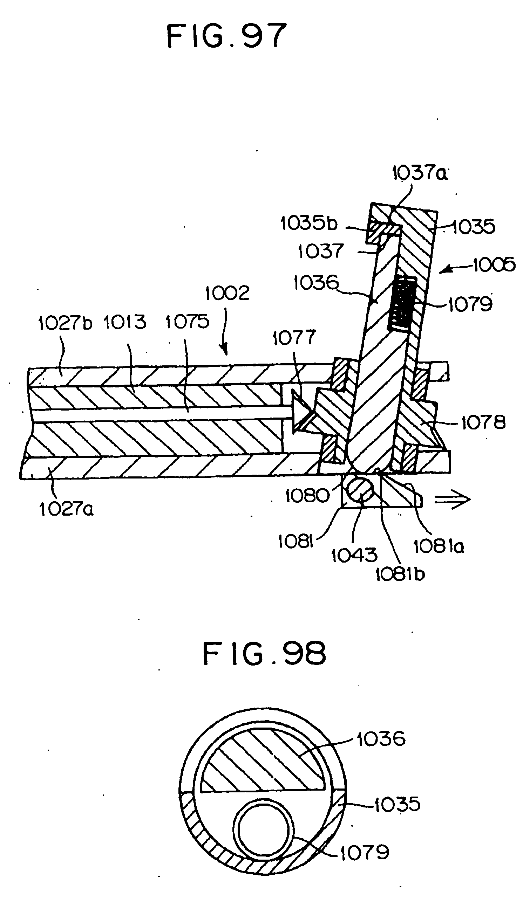

Figs. 96 to 98 show the second modification. Fig. 96 is a longitudinal side view showing the first and second jaws which are opened. Fig. 97 is a longitudinal side view showing the first and second jaws which are closed. Fig. 98 is a cross-sectional view along an F-F line shown in Fig. 96. The same components in Figs. 96 to 98 as those according to the first modification are designated by the same reference numerals, and a description thereof is omitted.

A space portion 1071 is arranged between the pinching plates 1006a and 1006b of the operation unit 1003. A driving shaft 1072 is arranged to be pierced through the space portion 1071. Both ends of the driving shaft 1072 are rotatably supported to a sliding bearing 1073 arranged to the pinching plates 1006a and 1006b. One end of the driving shaft 1072 is externally projected from the pinching plate 1006a, and the rotating dial 1020 is fixed to the projected portion by the nut 1021. Further, a driving bevel gear 1074 containing resin is fit to the middle portion of the driving shaft 1072.

A transmission shaft 1075 rotatably supported to the second fixing plate 1013 is arranged in the longitudinal direction of the insertion unit 1002. A first drive-transmission bevel gear 1076 engaged with a driving bevel gear 1074 is arranged at the proximal end of the transmission shaft 1075. The distal end of the transmission shaft 1075 is extended to the inside of the treating unit 1004, and a second drive-transmission bevel gear 1077 is arranged at the distal end of the distal end of the transmission shaft 1075.

A resin driven bevel gear 1078 engaged with the resin second drive-transmission bevel gear 1077 is accommodated in the space 1031 of the treating unit 1004. The driven bevel gear 1078 is inclined to the insertion unit 1002, and an angle θ formed by the central axis O of the driven bevel gear 1078 and the core of the insertion unit 1002 in the longitudinal direction is obtuse (θ > 90°). Further, the shaft portion of the driven bevel gear 1078 is at a predetermined angle to the direction orthogonal to the axis of the insertion unit 1002, and is rotatably supported to a sliding bearing 1033 inclined to the pinching plates 1027a and 1027b.

In addition, a coil spring 1079, serving as energizing means, is accommodated between the notch portion 1035c with steps and the notch portion 1036b with steps formed to the joint surface of the first jaw 1035 and the second jaw 1036. The coil spring 1079 energizes the pinching surface 1037a of the second jaw 1036 in the direction for opening the pinching surfaces 1035b and 1037a apart from the pinching surface 1035b of the first jaw 1035.

A semi-spherical portion 1080 is formed at the proximal end of the second jaw 1036. The semi-spherical portion 1080 is jointed a pressing member 1081 having a taper surface 1081a and a planar surface 1081b arranged at the distal end of the transmission rod 1043 that freely advances and returns by the closing knob 1045.

According to the second modification, the finger rotates the rotating dial 1020, thereby rotating the driving bevel gear 1074 via the driving shaft 1072. The rotation of the driving bevel gear 1074 is transmitted to the transmission shaft 1075 via the first drive-transmission bevel gear 1076, and the driven bevel gear 1078 is rotated via the second drive-transmission bevel gear 1077. Thereby, the first jaw 1035 and the second jaw 1036 integral with the driven bevel gear 1078 are rotated. The transmission rod 1043 is advanced by the closing knob 1045 from the state shown in Fig. 96 and then the taper surface 1081a of the pressing member 1081 presses up the second jaw 1036 while the taper surface 1081a is slid together with the semi-spherical portion 1080 of the second jaw 1036. Thereby, the coil spring 1079 is compressed and, thus, the pinching surfaces 1035b and 1037a of the first jaw 1035 and the second jaw 1036 are gradually close to each other. A planar surface 1081b of the pressing member 1081 comes into contact with the semi-spherical portion 1080 of the second jaw 1036 and, then, the pinching surface 1035b comes into contact with the pinching surface 1037a as shown in Fig. 97, thereby setting the closing state. Even when the finger is released from the closing knob 1045, the pinching surfaces 1035b and 1037a are kept to be closed because the planar surface 1081b of the pressing member 1081 comes into contact with the semi-spherical portion 1080 of the second jaw 1036.

When the closing knob 1045 returns the transmission rod 1043, the planar surface 1081b of the pressing member 1081 is evacuated from the semi-spherical portion 1080 of the second jaw 1036 to be slid to the taper surface 1081a. Thus, the energization force of the coil spring 1079 presses down the second jaw 1036. Thereby, referring to Fig. 96, the pinching surfaces 1035b and 1037a of the first jaw 1035 and the second jaw 1036 are opened.

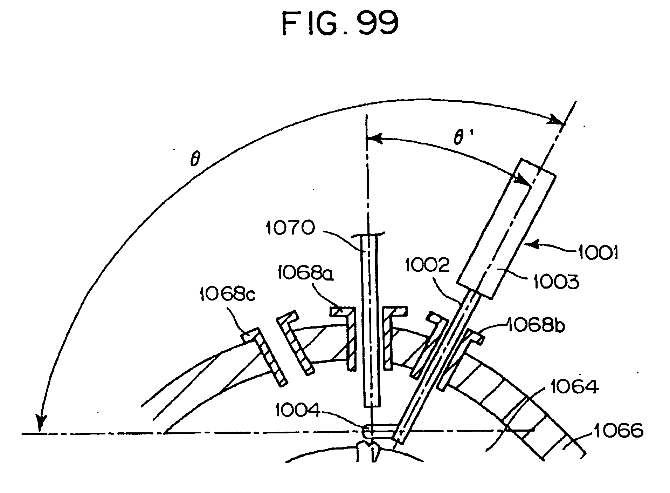

Fig. 99 shows the using state of the surgical treating unit according to the second modification. A surgical treating unit 1001 is structured by obtusely arranging the treating unit 1004 to the insertion unit 1002. Therefore, even upon inserting the insertion unit 1002 of the surgical treating unit 1001 into a chest cavity 1064 from a trocar 1068b at an angle θ' to a coronary artery 1065, the treating unit 1004 is horizontally kept to the tangential surface of the coronary artery 1065.

Therefore, it is possible to improve the anastomosis operation of the treating unit 1004 in the directions of 12 o'clock and 6 o'clock to the stoma of anastomosis of the coronary artery 1065. Further, the rotation of the rotating dial 1020 as power is transmitted to the treating unit 1004 by a gear mechanism, thereby improving the operability without any loss of the power transmission.

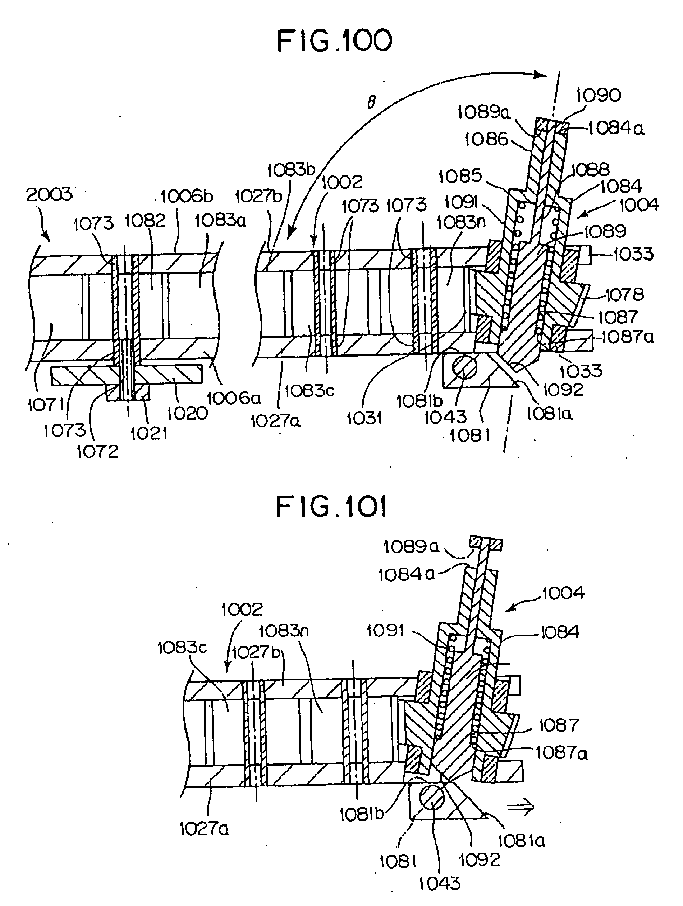

Figs. 100 and 101 show the third modification, Fig. 100 is a longitudinal cross-sectional view of the first and second pinching members which are closed, and Fig. 101 is a longitudinal cross-sectional view of the first and second pinching members which are opened. The same portions according to the first and second modifications are designated by the same reference numerals, and a description thereof is omitted.

A space portion 1071 is arranged between the pinching plates 1006a and 1006b of the operation unit 1003, and a driving shaft 1072 pierces through the space portion 1071. Both ends of the driving shaft 1072 are rotatably supported to a sliding bearing 1073 arranged to the pinching plates 1006a and 1006b. One end of the driving shaft 1072 is externally projected from the pinching plate 1006a, and the rotating dial 1020 is fixed to the projected portion by the nut 1021. Further, a driving gear 1082 comprising a resin spur gear is fit to the middle portion of the driving shaft 1072.

The driving gear 1082 is engaged with a plurality of drive-transmission gears 1083a, 1083b, 1083c, ... comprising resin spur gears arranged between the pinching plates 1027a and 1027b of the insertion unit 1002, and the rotation is then transmitted to the driving gear 1082. The last drive-transmission gear 1083n is engaged with the driven bevel gear 1078 arranged to the space 1031 in the treating unit 1004. The driven bevel gear 1078 is inclined to the insertion unit 1002, and an angle θ formed by the central axis O of the driven bevel gear 1078 and the core of the insertion unit 1002 in the longitudinal direction is obtuse (θ > 90°).

Further, a first pinching member 1084 is cylindrical, and has the proximal end of the driven bevel gear 1078 with the large diameter and the distal end with the small diameter. The first pinching member 1084 has, on the distal-end surface thereof, a circular pinching surface 1084a. A large-diameter cylindrical portion 1085 is arranged at the proximal end of the first pinching member 1084. A small-diameter cylindrical portion 1086 communicated with the large-diameter cylindrical portion 1085 is arranged at the distal end of the first pinching member 1084.

A second pinching member 1089 having a large-diameter shaft portion 1087 and a small-diameter shaft portion 1088 is accommodated, to freely be moved in the axial direction, to the large-diameter cylindrical portion 1085 and the small-diameter cylindrical portion 1086 of the first pinching member 1084. The distal end of the small-diameter shaft portion 1088 is projected from the pinching surface 1084a of the first pinching member 1084, and a disc 1090 is fixed to the distal end of the small-diameter shaft portion 1088. The disc 1090 has the same outer diameter as that of the pinching surface 1084a of the first pinching member 1084, and further has a pinching surface 1089a facing the pinching surface 1084a on the bottom surface of the disc 1090. Therefore, the pinching surfaces 1084a and 1089a become entirely circular pinching surfaces. The needle 1060 is pinched at any portion on the entire circular surfaces without rotating the first and second pinching members 1084 and 1089.

Further, a step 1087a is formed at the large-diameter shaft portion 1087 of the second pinching member 1089. A coil spring 1091, serving as energizing means, is accommodated between the step 1087a and the end surface of the large-diameter cylindrical portion 1085. The coil spring 1091 presses down the second pinching member 1089, and energizes the pinching surface 1089a in the closing direction to be adhered to the pinching surface 1084a of the first pinching member 1084.

An inclined portion 1092 is formed at the distal end of the second pinching member 1089. The inclined portion 1092 is jointed to the pressing member 1081 having the planar surface 1081b and the taper surface 1081a arranged at the distal end of the transmission rod 1043 that is freely movable by the closing knob 1045.

According to the third modification, the finger rotates the rotating dial 1020, thereby rotating the driving gear 1082 via the driving shaft 1072. The rotation of the driving gear 1082 is transmitted via the plurality of drive-transmission gears 1083a, 1083b, 1083c, ..., 1083n, thereby rotating the driven bevel gear 1078. Therefore, the first pinching member 1084 and the second pinching member 1089 integral with the driven bevel gear 1078 are rotated.

The closing knob 1045 advances the transmission rod 1043 from the state shown in Fig. 100 and the taper surface 1081a of the pressing member 1081 presses up the second pinching member 1089 while the taper surface 1081a of the pressing member 1081 is slid together with the inclined portion 1092 of the second pinching member 1089. Thereby, the coil spring 1091 is compressed, the pinching surfaces 1084a and 1089a of the first pinching member 1084 and the second pinching member 1089 are gradually opened, and the planar surface 1081a of the pressing member 1081 comes into contact with the bottom end of the second pinching member 1089. Then, the pinching surfaces 1084a and 1089a are opened as shown in Fig. 101.

Further, the closing knob 1045 returns the transmission rod 1043, then, the planar surface 1081b of the pressing member 1081 is evacuated from the inclined portion 1092 of the second pinching member 1089, and the planar surface 1081b is slid to the taper surface 1081a. Thus, the energization force of the coil spring 1091 presses down the second pinching member 1089. Thereby, referring to Fig. 100, the pinching surfaces 1084a and 1089a of the first pinching member 1084 and the second pinching member 1089 are closed. In the closing state of the pinching surfaces 1084a and 1089a, the inclined portion 1092 of the second pinching member 1089 does not come into contact with the taper surface 1081a of the pressing member 1081, or the inclined portion 1092 simply comes into contact with the taper surface 1081a. Therefore, the amount of rotation of the treating unit 1004 in the pinching state of the needle 1060 is small and the convenience is improved, as compared with the first and second modifications.

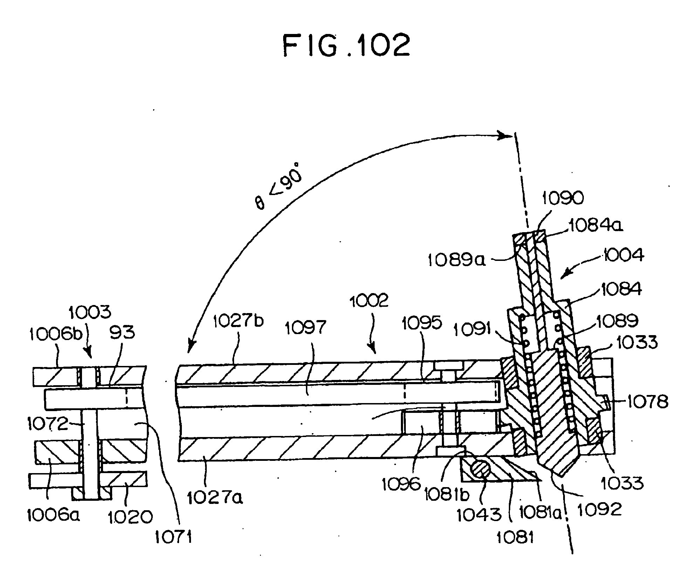

Fig. 102 is a longitudinal cross-sectional side view of the first and second pinching members which are closed according to the fourth modification. The same portions according to the first to third modifications are designated by the same reference numerals, and a description thereof is omitted.

The space portion 1071 is arranged between the pinching plates 1006a and 1006b of the operation unit 1003, and the driving shaft 1072 pierces through the space portion 1071. Both ends of the driving shaft 1072 are rotatably supported to the sliding bearing 1073 arranged to the pinching plates 1006a and 1006b. One end of the driving shaft 1072 is externally projected from the pinching plate 1006a, and the rotating dial 1020 is fixed to the projected portion by the nut 1021. Further, a driving pulley 1093 fixed to the middle portion of the driving shaft 1072 is fit to the driving shaft 1072.

The driven shaft 1094 is rotatably arranged between the pinching plates 1027a and 1027b at the distal end of the insertion unit 1002. A driven pulley 1095 is fit to one end of the driven shaft 1094, and a drive-transmission gear 1096 is fit to the other end of the driven shaft 1094. A belt 1097 is hung between the driving pulley 1093 and the driven pulley 1095, and the rotation of the driving pulley 1093 is transmitted to the driven pulley 1095 via the belt 1097.

The rotation of the driven pulley 1095 is transmitted to the drive-transmission gear 1096 via the driven shaft 1094, and the rotation of the drive-transmission gear 1096 is transmitted to the driven bevel gear 1078. The driven bevel gear 1078 is inclined to the insertion unit 1002, and the angle θ formed by the central axis O of the driven bevel gear 1078 and the core of the insertion unit 1002 in the longitudinal direction is acute angle (θ < 90°). Other structures are the same as those according to the third modification.

According to the fourth modification, the finger rotates the rotating dial 1020, thereby rotating the driving pulley 1093 via the driving shaft 1072. The rotation of the driving pulley 1093 is transmitted to the driven pulley 1095 via the belt 1097, and is transmitted to the drive-transmission gear 1096 via the driven shaft 1094, thereby rotating the driven bevel gear 1078. Therefore, the first pinching member 1084 and the second pinching member 1089 integral with the driven bevel gear 1078 are rotated. The belt 1097 and the gear mechanism transmit the rotation of the rotating dial 1020, so the weight is lighter, as compared with the third modification. The operability is improved. Since the number of gears is smaller, as compared with the third modification, the backlash is extremely reduced and the convenience is improved.

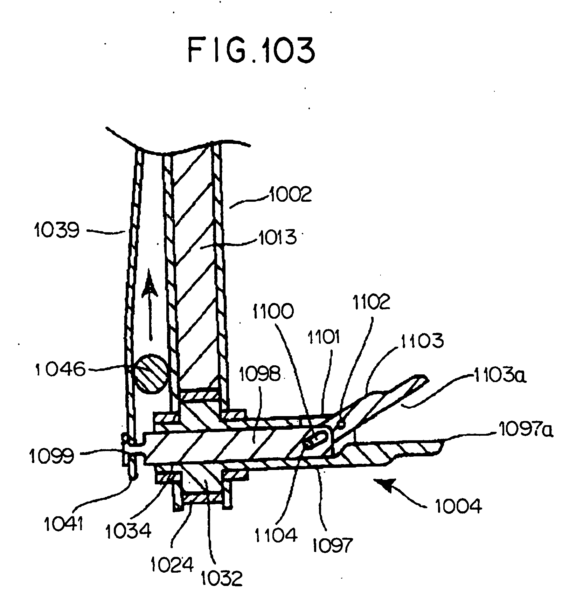

Fig. 103 is a longitudinal cross-sectional view showing the first and second pinching members which are opened according to the fifth modification. The same components as those according to the first modification are designated by the same reference numerals, and a description thereof is omitted. The pinching member 1097 perpendicularly projected to the side of the insertion unit 1002 is integrated to the rotating pulley 1032 arranged to the treating unit 1004, and a pinching surface 1097a is arranged to the side surface at the distal end of the first pinching member 1097.

The through-hole 1034 is arranged to the central shaft of the rotating pulley 1032, and a transmission shaft 1098 movable in the axial direction is arranged to the through-hole 1034. A thin-diameter portion 1099 is arranged at the proximal end of the transmission shaft 1098, and is engaged with the engaging unit 1041 of the plate spring 1039. An oblong hole 1100 is arranged at the distal end of the transmission shaft 1098. A notch portion 1101 is arranged at the middle portion of the first pinching member 1097, and a second pinching member 1103 rotatable to a pivot 1102 as supporting point is arranged to the notch portion 1101. A pin 1104 engaged with the oblong hole 1100 of the transmission shaft 1098 is arranged at the proximal end of the second pinching member 1103. A pinching surface 1103a facing the pinching surface 1097a of the first pinching member 1097 is arranged at the distal end of the second pinching member 1103.

Therefore, similarly to the first modification, the advance/return of the transmission rod 1043 modifies the plate spring 1039, thereby advancing/returning the transmission shaft 1098 in the shaft direction. The transmission shaft 1098 is advanced, thereby pressing the pin 1104 via the oblong hole 1100. The second pinching member 1103 is rotated and is opened in the separating direction from the first pinching member 1097 with the pivot 1102 as supporting point. The transmission shaft 1098 is returned, thereby pulling in the pin 1104 via the oblong hole 1100. The second pinching member 1103 is rotated and is closed in the close direction to the first jaw 1097 with the pivot 1102 as supporting point.

According to the fifth modification, the treating unit 1004 has the structure of pinching forceps comprising the first and second jaws 1097 and 1103. Thus, things other than the needle can be pinched and the treating unit 1004 can pinch any thing, as compared with the first to fourth modifications.

According to the first to fifth modifications, the operation unit 1003 and the insertion unit 1002 are planar. However, the operation unit 1003 and the insertion unit 1002 may be round-bar-shaped and the shape and material are not limited.

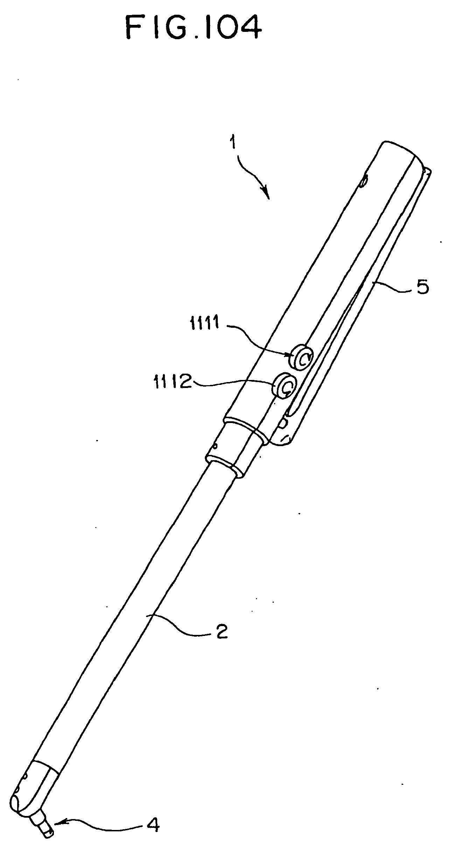

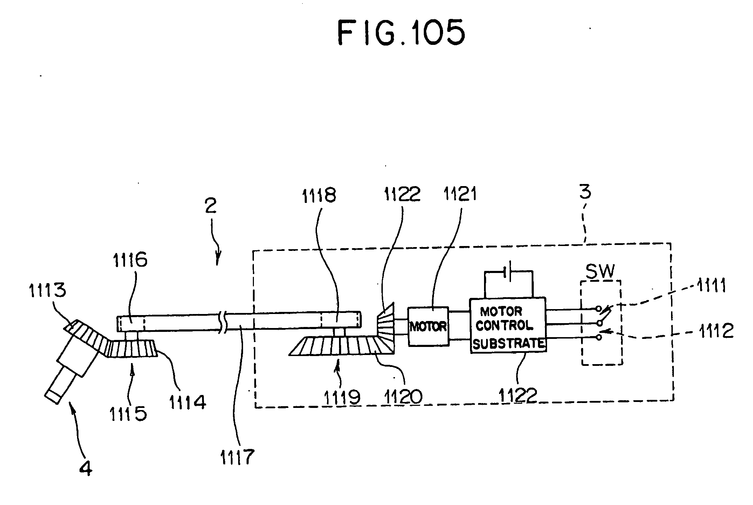

Figs. 104 and 105 show the sixth modification. Unlike the modification shown in Fig. 61, the rotation is electrically performed according to the sixth modification. Fig. 104 is a perspective view according to the sixth modification. Referring to Fig. 104, the operation unit 3 comprises a switch 1111 for instructing the rotation of two pinching members 1212 in one direction, and a switch 1112 for instructing the rotation in the direction reverse to the one direction. Therefore, the operator presses the switch (SW) corresponding to the rotating direction by his finger, thereby rotating the two pinching members 1212 in the direction.

Fig. 105 is an explanatory diagram showing the structure of the needle driver 1 according to the sixth modification. The second pinching member 1212b of the two pinching members 1212 comprises a bevel gear unit 1113. A rotating member 1115 having a bevel gear unit 1114 having teeth engaged with teeth of the bevel gear unit 1113 is arranged in the distal end of the insertion unit 2. The rotating member 1115 has a pulley unit 1116, and a timing belt 1117 is hung to the rotating member 1115.

A rotating member 1119 having a pulley unit 1118 is arranged in the operation unit 3. The rotating member 1119 has a bevel gear unit 1120, and teeth of the bevel gear unit 1120 are engaged with teeth of the bevel gear 1222 connected to the rotating shat of a motor 1121. The motor 1121 is controlled by a motor control substrate 1122. The switches (SWs) 1111 and 1112 are connected to the motor control substrate 1122, and the motor control substrate 1122 rotates the motor forward/backward in accordance with signals from the switches (SWs) 1111 and 1112.

The switch 1111 or 1112 is pressed and the motor control substrate 1122 thus controls the rotation of the motor 1121. Under the control operation, the switch 1111 or 1112 is pressed, then, the two pinching members 1212 perform predetermined rotation control, e.g., one rotation or controls the rotation at a preset angle.

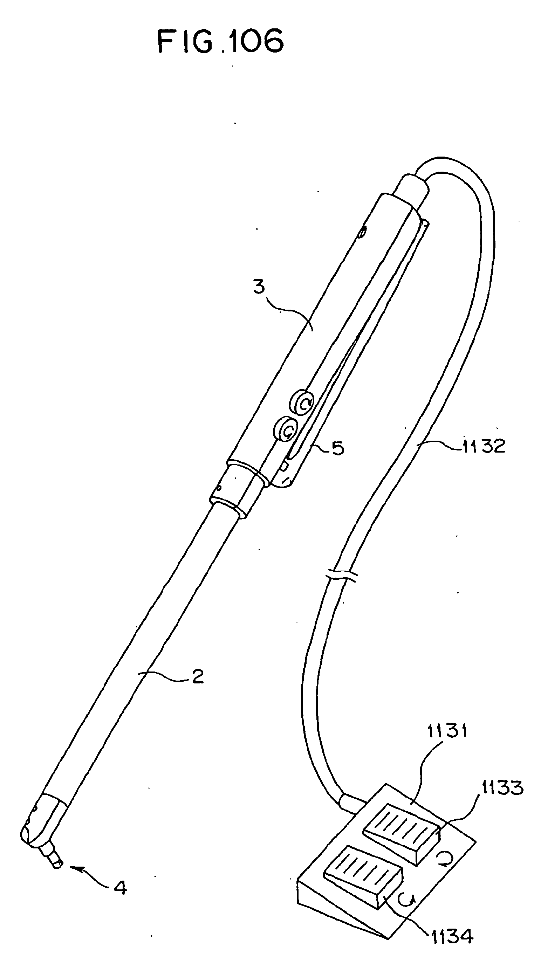

In addition to or in place of the switches 1111 and 1112 described above, a foot switch may be used. Fig. 106 is an explanatory diagram of an example using a foot switch 1131. The foot switch 1131 is connected to the operation unit 3 via a cable 1132, the operator presses any of two switches 1133 and 1134 of the foot switch 1131 by foot, and the two pinching members 1212 of the treating unit 4 are rotated in the desired direction.

### Industrial Applicability

In addition to the above-described surgical treating unit, the present invention can be applied to a medical unit using the pinching function, e.g., tool or holding tool in which the pinching function is necessary.

## Claims

1. A surgical therapeutic instrument comprising:
an insertion unit (2, 1002);
a treating unit (4, 1004) that is arranged at one end of the insertion unit (2, 1002) to be extended in an extending direction at a predetermined angle to the axial direction of the insertion unit (2, 1002);
an operation unit (3, 1003) that is arranged at the other end of the insertion unit (2, 1002);
a rotating operation unit (6) that is arranged to the operation unit (3, 1003) to rotate the treating unit (4, 1004); and
an opening/closing operation unit (5) that is arranged to the operation unit (3, 1003) to open/close the treating unit (4, 1004),
two pinching members (1035, 1037, 1084, 1089, 1212, 1212a, 1212b) that are provided at the treating unit (4, 1004), each of the two pinching members (1212) having a planar portion surface (1214a, 1216a, 1035b, 1037a, 1057a, 1084a, 1089a), wherein the planar portions surface (1214a, 1216a, 1035b, 1037a) of the two pinching members (1035, 1037, 1212) are configured to pinch a needle (9) therebetween when the pinching members (1212) are in a closed state;
a wedge member (1204, 1081a) that has a wedge-shaped distal end and moves along the axial direction in accordance with an opening/closing operation of the opening/closing operation unit (5);
wherein the treating unit (4, 1004) is rotatable around the axis in the extending direction in accordance with a rotating operation of the rotating operation unit (6),
**characterized in that**
one of the two pinching members comprises a conical wedge receiving portion (1213a, 1080, 1092) configured to receive the wedge member (1204, 1089a); and
an open/close operation of the treating unit (4) causes movement of at least the one of the two pinching members (1212) in the direction substantially orthogonal to planes of the planar portions comprising the wedge receiving portion (1213a, 1080, 1092) by moving the wedge member (1204, 1089a) in the axial direction of the insertion unit (2) to push the wedge receiving portion (1213a, 1080, 1092) in accordance with the opening/closing operation of the opening/closing operation unit (5).

2. A surgical therapeutic instrument according to Claim 1 ,
wherein the treating unit (4, 1004) comprises energizing means (1218, 1091) that always energizes at least one of the two pinching members (1212, 1084, 1089) in the direction for coming into contact with the other pinching member (1212, 1084, 1089), and
the one pinching member (1212, 1084, 1089) is moved in the separating direction of the other pinching member (1212, 1084, 1089) against energization force in the contact direction by the opening operation of the opening/closing operation unit (5).

3. A surgical therapeutic instrument according to Claim 1, wherein
the treating unit (4) comprises energizing means (1079) that always energizes at least one of the two pinching members (1035, 1057) in the separating direction from the other pinching member (1035, 1057), and
the one pinching member (1035, 1037) is moved in the contact direction to the other pinching member (1035, 1037) against energization force in the separating direction by the closing operation of the opening/closing operation (5), and the one pinching member (1035, 1037) is kept to be in contact with the other pinching member (1035, 1037) by predetermined operation of the opening/closing operation unit (5).

4. A surgical therapeutic instrument according to any one of Claims 1 to 3, wherein the predetermined angle is substantially perpendicular to the axial direction.

5. A surgical therapeutic instrument according to any one of Claims 1 to 3, wherein the predetermined angle is an acute angle in the axial direction from the insertion unit (2) to the treating unit (4).

6. A surgical, therapeutic instrument according to any one of Claims 1 to 3, wherein the predetermined angle is an obtuse angle in the axial direction from the insertion unit (2) to the treating unit (4).

7. A surgical therapeutic instrument according to any one of Claims 1 to 6, wherein the rotating operation of the rotating operation unit (6) is manual.

8. A surgical therapeutic instrument according to Claim 7, wherein the rotating operation unit (6) is a rotatable dial (6).

9. A surgical therapeutic instrument according to Claim 8, wherein the rotating direction around a rotating shaft of the dial (6) is the same as the rotating direction around the axis in the extending direction of the treating unit (4).

10. A surgical therapeutic instrument according to any one of Claims 1 to 9, further comprising:
a friction mechanism (1235e, 1236) comprising two pulley units (1206b, 1240a) and a belt (1211) disposed around the two pulley units (1206b, 1240a), that generates a predetermined friction based on the tension of the belt (1211) in the rotating operation of the rotating operation unit (6).

11. A surgical therapeutic instrument according to Claim 10, further comprising:
an adjusting mechanism comprising an oblong hole (1253) and a screw unit (1252), wherein the screw unit (1252) can be fixed at any position on an inner diameter of the oblong hole (1253) via a hut (1254) to fix a base member (1235) on which one of the two pulley units (1240a) is provided to an operation unit cover (1232) to adjust the predetermined amount of friction.

12. A surgical therapeutic instrument according to Claim 1, wherein one of the two pinching members (1212) is umbrella-shaped.

13. A surgical therapeutic instrument according to Claim 1, wherein one of the two pinching members (1035) is hook-shaped.

14. A surgical therapeutic instrument according to Claim 12 or 13, wherein the one of the two pinching members (1035, 1212) is moved in the separating direction from the other pinching member (1035, 1212) in accordance with the opening/closing operation.

15. A surgical therapeutic instrument according to Claim 12 or 13, wherein the other of the two pinching members (1035, 1212) is moved in the separating direction from the one of the two pinching members (1035, 1212) in accordance with the opening/closing operation.

16. A surgical therapeutic instrument according to any one of Claims 1 to 15, wherein the two pinching members (1035, 1212) have different colors.

17. A surgical therapeutic instrument according to any one of Claims 1 to 16, wherein a rotating angle for rotating operation of the rotating operation unit (6) is 360° or more.

18. A surgical therapeutic instrument according to any one of Claims 1 to 17, wherein rotating force from the rotating operation unit (6) to the treating unit (4) is sequentially transmitted to a first pulley (1240a), a belt (1211), a second pulley (1206a), and a bevel gear unit (1221).

19. A surgical therapeutic instrument according to Claim 18, wherein the rotating direction of the first pulley (1240a) is the same as that of the second pulley (1206a).

20. A surgical therapeutic instrument according to Claim 19, further comprising:
a first spur gear unit coaxially arranged to the first pulley (1240a); and
a second spur gear unit coaxially arranged to the rotating operation unit (6),
wherein the rotating direction of the rotating operation unit (6) is the same as that of the bevel gear unit (1221).

21. A surgical therapeutic instrument according to any one of Claims 18 to 20, further comprising:
a tension adjusting mechanism that adjusts the tension of the belt (1211).

22. A surgical therapeutic instrument according to Claim 1, wherein
the opening/closing operation unit (5) comprises a lever member having a pusher (1251), and
the wedge member (1204) comprises a V groove unit (1204c) that receives the pusher (1251).

23. A surgical therapeutic instrument according to Claim 22, wherein a play is arranged between the pusher (1251) and the V groove unit (1204c), and the lever member is energized in the opening direction.

24. A surgical therapeutic instrument according to any one of Claims 1 to 23, wherein the wedge member (1204) comprises an oblong hole (1204d) through which a shaft member (1207) of the second pulley (1206a) passes.

25. A surgical therapeutic instrument according to any one of Claims 22 to 24, wherein the operation unit (3, 1003) has a finger placing unit (1255) that partly covers the lever member.

26. A surgical therapeutic instrument according to any one of Claims 1 to 25, wherein the insertion unit (1002, 1003) has, at the distal end thereof, an air vent hole (1223) to the inside that a sterilization gas enters.

## Patentansprüche

1. Chirurgisches therapeutisches Instrument, das umfasst:
eine Einführeinheit (2, 1002);
eine Behandlungseinheit (4, 1004), die an einem Ende der Einführeinheit (2, 1002) angeordnet ist, um sich in einer Erstreckungsrichtung in einem vorbestimmten Winkel zu der axialen Richtung der Einführeinheit (2, 1002) zu erstrecken;
eine Betätigungseinheit (3, 1003), die an dem anderen Ende der Einführeinheit (2, 1002) angeordnet ist;
eine Drehbetätigungseinheit (6), die an der Betätigungseinheit (3, 1003) angeordnet ist, um die Behandlungseinheit (4, 1004) zu drehen; und
eine Öffnungs/Schließbetätigungseinheit (5), die an der Betätigungseinheit (3, 1003) angeordnet ist, um die Behandlungseinheit (4, 1004) zu öffnen/zu schließen,
zwei Zangenelemente (1035, 1037, 1084, 1089, 1212, 1212a, 1212b), die an der Behandlungseinheit (4, 1004) vorgesehen sind, wobei jedes der zwei Zangenelemente (1212) eine Oberfläche eines ebenen Abschnitts (1214a, 1216a, 1035b, 1037a, 1057a, 1084a, 1089a) hat, wobei die Oberflächen der ebenen Abschnitte (1214a, 1216a, 1035b, 1037a) der zwei Zangenelemente (1035, 1037, 1212) dazu eingerichtet sind, eine Nadel (9) zwischen sich einzuklemmen, wenn sich die Zangenelemente (1212) in einem geschlossenen Zustand befinden;
ein Keilelement (1204, 1081a), das ein keilförmiges distales Ende hat und sich gemäß einer Öffnungs/Schließbetätigung der Öffnungs/Schließbetätigungseinheit (5) entlang der axialen Richtung bewegt;
wobei die Behandlungseinheit (4, 1004) gemäß einer Drehbetätigung der Drehbetätigungseinheit (6) um die Achse in der Erstreckungsrichtung drehbar ist, **dadurch gekennzeichnet, dass**
eines der zwei Zangenelemente einen konischen Keilaufnahmeabschnitt (1213a, 1080, 1092) umfasst, der dazu eingerichtet ist, das Keilelement (1204, 1089a) aufzunehmen; und
eine Öffnungs/Schließbetätigung der Behandlungseinheit (4) eine Bewegung von mindestens einem der zwei Zangenelemente (1212) in der Richtung im Wesentlichen orthogonal zu Ebenen der ebenen Abschnitte, die den Keilaufnahmeabschnitt (1213a, 1080, 1092) umfassen, verursacht, indem das Keilelement (1204, 1089a) in der axialen Richtung der Einführeinheit (2) bewegt wird, um den Keilaufnahmeabschnitt (1213a, 1080, 1092) gemäß der Öffnungs/Schließbetätigung der Öffnungs/Schließbetätigungseinheit (5) zu schieben.

2. Chirurgisches therapeutisches Instrument gemäß Anspruch 1,
wobei die Behandlungseinheit (4, 1004) ein Antriebsmittel (1218, 1091) umfasst, das stets mindestens eines der zwei Zangenelemente (1212, 1084, 1089) in der Richtung antreibt, dass es in Kontakt mit dem anderen Zangenelement (1212, 1084, 1089) gelangt, und
das eine Zangenelement (1212, 1084, 1089) durch die Öffnungsbetätigung der Öffnungs/Schließbetätigungseinheit (5) gegen eine Antriebskraft in der Kontaktrichtung in der Separierungsrichtung des anderen Zangenelements (1212, 1084, 1089) bewegt wird.

3. Chirurgisches therapeutisches Instrument gemäß Anspruch 1, wobei
die Behandlungseinheit (4) ein Antriebsmittel (1079) umfasst, das stets mindestens eines der zwei Zangenelemente (1035, 1057) in der Separierungsrichtung von dem anderen Zangenelement (1035, 1057) antreibt, und das eine Zangenelement (1035, 1057) durch die Schließbetätigung der Öffnungs/Schließbetätigungseinheit (5) gegen eine Antriebskraft in der Separierungsrichtung in der Kontaktrichtung zu dem anderen Zangenelement (1035, 1037) bewegt wird und das andere Zangenelement (1035, 1037) durch eine vorbestimmte Betätigung der Öffnungs/Schließbetätigungseinheit (5) mit dem anderen Zangenelement (1035, 1037) in Kontakt gehalten wird.

4. Chirurgisches therapeutisches Instrument gemäß einem der Ansprüche 1 bis 3, wobei der vorbestimmte Winkel im Wesentlichen senkrecht zu der axialen Richtung ist.

5. Chirurgisches therapeutisches Instrument gemäß einem der Ansprüche 1 bis 3, wobei der vorbestimmte Winkel ein spitzer Winkel in der axialen Richtung von der Einführeinheit (2) zu der Behandlungseinheit (4) ist.

6. Chirurgisches therapeutisches Instrument gemäß einem der Ansprüche 1 bis 3, wobei der vorbestimmte Winkel ein stumpfer Winkel in der axialen Richtung von der Einführeinheit (2) zu der Behandlungseinheit (4) ist.

7. Chirurgisches therapeutisches Instrument gemäß einem der Ansprüche 1 bis 6, wobei die Drehbetätigung der Drehbetätigungseinheit (6) manuell ist.

8. Chirurgisches therapeutisches Instrument gemäß Anspruch 7, wobei die Drehbetätigungseinheit (6) eine drehbare Einstellscheibe (6) ist.

9. Chirurgisches therapeutisches Instrument gemäß Anspruch 8, wobei die Drehrichtung um eine Drehwelle der Einstellscheibe (6) die gleiche ist wie die Drehrichtung um die Achse in der Erstreckungsrichtung der Behandlungseinheit (4).

10. Chirurgisches therapeutisches Instrument gemäß einem der Ansprüche 1 bis 9, das ferner umfasst:
einen Reibungsmechanismus (1235e, 1236), der zwei Rolleneinheiten (1206b, 1240a) und einen um die Rolleneinheiten (1206b, 1240a) angeordneten Riemen (1211) umfasst und der auf der Basis der Spannung des Riemens (1211) in der Drehbetätigung der Drehbetätigungseinheit (6) eine vorbestimmte Reibung erzeugt.

11. Chirurgisches therapeutisches Instrument gemäß Anspruch 10, das ferner umfasst:
einen Einstellmechanismus, der ein Langloch (1253) und eine Schraubeneinheit (1252) umfasst, wobei die Schraubeneinheit (1252) über eine Mutter (1254) an jeder Position an einem Innendurchmesser des Langlochs (1253) fixiert werden kann, um ein Basiselement (1235), an dem eine der zwei Rolleneinheiten (1240a) vorgesehen ist, an einer Betätigungseinheitsabdeckung (1232) zu fixieren, um den vorbestimmten Betrag an Reibung einzustellen.

12. Chirurgisches therapeutisches Instrument gemäß Anspruch 1, wobei eines der zwei Zangenelemente (1212) schirmförmig ist.

13. Chirurgisches therapeutisches Instrument gemäß Anspruch 1, wobei eines der zwei Zangenelemente (1035) hakenförmig ist.

14. Chirurgisches therapeutisches Instrument gemäß Anspruch 12 oder 13, wobei das eine der zwei Zangenelemente (1035, 1212) gemäß der Öffnungs/Schließbetätigung in der Separierungsrichtung von dem anderen Zangenelement (1035, 1212) bewegt wird.

15. Chirurgisches therapeutisches Instrument gemäß Anspruch 12 oder 13, wobei das andere der zwei Zangenelemente (1035, 1212) gemäß der Öffnungs/Schließbetätigung in der Separierungsrichtung von dem einen der zwei Zangenelemente (1035, 1212) bewegt wird.

16. Chirurgisches therapeutisches Instrument gemäß einem der Ansprüche 1 bis 15, wobei die zwei Zangenelemente (1035, 1212) verschiedene Farben haben.

17. Chirurgisches therapeutisches Instrument gemäß einem der Ansprüche 1 bis 16, wobei ein Drehwinkel für eine Drehbetätigung der Drehbetätigungseinheit (6) 360° oder mehr ist.

18. Chirurgisches therapeutisches Instrument gemäß einem der Ansprüche 1 bis 17, wobei eine Drehkraft von der Drehbetätigungseinheit (6) an die Behandlungseinheit (4) sequenziell auf eine erste Rolle (1240a), einen Riemen (1211), eine zweite Rolle (1206a) und eine Kegelradeinheit (1221) übertragen wird.

19. Chirurgisches therapeutisches Instrument gemäß Anspruch 18, wobei die Drehrichtung der ersten Rolle (1240a) die gleiche ist wie die der zweiten Rolle (1206a).

20. Chirurgisches therapeutisches Instrument gemäß Anspruch 19, das ferner umfasst:
eine erste Stirnradeinheit, die koaxial zu der ersten Rolle (1240a) angeordnet ist; und
eine zweite Stirnradeinheit, die koaxial zu der Drehbetätigungseinheit (6) angeordnet ist,
wobei die Drehrichtung der Drehbetätigungseinheit (6) die gleiche ist wie die der Kegelradeinheit (1221).

21. Chirurgisches therapeutisches Instrument gemäß einem der Ansprüche 18 bis 20, das ferner umfasst:
einen Spannungseinstellmechanismus, der die Spannung des Riemens (1211) einstellt.

22. Chirurgisches therapeutisches Instrument gemäß Anspruch 1, wobei
die Öffnungs/Schließbetätigungseinheit (5) ein Hebelelement mit einem Schieber (1251) umfasst und
das Keilelement (1204) eine V-Nuteinheit (1204c) umfasst, die den Schieber (1251) aufnimmt.

23. Chirurgisches therapeutisches Instrument gemäß Anspruch 22, wobei ein Spiel zwischen dem Schieber (1251) und der V-Nuteinheit (1204c) vorhanden ist und das Hebelelement in der Öffnungsrichtung angetrieben wird.

24. Chirurgisches therapeutisches Instrument gemäß einem der Ansprüche 1 bis 23, wobei das Keilelement (1204) ein Langloch (1204d) umfasst, durch das ein Wellenelemente (1207) der zweiten Rolle (1206a) hindurchgeführt ist.

25. Chirurgisches therapeutisches Instrument gemäß einem der Ansprüche 22 bis 24, wobei die Betätigungseinheit (3, 1003) eine Fingerplatzierungseinheit (1255) hat, die das Hebelelement teilweise abdeckt.

26. Chirurgisches therapeutisches Instrument gemäß einem der Ansprüche 1 bis 25, wobei die Einführeinheit (1002, 1003) an ihrem distalen Ende ein Belüftungsloch (1223) zu der Innenseite hat, durch das ein Sterilisationsgas eintritt.

## Revendications

1. Instrument thérapeutique chirurgical comprenant :
une unité (2, 1002) d'insertion ;
une unité (4, 1004) de traitement qui est agencée à une extrémité de l'unité (2, 1002) d'insertion pour être étendue dans un sens d'extension à un angle prédéterminé par rapport au sens axial de l'unité (2, 1002) d'insertion ;
une unité (3, 1003) d'opération qui est agencée à l'autre extrémité de l'unité (2, 1002) d'insertion ;
une unité (6) d'opération de rotation qui est agencée sur l'unité (3, 1003) d'opération pour faire tourner l'unité (4, 1004) de traitement ; et
une unité (5) d'opération d'ouverture/fermeture qui est agencée sur l'unité (3, 1003) d'opération pour ouvrir/fermer l'unité (4, 1004) de traitement,
deux éléments (1035, 1037, 1084, 1089, 1212, 1212a, 1212b) de pincement qui sont prévus au niveau de l'unité (4, 1004) de traitement, chacun des deux éléments (1212) de pincement ayant une surface (1214a, 1216a, 1035b, 1037a, 1057a, 1084a, 1089a) de partie plane, dans lequel les surfaces (1214a, 1216a, 1035b, 1037a) de partie plane des deux éléments (1035, 1037, 1212) de pincement sont configurées pour pincer une aiguille (9) entre celles-ci lorsque les éléments (1212) de pincement sont dans un état fermé ;
un élément en biseau (1204, 1081a) qui a une extrémité distale cunéiforme et se déplace le long du sens axial conformément à une opération d'ouverture/fermeture de l'unité (5) d'opération d'ouverture/fermeture ;
dans lequel l'unité (4, 1004) de traitement est rotative autour de l'axe dans le sens d'extension conformément à une opération de rotation de l'unité (6) d'opération de rotation,
**caractérisé en ce que**
un des deux éléments de pincement comprend une partie conique (1213a, 1080, 1092) de réception de biseau configurée pour recevoir l'élément en biseau (1204, 1089a) ; et
une opération d'ouverture/fermeture de l'unité (4) de traitement cause un mouvement d'au moins un des deux éléments (1212) de pincement dans le sens sensiblement orthogonal à des plans des parties planes comprenant la partie (1213a, 1080, 1092) de réception de biseau en déplaçant l'élément en biseau (1204, 1089a) dans le sens axial de l'unité (2) d'insertion pour pousser la partie (1213a, 1080, 1092) de réception de biseau conformément à l'opération d'ouverture/fermeture de l'unité (5) d'opération d'ouverture/fermeture.

2. Instrument thérapeutique chirurgical selon la revendication 1,
dans lequel l'unité (4, 1004) de traitement comprend des moyens (1218, 1091) d'excitation qui excitent toujours au moins un des deux éléments (1212, 1084, 1089) de pincement dans le sens pour venir en contact avec l'autre élément (1212, 1084, 1089) de pincement, et
l'élément (1212, 1084, 1089) de pincement est déplacé dans le sens de la séparation de l'autre élément (1212, 1084, 1089) de pincement contre une force d'excitation dans le sens de contact par l'opération d'ouverture de l'unité (5) d'opération d'ouverture/fermeture.

3. Instrument thérapeutique chirurgical selon la revendication 1, dans lequel
l'unité (4, 1004) de traitement comprend un moyen (1079) d'excitation qui excitent toujours au moins un des deux éléments (1035, 1057) de pincement dans le sens de la séparation de l'autre élément (1035, 1057) de pincement, et
l'élément (1035, 1037) de pincement est déplacé dans le sens de contact vers l'autre élément (1035, 1037) de pincement contre une force d'excitation dans le sens de la séparation par l'opération de fermeture de l'opération (5) d'ouverture/fermeture, et l'élément (1035, 1037) de pincement est maintenu pour être en contact avec l'autre élément (1035, 1037) de pincement par une opération prédéterminée de l'unité (5) d'opération d'ouverture/fermeture.

4. Instrument thérapeutique chirurgical selon l'une quelconque des revendications 1 à 3, dans lequel l'angle prédéterminé est sensiblement perpendiculaire au sens axial.

5. Instrument thérapeutique chirurgical selon l'une quelconque des revendications 1 à 3, dans lequel l'angle prédéterminé est un angle aigu dans le sens axial de l'unité (2) d'insertion jusqu'à l'unité (4) de traitement.

6. Instrument thérapeutique chirurgical selon l'une quelconque des revendications 1 à 3, dans lequel l'angle prédéterminé est un angle obtus dans le sens axial de l'unité (2) d'insertion jusqu'à l'unité (4) de traitement.

7. Instrument thérapeutique chirurgical selon l'une quelconque des revendications 1 à 6, dans lequel l'opération de rotation de l'unité (6) d'opération de rotation est manuelle.

8. Instrument thérapeutique chirurgical selon la revendication 7, dans lequel l'unité (6) d'opération de rotation est un cadran rotatif (6).

9. Instrument thérapeutique chirurgical selon la revendication 8, dans lequel le sens de rotation autour d'un arbre rotatif du cadran (6) est le même que le sens de rotation autour de l'axe dans le sens d'extension de l'unité (4) de traitement.

10. Instrument thérapeutique chirurgical selon l'une quelconque des revendications 1 à 9, comprenant en outre :
un mécanisme de frottement (1235e, 1236) comprenant deux unités (1206b, 1240a) de poulie et une courroie (1211) disposée autour des deux unités (1206b, 1240a) de poulie, qui génère un frottement prédéterminé sur la base de la tension de la courroie (1211) dans l'opération de rotation de l'unité (6) d'opération de rotation.

11. Instrument thérapeutique chirurgical selon la revendication 10, comprenant en outre :
un mécanisme d'ajustement comprenant un trou oblong (1253) et une unité (1252) de vis, dans lequel l'unité (1252) de vis peut être fixée en une quelconque position sur un diamètre intérieur du trou oblong (1253) par l'intermédiaire d'un écrou (1254) pour fixer un élément (1235) de base sur celle des deux unités (1240a) de poulie qui est prévue sur un capot (1232) d'unité d'opération pour ajuster la quantité prédéterminée de frottement.

12. Instrument thérapeutique chirurgical selon la revendication 1, dans lequel un des deux éléments (1212) de pincement est en forme de parapluie.

13. Instrument thérapeutique chirurgical selon la revendication 1, dans lequel un des deux éléments (1035) de pincement est en forme de crochet.

14. Instrument thérapeutique chirurgical selon la revendication 12 ou 13, dans lequel l'un des deux éléments (1035, 1212) de pincement est déplacé dans le sens de la séparation de l'autre élément (1035, 1212) de pincement conformément à l'opération d'ouverture/fermeture.

15. Instrument thérapeutique chirurgical selon la revendication 12 ou 13, dans lequel l'autre des deux éléments (1035, 1212) de pincement est déplacé dans le sens de la séparation de l'un des deux éléments (1035, 1212) de pincement conformément à l'opération d'ouverture/fermeture.

16. Instrument thérapeutique chirurgical selon l'une quelconque des revendications 1 à 15, dans lequel les deux éléments (1035, 1212) de pincement ont des couleurs différentes.

17. Instrument thérapeutique chirurgical selon l'une quelconque des revendications 1 à 16, dans lequel un angle de rotation pour une opération de rotation de l'unité (6) d'opération de rotation est 360° ou plus.

18. Instrument thérapeutique chirurgical selon l'une quelconque des revendications 1 à 17, dans lequel une force de rotation de l'unité (6) d'opération de rotation jusqu'à l'unité (4) de traitement est séquentiellement transmise à une première poulie (1240a), une courroie (1211), une deuxième poulie (1206a), et une unité (1221) de roue conique.

19. Instrument thérapeutique chirurgical selon la revendication 18, dans lequel le sens de rotation de la première poulie (1240a) est le même que celui de la deuxième poulie (1206a).

20. Instrument thérapeutique chirurgical selon la revendication 19, comprenant en outre :
une première unité de roue droite cylindrique coaxialement agencée sur la première poulie (1240a) ; et
une deuxième unité de roue droite cylindrique coaxialement agencée sur l'unité (6) d'opération de rotation,
dans lequel le sens de rotation de l'unité (6) d'opération de rotation est le même que celui de l'unité (1221) de roue conique.

21. Instrument thérapeutique chirurgical selon l'une quelconque des revendications 18 à 20, comprenant en outre :
un mécanisme d'ajustement de tension qui ajuste la tension de la courroie (1211).

22. Instrument thérapeutique chirurgical selon la revendication 1, dans lequel
l'unité (5) d'opération d'ouverture/fermeture comprend un élément de levier ayant un dispositif de poussée (1251), et
l'élément en biseau (1204) comprend une unité (1204c) de rainure en V qui reçoit le dispositif de poussée (1251).

23. Instrument thérapeutique chirurgical selon la revendication 22, dans lequel un jeu est agencé entre le dispositif de poussée (1251) et l'unité (1204c) de rainure en V, et l'élément de levier est excité dans le sens d'ouverture.

24. Instrument thérapeutique chirurgical selon l'une quelconque des revendications 1 à 23, dans lequel l'élément en biseau (1204) comprend un trou oblong (1204d) à travers lequel un élément (1207) d'arbre de la deuxième poulie (1206a) passe.

25. Instrument thérapeutique chirurgical selon l'une quelconque des revendications 22 à 24, dans lequel l'unité (3, 1003) d'opération a une unité (1255) de mise en place de doigt qui couvre partiellement l'élément de levier.

26. Instrument thérapeutique chirurgical selon l'une quelconque des revendications 1 à 25, dans lequel l'unité (1002, 1003) d'insertion a, à l'extrémité distale de celle-ci, un orifice (1223) d'aération vers l'intérieur qu'un gaz de stérilisation pénètre.
